# EUROPEAN PATENT APPLICATION

(11) **EP 4 660 194 A1**
(43) Date of publication of application: **10.12.2025**
(21) Application number: 24749750.6
(22) Date of filing: 02.02.2024
(51) Int. Cl.: C07D 405/14, C07D 407/14, A61K 31/435, A61K 31/4184, A61K 31/4188, A61P 3/10, A61P 3/04

(54) **SALT AND CRYSTAL FORM OF CYCLOALKENE COMPOUND, AND PREPARATION METHOD THEREFOR AND USE THEREOF**

(30) Priority: 02.02.2023 CN 202310093824
(71) Applicant: Jiangsu Hansoh Pharmaceutical Group Co., Ltd., Lianyungang, Jiangsu 222047 (CN); Shanghai Hansoh Biomedical Co., Ltd., Shanghai 201203 (CN)
(72) Inventor: YANG, Shiyi, Shanghai 201203 (CN); XU, Wei, Shanghai 201203 (CN); GUO, Linsong, Shanghai 201203 (CN)
(74) Representative: Murgitroyd & Company
(86) International application number: PCT/CN2024/075434
(87) International publication number: WO 2024/160271

(57) **Abstract**

A salt and a crystal form of a cycloalkene compound, and a preparation method therefor and the use thereof. Specifically, a salt and a crystal form of a compound as represented by general formula (I), a preparation method, and a pharmaceutical composition comprising a therapeutically effective amount of the salt, and the use thereof as a regulator in the preparation of a drug for treating metabolic diseases and related diseases.

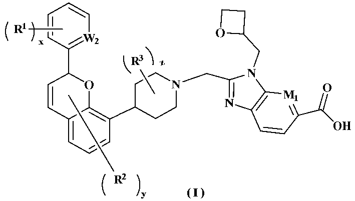

## Description

### FIELD OF THE INVENTION

The present disclosure belongs to the field of biomedicine, and specifically relates to a salt and crystal form of a cycloalkene compound, and a preparation method therefor and the use thereof.

### BACKGROUD OF THE INVENTION

Diabetes mellitus is a common endocrine metabolic disease, which causes metabolic disorders due to various reasons and leads to multi-system and multi-organ damage. The incidence rate is high. There are about 425 million diabetic patients worldwide. The incidence rate of diabetes in China is about 10%, of which type II diabetes accounts for 90%, and the incidence rate is still increasing, and the age of onset is becoming increasingly younger.

Currently, there are many types of drugs on the market for the treatment of type 2 diabetes, including insulin, biguanides, glucagon-like peptide 1 (GLP-1) receptor agonists, dipeptidyl peptidase (DPP-IV) inhibitors, sodium-glucose cotransporter 2 (SGLT-2) inhibitors, α-glucosidase inhibitors, etc., among which GLP-1 receptor agonists are the most concerned.

GLP-1 is a peptide-based hormone secreted by human intestinal L cells. Its receptors are distributed in pancreatic islet cells, various gastrointestinal cells, central nervous system and peripheral nervous system neurons. After activation, GLP-1 receptors have physiological effects such as promoting insulin secretion, inhibiting glucagon secretion, suppressing appetite and delaying gastric emptying. Clinical evidence shows that compared with other hypoglycemic drugs, GLP-1 receptor agonists have better hypoglycemic effects and are not prone to side effects such as hypoglycemia. In addition, it has additional cardiovascular benefits, and can reduce food intake and delay gastric emptying, which is conducive to weight control.

The GLP-1 receptor agonists currently on the market are all polypeptide-based drugs, most of which require subcutaneous administration, and patient compliance is poor, while the bioavailability of oral polypeptides is very low. Therefore, there is a great clinical demand for the development of oral small molecule GLP-1 receptor agonists.

At present, no small molecule GLP-1 receptor agonists have been approved. 3 small molecule GLP-1 receptor agonists have entered the clinical research stage, such as PF-06882961 and PF-07081532 developed by Pifzer, and TTP273 developed by vTv, which are currently in the clinical phase I/II research stage. Among them, PF-06882961 showed significant hypoglycemic and weight-reducing effects in early clinical trials, and its safety is similar to that of peptide-based GLP-1 receptor agonists. It is expected to provide more treatment options for patients with diabetes, obesity, and NASH in the future.

There is a huge clinical demand for GLP-1 receptor agonists. Oral small molecule GLP-1 receptor agonists with lower cost and better compliance have the potential to treat a variety of metabolic diseases and have broad market prospects.

PCT/CN2022/110017 discloses the structures of a series of polycyclic compounds of cycloalkene derivatives. In subsequent research and development, in order to make the product easy to handle, filter and dry, and to find a suitable crystal that is easy to store and has long-term stability, the present disclosure conducts a comprehensive study on the salt form of the above-mentioned compounds.

### SUMMARY OF THE INVENTION

All contents involved in patent PCT/CN2022/110017 are added to the present disclosure by reference.

The purpose of the present disclosure is to provide an base salt of a compound as represented by general formula (I) or a stereoisomer thereof, wherein the structure of the compound is as represented by formula (I): 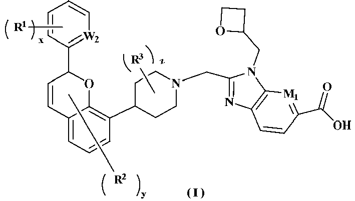 wherein:
each R¹ is independently selected from hydrogen, deuterium, fluorine, chlorine, cyano, C₁₋₃ alkyl, C₁₋₃ deuteroalkyl, C₁₋₃ haloalkyl, C₁₋₃ alkoxy, C₁₋₃ deuteroalkoxy or C₁₋₃ haloalkoxy; preferably, hydrogen, deuterium, fluorine, chlorine, methoxy or -OCD3;
each R² is independently selected from hydrogen, deuterium, fluorine, chlorine, cyano, C₁₋₃ alkyl, C₁₋₃ deuteroalkyl, C₁₋₃ haloalkyl, C₁₋₃ alkoxy, C₁₋₃ deuteroalkoxy or C₁₋₃ haloalkoxy; preferably hydrogen, deuterium, fluorine, chlorine or methyl;
each R³ is independently selected from hydrogen, deuterium or fluorine; preferably hydrogen;
M₁ is N or CH; preferably CH;
W₂ is N or CH; preferably CH;
x, y and z are each independently 0, 1 or 2;
wherein the base is an organic base or an inorganic base, and the organic base is selected from diethylamine, diethanolamine, triethylamine, triethanolamine, choline hydroxide, tromethamine, meglumine, N-hydroxyethylmorpholine, piperazine, N-hydroxyethylpyrrolidine, N,N-dibenzylethylenediamine, 2-diethylaminoethanol, ethanolamine, betaine, L-arginine, lysine, phenethylbenzylamine, benzathine penicillin, dimethylaminoethanol, imidazole or a mixture thereof; and the inorganic base is selected from sodium hydroxide, potassium hydroxide, calcium hydroxide, magnesium hydroxide, zinc hydroxide, ammonia water or a mixture thereof.

In further preferred certain embodiments of the present disclosure, the compound is further as represented by the following general formula (I-1) - (I-4):

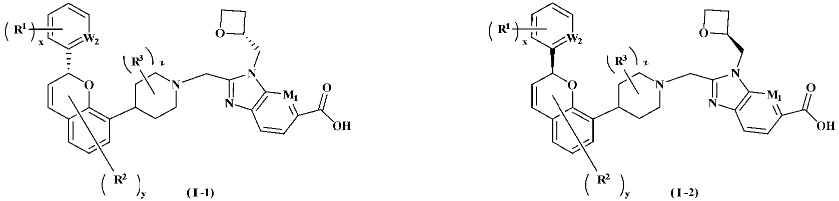

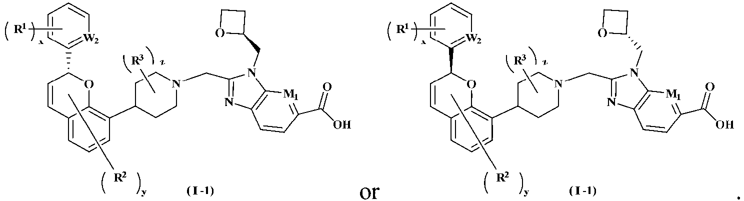

In certain embodiments of the present disclosure, the general formula (I) is selected from the following compounds: 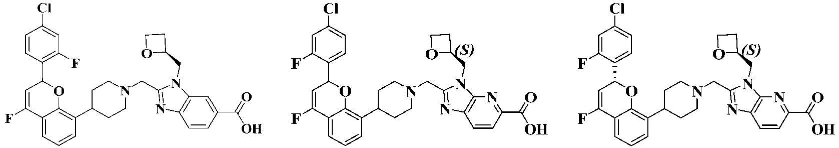 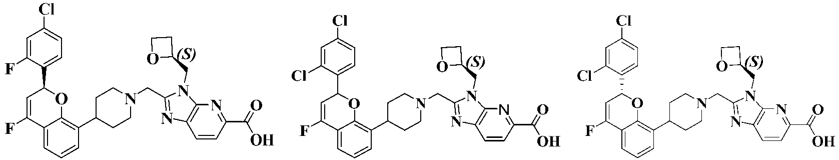 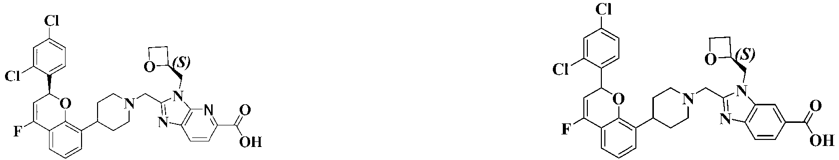 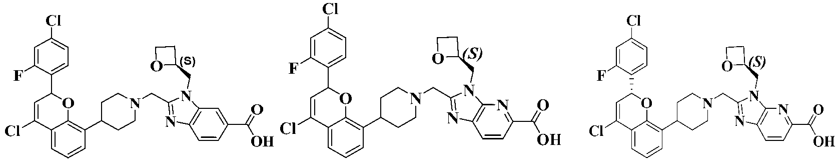 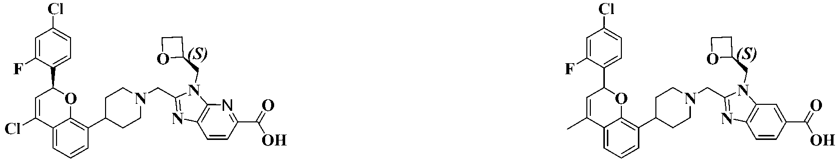 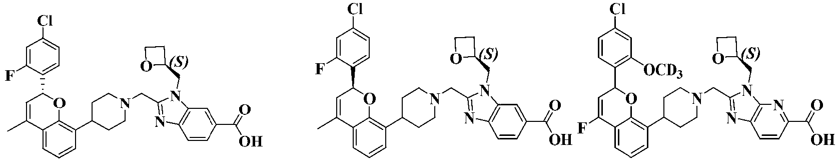 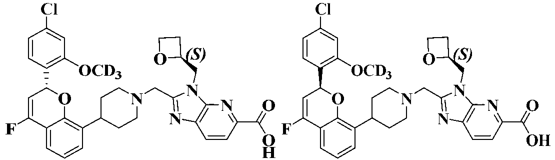 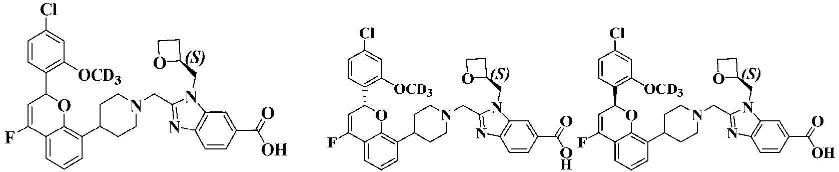 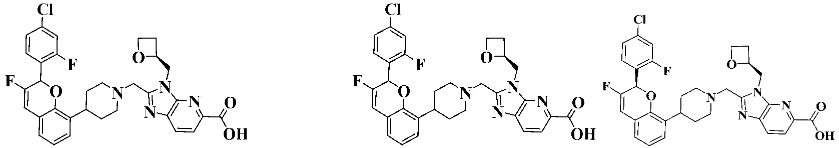 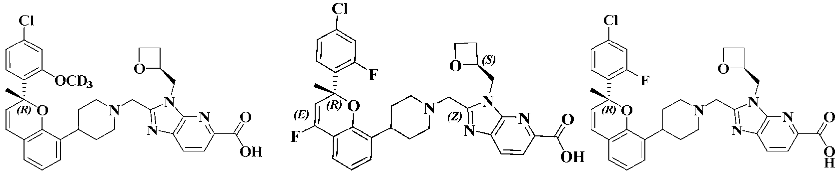 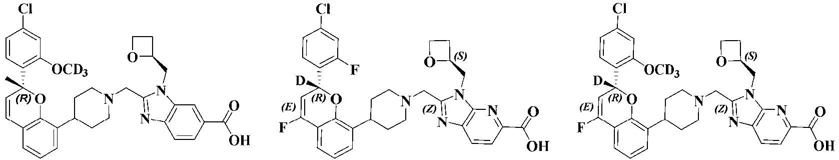 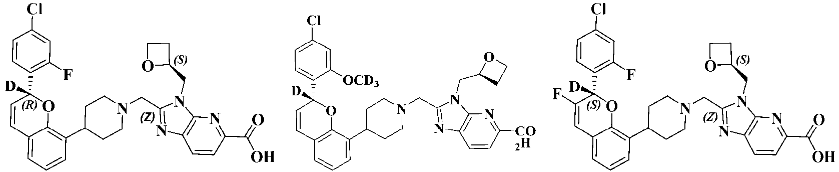 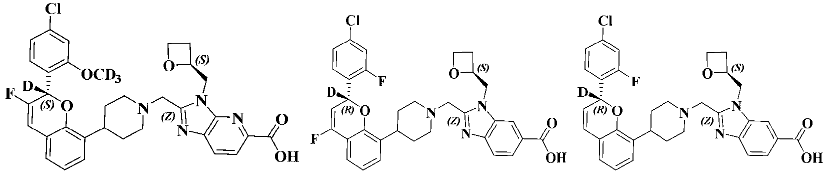 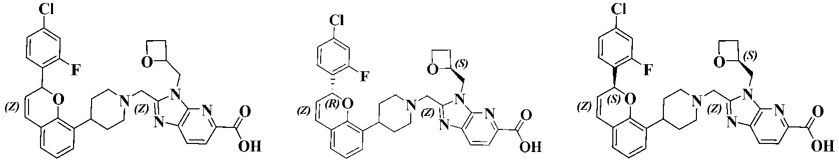 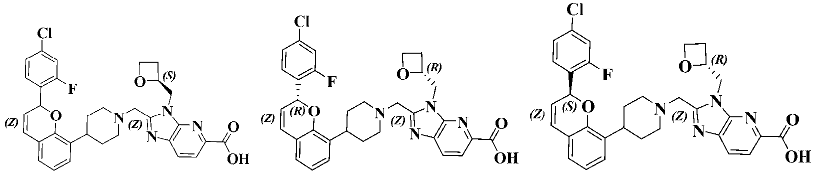 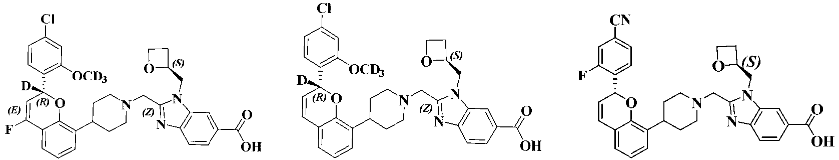 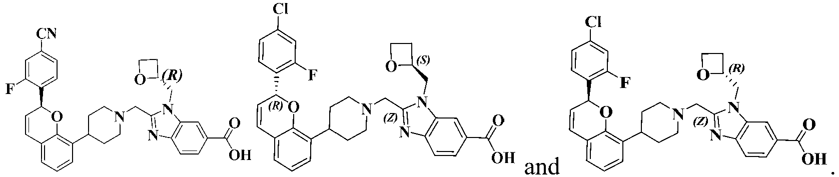 wherein the base is an organic base or an inorganic base, and the organic base is selected from diethylamine, diethanolamine, triethylamine, triethanolamine, choline hydroxide, tromethamine, or a mixture thereof; and the inorganic base is selected from sodium hydroxide, potassium hydroxide, calcium hydroxide, magnesium hydroxide, or a mixture thereof.

In a preferred embodiment of the present disclosure, a base salt of a compound of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid or a stereoisomer thereof is provided, wherein the compound or the stereoisomer thereof and base salt are tromethamine salt.

In a further preferred embodiment of the present disclosure, the number of the base is 0.5-3, preferably 0.5, 1, 1.5, 2, 2.5 or 3, further preferably 0.5, 1, 2 or 3, and even further preferably 1.

In certain embodiments of the present disclosure, the crystal form is a crystal form of the salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid, wherein the crystal form is an anhydrous crystal form.

In certain embodiments of the present disclosure, the crystal form is a crystal form of the salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid, wherein the crystal form is a hydrate containing 0.2 to 3 waters, preferably 0.5 water, 1 water, 2 waters or 3 waters.

In certain embodiments of the present disclosure, the water molecules of the hydrate are channel water or crystal water.

In a preferred embodiment of the present disclosure, a base salt of a compound of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid or a stereoisomer thereof is provided, wherein the compound has a structure as follows:

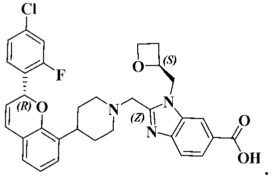

In certain embodiments of the present disclosure, the crystal form is the crystal form A of the tromethamine salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid, where:
the X-ray powder diffraction pattern of the crystal form A of tromethamine salt has a characteristic peak at 2θ of 7.7±0.2°, or has a characteristic peak at 2θ of 9.8±0.2°, or has a characteristic peak at 2θ of 10.8±0.2°, or has a characteristic peak at 2θ of 11.6±0.2°, or has a characteristic peak at 2θ of 14.0±0.2°, or has a characteristic peak at 2θ of 14.5±0.2°, or has a characteristic peak at 2θ of 14.8±0.2°, or has a characteristic peak at 2θ of 15.1±0.2°, or has a characteristic peak at 2θ of 15.9±0.2°, or has a characteristic peak at 20 of 18.0±0.2°, or has a characteristic peak at 2θ of 18.7±0.2°, or has a characteristic peak at 2θ of 19.3±0.2°, or has a characteristic peak at 2θ of 20.0±0.2°, or has a characteristic peak at 2θ of 20.5±0.2°, or has a characteristic peak at 2θ of 21.1±0.2°, or has a characteristic peak at 2θ of 22.6±0.2°, or has a characteristic peak at 20 of 23.7±0.2°, or has a characteristic peak at 20 of 25.1±0.2°; preferably, the X-ray powder diffraction pattern has characteristic peaks at any 2, 4, 6, 8, 10 or 12 of the above-mentioned 2θ.

In certain embodiments of the present disclosure, the X-ray powder diffraction pattern of the crystal form A of tromethamine salt has a characteristic peak at one or more of 2θ of 7.7±0.2°, 9.8±0.2°, 14.0±0.2° or 15.1±0.2°; preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can further have a characteristic peak at one or more of 2θ of 14.5±0.2°, 18.7±0.2°, 19.3±0.2°, 20.0±0.2°, 20.5±0.2° or 21.1±0.2°, preferably at 2, 3, 4 or 6 of the above-mentioned 2θ; for example:
7.7±0.2°, 9.8±0.2°, 14.0±0.2°;
7.7±0.2°, 9.8±0.2°, 14.0±0.2°, 15.1±0.2°;
7.7±0.2°, 9.8±0.2°, 14.0±0.2°, 14.5±0.2°;
7.7±0.2°, 9.8±0.2°, 14.0±0.2°, 18.7±0.2°;
7.7±0.2°, 9.8±0.2°, 14.0±0.2°, 19.3±0.2°;
7.7±0.2°, 9.8±0.2°, 14.5±0.2°, 18.7±0.2°;
7.7±0.2°, 9.8±0.2°, 14.0±0.2°, 20.5±0.2°;
7.7±0.2°, 9.8±0.2°, 14.0±0.2°, 20.0±0.2°;
9.8±0.2°, 14.0±0.2°, 14.5+0.2°, 18.7±0.2°;
7.7±0.2°, 9.8±0.2°, 14.0±0.2°, 14.5±0.2°, 15.1±0.2°;
7.7±0.2°, 9.8±0.2°, 14.0±0.2°, 14.5±0.2°, 18.7±0.2°;
7.7±0.2°, 9.8±0.2°, 14.0±0.2°, 18.7±0.2°, 20.0±0.2°;
7.7±0.2°, 9.8±0.2°, 14.0±0.2°, 19.3+0.2°, 20.5±0.2°;
7.7±0.2°, 9.8±0.2°, 14.5±0.2°, 18.7±0.2°, 20.5±0.2°;
7.7±0.2°, 9.8±0.2°, 14.0±0.2°, 19.3+0.2°, 20.5±0.2°;
7.7±0.2°, 9.8±0.2°, 14.0±0.2°, 20.0±0.2°, 21.1±0.2°;
9.8±0.2°, 14.0±0.2°, 14.5±0.2°, 18.7±0.2°, 21.1±0.2°;
7.7±0.2°, 9.8±0.2°, 14.0±0.2°, 14.5±0.2°, 15.1±0.2°, 18.7±0.2°;
7.7±0.2°, 9.8±0.2°, 14.0±0.2°, 14.5±0.2°, 18.7±0.2°, 19.3±0.2°;
7.7±0.2°, 9.8±0.2°, 14.0±0.2°, 18.7±0.2°, 19.3±0.2°, 20.0±0.2°;
7.7±0.2°, 9.8±0.2°, 14.0±0.2°, 19.3±0.2°, 20.5±0.2°, 21.1±0.2°;
7.7±0.2°, 9.8±0.2°, 14.5±0.2°, 18.7±0.2°, 20.5±0.2°, 21.1±0.2°;
7.7±0.2°, 9.8±0.2°, 14.0±0.2°, 19.3±0.2°, 20.5±0.2°, 21.1±0.2°;
7.7±0.2°, 14.0±0.2°, 14.5±0.2°, 19.3±0.2°, 20.0±0.2°, 21.1±0.2°;
9.8±0.2°, 14.0±0.2°, 14.5±0.2°, 18.7±0.2°, 19.3±0.2°, 21.1±0.2°;
more preferably, the X-ray powder diffraction pattern thereof optionally also has a diffraction peak at one or more of 2θ of 10.8±0.2°, 11.6±0.2°, 14.5±0.2°, 18.0±0.2°, 20.5±0.2° or 22.6± 0.2°; preferably at any 2 to 4, or any 5 or 6 of the above-mentioned 2θ, and further preferably at any 4 or 6 of the above-mentioned 2θ; for example:
   10.8±0.2°, 11.6±0.2°, 14.5±0.2°, 18.0±0.2°;
   10.8±0.2°, 11.6±0.2°, 14.5±0.2°, 20.5±0.2°;
   10.8±0.2°, 11.6±0.2°, 14.5±0.2°, 22.6±0.2°;
   10.8±0.2°, 11.6±0.2°, 18.0±0.2°, 20.5±0.2°;
   11.6±0.2°, 14.5±0.2°, 18.0±0.2°, 20.5±0.2°;
   10.8±0.2°, 11.6±0.2°, 14.5±0.2°, 18.0±0.2°, 20.5±0.2°;
   10.8±0.2°, 11.6+0.2°, 14.5±0.2°, 20.5±0.2°, 22.6±0.2°;
   10.8±0.2°, 11.6+0.2°, 14.5±0.2°, 18.0±0.2°, 20.5±0.2°, 22.6±0.2°;

In certain embodiments of the present disclosure, the X-ray powder diffraction pattern of the crystal form A of tromethamine salt has characteristic peaks at 2θ of 9.8±0.2° and 14.0±0.2°; preferably, the X-ray powder diffraction pattern further has characteristic peaks at 2θ of 7.7±0.2° and 15.1±0.2°; more preferably, the X-ray powder diffraction pattern further has characteristic peaks at 2θ of 18.7±0.2°, 19.3±0.2°, 20.0±0.2° and 21.1±0.2°; further preferably, the X-ray powder diffraction pattern further has characteristic peaks at 2θ of 14.5±0.2° and 20.5±0.2°; more further preferably, the X-ray powder diffraction pattern further has a characteristic peak at one or more of 10.8±0.2°, 11.6±0.2°, 18.0±0.2° and 22.6±0.2°.

In certain embodiments of the present disclosure, by using Cu-Kα radiation, the X-ray characteristic diffraction peaks represented by 2θ angles and interplanar spacing d values are shown in Table 1.

**Table 1**

| Serial No. | XRPD ray diffraction data of the crystal form A of tromethamine salt | | | |
|---|---|---|---|---|
| | 2θ (±0.2 °) | d value | Peak height | Proportion (I%) |
| 1 | 7.73 | 11.4281 | 580 | 76.5 |
| 2 | 9.819 | 9.0002 | 736 | 97.1 |
| 3 | 10.771 | 8.2072 | 202 | 26.6 |
| 4 | 11.584 | 7.6329 | 226 | 29.8 |
| 5 | 12.883 | 6.866 | 49 | 6.5 |
| 6 | 13.993 | 6.3237 | 758 | 100 |
| 7 | 14.527 | 6.0926 | 335 | 44.2 |
| 8 | 14.824 | 5.9708 | 138 | 18.2 |
| 9 | 15.065 | 5.8759 | 577 | 76.1 |
| 10 | 15.877 | 5.5773 | 101 | 13.3 |
| 11 | 16.467 | 5.3789 | 60 | 7.9 |
| 12 | 17.928 | 4.9436 | 190 | 25.1 |
| 13 | 18.74 | 4.7312 | 369 | 48.7 |
| 14 | 19.344 | 4.5848 | 562 | 74.1 |
| 15 | 19.952 | 4.4464 | 518 | 68.3 |
| 16 | 20.542 | 4.32 | 340 | 44.9 |
| 17 | 20.81 | 4.265 | 101 | 13.3 |
| 18 | 21.132 | 4.2006 | 409 | 54 |
| 19 | 21.698 | 4.0924 | 80 | 10.6 |
| 20 | 22.633 | 3.9254 | 228 | 30.1 |
| 21 | 23.258 | 3.8213 | 53 | 7 |
| 22 | 23.661 | 3.7572 | 192 | 25.3 |
| 23 | 24.821 | 3.5841 | 144 | 19 |
| 24 | 25.102 | 3.5446 | 238 | 31.4 |
| 25 | 25.674 | 3.4669 | 180 | 23.7 |
| 26 | 26.036 | 3.4195 | 78 | 10.3 |
| 27 | 26.852 | 3.3175 | 78 | 10.3 |
| 28 | 27.395 | 3.253 | 115 | 15.2 |
| 29 | 27.715 | 3.2161 | 92 | 12.1 |
| 30 | 28.548 | 3.1241 | 47 | 6.2 |
| 31 | 28.716 | 3.1063 | 67 | 8.8 |
| 32 | 31.449 | 2.8422 | 45 | 5.9 |
| 33 | 32.729 | 2.734 | 72 | 9.5 |
| 34 | 35.727 | 2.5111 | 46 | 6.1 |
| 35 | 39.274 | 2.2921 | 46 | 6.1 |

The crystal form A of tromethamine salt of the compound of the present disclosure has an X-ray powder diffraction pattern substantially as shown in FIG. 1, a DSC pattern substantially as shown in FIG. 2, and a TGA pattern substantially as shown in FIG. 3.

In certain embodiments of the present disclosure, the crystal form is the crystal form B of tromethamine salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid. The X-ray powder diffraction pattern of the crystal form B of tromethamine salt has a characteristic peak at 2θ of 8.2±0.2°, or has a characteristic peak at 2θ of 10.1±0.2°, or has a characteristic peak at 2θ of 12.3±0.2°, or has a characteristic peak at 2θ of 14.4±0.2°, or has a characteristic peak at 2θ of 14.8±0.2°, or has a characteristic peak at 2θ of 16.0±0.2°, or has a characteristic peak at 2θ of 16.2±0.2°, or has a characteristic peak at 2θ of 17.5±0.2°, or has a characteristic peak at 2θ of 17.7±0.2°, or has a characteristic peak at 2θ of 18.3±0.2°, or has a characteristic peak at 2θ of 18.7±0.2°, or has a characteristic peak at 2θ of 19.7±0.2°, or has a characteristic peak at 2θ of 20.5±0.2°, or has a characteristic peak at 2θ of 20.9±0.2°, or has a characteristic peak at 2θ of 21.9±0.2°, or has a characteristic peak at 2θ of 22.1±0.2°, or has a characteristic peak at 2θ of 22.4±0.2°, or has a characteristic peak at 2θ of 24.7±0.2°; preferably, the X-ray powder diffraction pattern has characteristic peaks at any 2, 4, 6, 8, 10 or 12 of the above-mentioned 2θ.

In certain embodiments of the present disclosure, the X-ray powder diffraction pattern of the crystal form B of tromethamine salt has a characteristic peak at one or more of 2θ of 10.1±0.2°, 14.4±0.2°, 18.7±0.2° or 21.9±0.2°; preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can further have a characteristic peak at one or more of 2θ of 8.2±0.2°, 12.3±0.2°, 14.8±0.2°, 19.7±0.2°, 20.5±0.2° or 22.1±0.2°, preferably at 2, 3, 4 or 6 of the above-mentioned 2θ; for example:
10.1±0.2°, 14.4±0.2°, 18.7±0.2°;
10.1±0.2°, 14.4±0.2°, 18.7±0.2°, 21.9±0.2°;
8.2±0.2°, 14.4±0.2°, 18.7+0.2°, 21.9±0.2°;
10.1±0.2°, 12.3±0.2°, 14.4±0.2°, 18.7±0.2°;
10.1±0.2°, 14.4±0.2°, 14.8±0.2°, 18.7±0.2°;
10.1±0.2°, 14.4±0.2°, 18.7±0.2°, 19.7±0.2°, ;
10.1±0.2°, 12.3±0.2°, 14.4±0.2°, 21.9±0.2°;
10.1±0.2°, 14.4±0.2°, 14.8±0.2°, 18.7±0.2°;
8.2±0.2°, 12.3±0.2°, 14.4±0.2°, 18.7±0.2°;
10.1±0.2°, 12.3±0.2°, 14.4±0.2°, 18.7±0.2°, 21.9±0.2°;
8.2±0.2°, 12.3±0.2°, 14.4±0.2°, 18.7±0.2°, 21.9±0.2°;
10.1±0.2°, 12.3±0.2°, 14.4±0.2°, 14.8±0.2°, 18.7±0.2°;
10.1±0.2°, 14.4±0.2°, 14.8±0.2°, 18.7±0.2°, 21.9±0.2°;
10.1±0.2°, 14.4±0.2°, 14.8±0.2°, 18.7±0.2°, 19.7±0.2°;
8.2±0.2°, 10.1±0.2°, 12.3±0.2°, 14.4±0.2°, 21.9±0.2°;
10.1±0.2°, 14.4±0.2°, 14.8±0.2°, 18.7±0.2°, 21.9±0.2°;
8.2±0.2°, 12.3±0.2°, 14.4±0.2°, 14.8±0.2°, 18.7±0.2°;
10.1±0.2°, 12.3±0.2°, 14.4±0.2°, 14.8±0.2°, 18.7±0.2°, 21.9±0.2°;
8.2±0.2°, 12.3±0.2°, 14.4±0.2°, 18.7±0.2°, 21.9±0.2°, 22.1±0.2°;
10.1±0.2°, 12.3±0.2°, 14.4±0.2°, 14.8±0.2°, 18.7±0.2°, 19.7±0.2°;
10.1±0.2°, 14.4±0.2°, 14.8±0.2°, 18.7±0.2°, 21.9±0.2°, 22.1±0.2°;
10.1±0.2°, 14.4±0.2°, 14.8±0.2°, 18.7±0.2°, 19.7±0.2°, 22.1±0.2°;
8.2±0.2°, 10.1±0.2°, 12.3±0.2°, 14.4±0.2°, 14.8±0.2°, 21.9±0.2°;
10.1±0.2°, 14.4±0.2°, 14.8±0.2°, 18.7±0.2°, 21.9±0.2°, 22.1±0.2°;
8.2±0.2°, 12.3±0.2°, 14.4±0.2°, 14.8±0.2°, 18.7±0.2°, 22.1±0.2°;
more preferably, the X-ray powder diffraction pattern thereof optionally also has a diffraction peak at one or more of 2θ of 12.3±0.2°, 16.0±0.2°, 16.2±0.2°, 18.3±0.2°, 19.7±0.2° or 20.9±0.2°; preferably at any 2 to 4, or any 5 or 6 of the above-mentioned 2θ, and further preferably at any 4 or 6 of the above-mentioned 2θ; for example:
   12.3±0.2°, 16.0±0.2°, 16.2±0.2°, 18.3±0.2°;
   12.3±0.2°, 16.0±0.2°, 16.2±0.2°, 19.7±0.2°;
   12.3±0.2°, 16.0±0.2°, 16.2±0.2°, 20.9±0.2°;
   12.3±0.2°, 16.2±0.2°, 18.3±0.2°, 19.7±0.2°;
   12.3±0.2°, 16.0±0.2°, 16.2±0.2°, 18.3±0.2°, 19.7±0.2°;
   12.3±0.2°, 16.0±0.2°, 16.2±0.2°, 18.3±0.2°, 20.9±0.2°;
   16.0±0.2°, 16.2±0.2°, 18.3±0.2°, , 19.7±0.2°, 20.9±0.2°;
   12.3±0.2°, 16.0±0.2°, 16.2±0.2°, 18.3±0.2°, 19.7±0.2°, 20.9±0.2°;

In certain embodiments of the present disclosure, the X-ray powder diffraction pattern of the crystal form B of tromethamine salt has characteristic peaks at 2θ of 10.1±0.2° and 14.4±0.2°; preferably, the X-ray powder diffraction pattern further has characteristic peaks at 2θ of 18.7±0.2° and 21.9±0.2°; more preferably, the X-ray powder diffraction pattern further has characteristic peaks at 2θ of 8.2±0.2°, 14.8±0.2°, 20.5±0.2° and 22.1±0.2°; further preferably, the X-ray powder diffraction pattern further has characteristic peaks at 2θ of 12.3+0.2° and 19.7±0.2°; more further preferably, the X-ray powder diffraction pattern further has a characteristic peak at one or more of 16.0±0.2°, 16.2±0.2°, 18.3±0.2° and 20.9±0.2°.

In certain embodiments of the present disclosure, by using Cu-Kα radiation, the X-ray characteristic diffraction peaks represented by 2θ angles and interplanar spacing d values are shown in Table 2.

**Table 2**

| Serial No. | XRPD ray diffraction data of the crystal form B of tromethamine salt | | | |
|---|---|---|---|---|
| | 2θ (±0.2 °) | d value | Peak height | Proportion (I%) |
| 1 | 8.198 | 10.7767 | 551 | 50 |
| 2 | 10.059 | 8.7865 | 770 | 69.8 |
| 3 | 12.307 | 7.1859 | 305 | 27.7 |
| 4 | 14.397 | 6.1471 | 1103 | 100 |
| 5 | 14.847 | 5.9618 | 570 | 51.7 |
| 6 | 15.964 | 5.5472 | 172 | 15.6 |
| 7 | 16.163 | 5.4792 | 154 | 14 |
| 8 | 16.405 | 5.3991 | 79 | 7.2 |
| 9 | 17.198 | 5.1517 | 90 | 8.2 |
| 10 | 17.536 | 5.0531 | 111 | 10.1 |
| 11 | 17.721 | 5.0008 | 106 | 9.6 |
| 12 | 18.267 | 4.8525 | 150 | 13.6 |
| 13 | 18.679 | 4.7464 | 639 | 57.9 |
| 14 | 19.731 | 4.4958 | 274 | 24.8 |
| 15 | 20.542 | 4.3201 | 551 | 50 |
| 16 | 20.869 | 4.2532 | 249 | 22.6 |
| 17 | 21.881 | 4.0586 | 752 | 68.2 |
| 18 | 22.085 | 4.0216 | 556 | 50.4 |
| 19 | 22.362 | 3.9724 | 124 | 11.2 |
| 20 | 23.134 | 3.8415 | 92 | 8.3 |
| 21 | 23.51 | 3.7809 | 79 | 7.2 |
| 22 | 24.7 | 3.6014 | 149 | 13.5 |
| 23 | 26.219 | 3.3962 | 60 | 5.4 |
| 24 | 27.053 | 3.2933 | 71 | 6.4 |
| 25 | 29.789 | 2.9967 | 90 | 8.2 |
| 26 | 32.987 | 2.7132 | 50 | 4.5 |
| 27 | 33.377 | 2.6823 | 63 | 5.7 |
| 28 | 34.286 | 2.6132 | 44 | 4 |

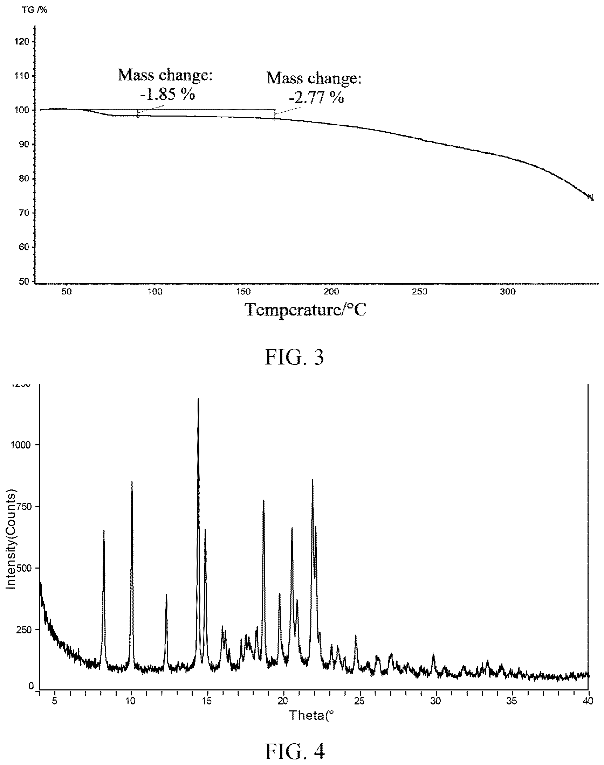

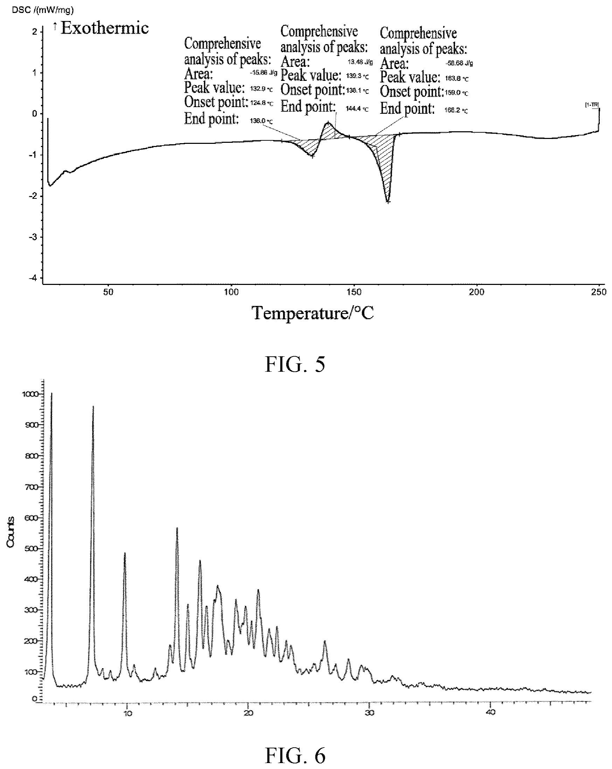

The crystal form B of tromethamine salt of the compound of the present disclosure has an X-ray powder diffraction pattern substantially as shown in FIG. 4, and a DSC pattern substantially as shown in FIG. 5.

In certain embodiments of the present disclosure, the crystal form is the crystal form C of tromethamine salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid. The X-ray powder diffraction pattern of the crystal form C of tromethamine salt has a characteristic peak at 2θ of 3.6±0.2°, or has a characteristic peak at 2θ of 7.1±0.2°, or has a characteristic peak at 2θ of 9.7±0.2°, or has a characteristic peak at 2θ of 10.6±0.2°, or has a characteristic peak at 2θ of 13.5±0.2°, or has a characteristic peak at 2θ of 14.1±0.2°, or has a characteristic peak at 2θ of 15.0±0.2°, or has a characteristic peak at 2θ of 16.0±0.2°, or has a characteristic peak at 2θ of 16.5±0.2°, or has a characteristic peak at 2θ of 17.1±0.2°, or has a characteristic peak at 2θ of 17.6±0.2°, or has a characteristic peak at 2θ of 19.0±0.2°, or has a characteristic peak at 2θ of 19.7±0.2°, or has a characteristic peak at 2θ of 20.8±0.2°, or has a characteristic peak at 2θ of 21.8±0.2°, or has a characteristic peak at 2θ of 22.3±0.2°, or has a characteristic peak at 2θ of 23.1±0.2°, or has a characteristic peak at 2θ of 26.4±0.2°, or has a characteristic peak at 2θ of 28.3±0.2°; preferably, the X-ray powder diffraction pattern has characteristic peaks at any 2, 4, 6, 8, 10 or 12 of the above-mentioned 2θ.
the X-ray powder diffraction pattern of the crystal form C of tromethamine salt has a characteristic peak at one or more of 2θ of 3.6+0.2°, 7.1±0.2°, 9.7±0.2°, 14.1±0.2° or 16.0±0.2°; preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can further have a characteristic peak at one or more of 2θ of 15.0±0.2°, 16.5±0.2°, 17.1±0.2°, 17.6±0.2°, 19.7±0.2° or 20.8±0.2°, preferably at 2, 3, 4 or 6 of the above-mentioned 2θ;
3.6±0.2°, 7.1±0.2°, 9.7±0.2°;
3.6±0.2°, 7.1±0.2°, 9.7±0.2°, 14.1±0.2°;
3.6±0.2°, 7.1±0.2°, 9.7±0.2°, 14.1±0.2°;
3.6±0.2°, 7.1±0.2°, 9.7±0.2°, 15.0±0.2°;
3.6±0.2°, 7.1±0.2°, 9.7±0.2°, 16.5+0.2°;
3.6±0.2°, 7.1±0.2°, 9.7±0.2°, 17.1±0.2°;
3.6±0.2°, 7.1±0.2°, 14.1±0.2°, 17.6±0.2°;
3.6±0.2°, 7.1±0.2°, 17.6±0.2°, 19.7±0.2°;
9.7±0.2°, 14.1±0.2°, 16.0±0.2°, 17.1±0.2°;
3.6±0.2°, 7.1±0.2°, 9.7±0.2°, 14.1±0.2°, 16.0±0.2°;
3.6±0.2°, 7.1±0.2°, 9.7±0.2°, 14.1±0.2°, 15.0±0.2°;
3.6±0.2°, 7.1±0.2°, 9.7±0.2°, 14.1±0.2°, 16.5±0.2°;
3.6±0.2°, 7.1±0.2°, 9.7±0.2°, 14.1±0.2°, 17.1±0.2°;
3.6±0.2°, 7.1±0.2°, 9.7±0.2°, 14.1±0.2°, 17.6±0.2°;
3.6±0.2°, 7.1±0.2°, 9.7±0.2°, 17.6±0.2°, 19.7±0.2°;
3.6±0.2°, 7.1±0.2°, 14.1±0.2°, 17.1±0.2°, 17.6±0.2°;
3.6±0.2°, 9.7±0.2°, 14.1±0.2°, 17.1±0.2°, 17.6±0.2°;
3.6±0.2°, 7.1±0.2°, 9.7±0.2°, 14.1±0.2°, 16.0±0.2°, 17.1±0.2°;
3.6±0.2°, 7.1±0.2°, 9.7±0.2°, 14.1±0.2°, 15.0±0.2°, 17.1±0.2°;
3.6±0.2°, 7.1±0.2°, 9.7±0.2°, 14.1±0.2°, 16.5±0.2°, 17.6±0.2°;
3.6±0.2°, 7.1±0.2°, 9.7±0.2°, 14.1±0.2°, 17.6±0.2°, 19.7±0.2°;
3.6±0.2°, 7.1±0.2°, 9.7±0.2°, 15.0±0.2°, 17.6±0.2°, 19.7+0.2°;
3.6±0.2°, 9.7±0.2°, 14.1±0.2°, 15.0±0.2°, 16.0±0.2°, 17.1±0.2°;
3.6±0.2°, 7.1±0.2°, 14.1±0.2°, 15.0±0.2°, 17.1±0.2°, 17.6±0.2°;
9.7±0.2°, 14.1±0.2°, 15.0±0.2°, 16.0±0.2°, 17.1±0.2°, 20.8±0.2°;
3.6±0.2°, 7.1±0.2°, 9.7±0.2°, 14.1±0.2°, 16.0±0.2°, 17.1±0.2°, 20.8±0.2°;
3.6±0.2°, 7.1±0.2°, 9.7±0.2°, 14.1±0.2°, 15.0±0.2°, 17.1±0.2°, 19.7+0.2°;
3.6±0.2°, 7.1±0.2°, 9.7±0.2°, 14.1±0.2°, 16.5±0.2°, 17.6±0.2°, 19.7+0.2°;
3.6±0.2°, 7.1±0.2°, 9.7±0.2°, 14.1±0.2°, 17.1±0.2°, 17.6±0.2°, 19.7±0.2°;
3.6±0.2°, 7.1±0.2°, 9.7±0.2°, 14.1±0.2°, 17.6±0.2°, 19.7±0.2°, 20.8±0.2°;
3.6±0.2°, 7.1±0.2°, 9.7±0.2°, 15.0±0.2°, 17.6±0.2°, 19.7±0.2°, 20.8±0.2°;
3.6±0.2°, 7.1±0.2°, 14.1±0.2°, 15.0±0.2°, 17.1±0.2°, 17.6±0.2°, 20.8±0.2°;
more preferably, the X-ray powder diffraction pattern thereof optionally also has a diffraction peak at one or more of 2θ of 13.5±0.2°, 17.1±0.2°, 19.0±0.2°, 19.7±0.2°, 21.8±0.2° or 26.4± 0.2°; preferably at any 2 to 4, or any 5 or 6 of the above-mentioned 2θ, and further preferably at any 4 or 6 of the above-mentioned 2θ; for example:
   13.5±0.2°, 17.1±0.2°, 19.0±0.2°, 19.7±0.2°;
   13.5±0.2°, 17.1±0.2°, 19.0±0.2°, 21.8±0.2°;
   13.5±0.2°, 17.1±0.2°, 19.0±0.2°, 26.4±0.2°;
   17.1±0.2°, 19.0±0.2°, 21.8+0.2°, 26.4±0.2°;
   13.5±0.2°, 17.1±0.2°, 19.0±0.2°, 21.8±0.2°, 26.4±0.2°;
   13.5±0.2°, 17.1±0.2°, 19.0±0.2°, 19.7±0.2°, 21.8±0.2°;
   17.1±0.2°, 19.0±0.2°, 19.7±0.2°, 21.8±0.2°, 26.4±0.2°;
   13.5±0.2°, 17.1±0.2°, 19.0±0.2°, 19.7±0.2°, 21.8±0.2°, 26.4±0.2°;

In certain embodiments of the present disclosure, the X-ray powder diffraction pattern of the crystal form C of tromethamine salt has characteristic peaks at 2θ of 3.6±0.2° and 7.1±0.2°; preferably, the X-ray powder diffraction pattern further has characteristic peaks at 2θ of 9.7±0.2° and 14.1±0.2°; more preferably, the X-ray powder diffraction pattern further has characteristic peaks at 2θ of 15.0±0.2°, 16.0±0.2°, 16.5±0.2°, 17.6±0.2° and 20.8±0.2°; further preferably, the X-ray powder diffraction pattern further has characteristic peaks at 2θ of 17.1±0.2° and 19.7±0.2°; more further preferably, the X-ray powder diffraction pattern further has a characteristic peak at one or more of 13.5±0.2°, 19.0±0.2°, 21.8±0.2° and 26.4±0.2°;

In certain embodiments of the present disclosure, by using Cu-Kα radiation, the X-ray characteristic diffraction peaks represented by 2θ angles and interplanar spacing d values are shown in Table 3.

**Table 3**

| XRPD ray diffraction data of the crystal form C of tromethamine salt | |
|---|---|
| Serial No. | 2θ (±0.2 °) |
| 1 | 3.638 |
| 2 | 7.059 |
| 3 | 9.72 |
| 4 | 10.57 |
| 5 | 13.506 |
| 6 | 14.077 |
| 7 | 14.951 |
| 8 | 15.964 |
| 9 | 16.512 |
| 10 | 17.138 |
| 11 | 17.605 |
| 12 | 18.942 |
| 13 | 19.672 |
| 14 | 20.846 |
| 15 | 21.781 |
| 16 | 22.327 |
| 17 | 23.121 |
| 18 | 23.603 |
| 19 | 26.361 |
| 20 | 28.328 |
| 21 | 29.445 |
| 22 | 29.746 |
| 23 | 31.937 |
| 24 | 32.498 |
| 25 | 35.67 |

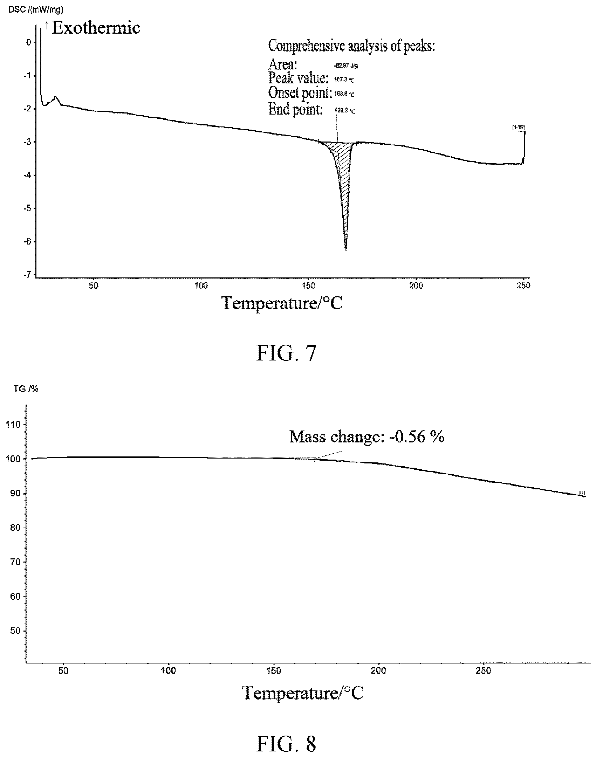

The crystal form C of tromethamine salt of the compound of the present disclosure has an X-ray powder diffraction pattern substantially as shown in FIG. 6, a DSC pattern substantially as shown in FIG. 7, and a TGA pattern substantially as shown in FIG. 8.

In certain embodiments of the present disclosure, the crystal form C of tromethamine salt is an anhydrate crystal form.

In certain embodiments of the present disclosure, the crystal form is the crystal form D of tromethamine salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid. The X-ray powder diffraction pattern of the crystal form D of tromethamine salt has a characteristic peak at 2θ of 7.4±0.2°, or has a characteristic peak at 2θ of 7.7±0.2°, or has a characteristic peak at 2θ of 9.8±0.2°, or has a characteristic peak at 2θ of 10.8±0.2°, or has a characteristic peak at 2θ of 11.6±0.2°, or has a characteristic peak at 2θ of 13.1±0.2°, or has a characteristic peak at 2θ of 14.0±0.2°, or has a characteristic peak at 2θ of 14.5±0.2°, or has a characteristic peak at 2θ of 15.1±0.2°, or has a characteristic peak at 2θ of 15.4±0.2°, or has a characteristic peak at 2θ of 15.8±0.2°, or has a characteristic peak at 2θ of 17.9±0.2°, or has a characteristic peak at 2θ of 18.8±0.2°, or has a characteristic peak at 2θ of 19.3±0.2°, or has a characteristic peak at 2θ of 20.0±0.2°, or has a characteristic peak at 2θ of 20.5±0.2°, or has a characteristic peak at 2θ of 21.2±0.2°, or has a characteristic peak at 2θ of 21.8±0.2°, or has a characteristic peak at 2θ of 23.3±0.2°; preferably, the X-ray powder diffraction pattern has characteristic peaks at any 2, 4, 6, 8, 10 or 12 of the above-mentioned 2θ.

In certain embodiments of the present disclosure, the X-ray powder diffraction pattern of the crystal form D of tromethamine salt has a characteristic peak at one or more of 2θ of 7.4±0.2°, 9.8±0.2°, 13.1±0.2° or 14.0±0.2°; preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can further have a characteristic peak at one or more of 2θ of 7.7±0.2°, 15.1±0.2°, 17.9±0.2°, 18.8±0.2°, 19.3±0.2° or 20.0±0.2°, preferably at 2, 3, 4 or 6 of the above-mentioned 2θ; for example:
7.4±0.2°, 9.8±0.2°, 13.1±0.2°;
7.4±0.2°, 9.8±0.2°, 13.1±0.2°, 14.0±0.2°;
7.4±0.2°, 7.7±0.2°, 9.8±0.2°, 13.1±0.2°;
7.4±0.2°, 9.8±0.2°, 13.1±0.2°, 14.0±0.2°;
7.4±0.2°, 9.8±0.2°, 13.1±0.2°, 14.0±0.2°;
7.4±0.2°, 9.8±0.2°, 13.1±0.2°, 14.0±0.2°;
7.4±0.2°, 13.1±0.2°, 14.0±0.2°, 17.9±0.2°;
9.8±0.2°, 13.1±0.2°, 14.0±0.2°, 15.1±0.2°;
7.7±0.2°, 9.8±0.2°, 14.0±0.2°, 15.1±0.2°;
7.4±0.2°, 9.8±0.2°, 13.1±0.2°, 14.0±0.2°, 19.3±0.2°;
7.4±0.2°, 7.7±0.2°, 9.8±0.2°, 13.1±0.2°, 14.0±0.2°;
7.4±0.2°, 9.8±0.2°, 13.1±0.2°, 14.0±0.2°, 15.1±0.2°;
7.4±0.2°, 9.8±0.2°, 13.1±0.2°, 14.0±0.2°, 17.9±0.2°;
7.4±0.2°, 9.8±0.2°, 13.1±0.2°, 14.0±0.2°, 18.8±0.2°;
7.4±0.2°, 13.1±0.2°, 14.0±0.2°, 17.9±0.2°, 18.8±0.2°;
9.8±0.2°, 13.1±0.2°, 14.0±0.2°, 15.1±0.2°, 17.9±0.2°;
7.7±0.2°, 9.8±0.2°, 14.0±0.2°, 15.1±0.2°, 17.9±0.2°;
7.4±0.2°, 9.8±0.2°, 13.1±0.2°, 14.0±0.2°, 19.3±0.2°, 20.0±0.2°;
7.4±0.2°, 7.7±0.2°, 9.8±0.2°, 13.1±0.2°, 14.0±0.2°, 15.1±0.2°;
7.4±0.2°, 9.8±0.2°, 13.1±0.2°, 14.0±0.2°, 15.1±0.2°, 18.8±0.2°;
7.4±0.2°, 9.8±0.2°, 13.1±0.2°, 14.0±0.2°, 17.9±0.2°, 18.8±0.2°;
7.4±0.2°, 9.8±0.2°, 13.1±0.2°, 14.0±0.2°, 18.8±0.2°, 20.0±0.2°;
7.4±0.2°, 13.1±0.2°, 14.0±0.2°, 17.9±0.2°, 18.8±0.2°, 20.0±0.2°;
9.8±0.2°, 13.1±0.2°, 14.0±0.2°, 15.1±0.2°, 17.9+0.2°, 18.8±0.2°;
7.7±0.2°, 9.8±0.2°, 14.0±0.2°, 15.1±0.2°, 17.9±0.2°, 20.0±0.2°;
more preferably, the X-ray powder diffraction pattern thereof optionally also has a diffraction peak at one or more of 2θ of 14.5±0.2°, 15.4±0.2°, 15.8+0.2°, 17.9±0.2°, 19.3±0.2° or 21.2± 0.2°; preferably at any 2 to 4, or any 5 or 6 of the above-mentioned 2θ, and further preferably at any 4 or 6 of the above-mentioned 2θ; for example:
   14.5±0.2°, 15.4±0.2°, 15.8±0.2°, 17.9±0.2°;
   14.5±0.2°, 15.4±0.2°, 15.8±0.2°, 19.3±0.2°;
   14.5±0.2°, 15.4±0.2°, 15.8+0.2°, 21.2±0.2°;
   14.5±0.2°, 15.8±0.2°, 17.9±0.2°, 21.2±0.2°;
   14.5±0.2°, 15.4±0.2°, 15.8±0.2°, 17.9±0.2°, 19.3±0.2°;
   14.5±0.2°, 15.4±0.2°, 15.8±0.2°, 19.3+0.2°, 21.2±0.2°;
   14.5±0.2°, 15.4±0.2°, 15.8±0.2°, 17.9±0.2°, 21.2±0.2°;
   14.5±0.2°, 15.4±0.2°, 15.8±0.2°, 17.9±0.2°, 19.3±0.2°, 21.2±0.2°;

In certain embodiments of the present disclosure, the X-ray powder diffraction pattern of the crystal form D of tromethamine salt has characteristic peaks at 2θ of 9.8±0.2° and 14.0±0.2°; preferably, the X-ray powder diffraction pattern further has characteristic peaks at 2θ of 7.4±0.2° and 13.1±0.2°; more preferably, the X-ray powder diffraction pattern further has characteristic peaks at 2θ of 7.7±0.2°, 15.1±0.2°, 18.8±0.2° and 20.0±0.2°; further preferably, the X-ray powder diffraction pattern further has characteristic peaks at 2θ of 17.9±0.2° and 19.3±0.2°; more further preferably, the X-ray powder diffraction pattern further has a characteristic peak at one or more of 14.5±0.2°, 15.4±0.2°, 15.8±0.2° and 21.2±0.2°.

In certain embodiments of the present disclosure, by using Cu-Kα radiation, the X-ray characteristic diffraction peaks represented by 2θ angles and interplanar spacing d values are shown in Table 4.

**Table 4**

| Serial No. | XRPD ray diffraction data of the crystal form D of tromethamine salt | | | |
|---|---|---|---|---|
| | 2θ (±0.2 °) | d value | Peak height | Proportion (I%) |
| 1 | 7.351 | 12.0165 | 238 | 51.6 |
| 2 | 7.746 | 11.4043 | 214 | 46.4 |
| 3 | 9.835 | 8.9855 | 318 | 69 |
| 4 | 10.773 | 8.2057 | 128 | 27.8 |
| 5 | 11.582 | 7.6342 | 107 | 23.2 |
| 6 | 13.144 | 6.7301 | 280 | 60.7 |
| 7 | 13.978 | 6.3305 | 461 | 100 |
| 8 | 14.542 | 6.0863 | 199 | 43.2 |
| 9 | 14.809 | 5.9769 | 104 | 22.6 |
| 10 | 15.051 | 5.8816 | 225 | 48.8 |
| 11 | 15.437 | 5.7354 | 151 | 32.8 |
| 12 | 15.778 | 5.6121 | 216 | 46.9 |
| 13 | 16.406 | 5.3986 | 98 | 21.3 |
| 14 | 17.87 | 4.9594 | 229 | 49.7 |
| 15 | 18.758 | 4.7267 | 271 | 58.8 |
| 16 | 19.346 | 4.5844 | 220 | 47.7 |
| 17 | 19.971 | 4.4423 | 264 | 57.3 |
| 18 | 20.511 | 4.3265 | 201 | 43.6 |
| 19 | 21.235 | 4.1806 | 215 | 46.6 |
| 20 | 21.782 | 4.0769 | 166 | 36 |
| 21 | 22.653 | 3.922 | 125 | 27.1 |
| 22 | 23.323 | 3.8108 | 176 | 38.2 |
| 23 | 23.664 | 3.7567 | 111 | 24.1 |
| 24 | 24.587 | 3.6178 | 65 | 14.1 |
| 25 | 25.138 | 3.5396 | 116 | 25.2 |
| 26 | 25.675 | 3.4668 | 117 | 25.4 |
| 27 | 26.424 | 3.3702 | 76 | 16.5 |
| 28 | 27.418 | 3.2503 | 65 | 14.1 |
| 29 | 27.735 | 3.2138 | 55 | 11.9 |
| 30 | 28.78 | 3.0994 | 51 | 11.1 |

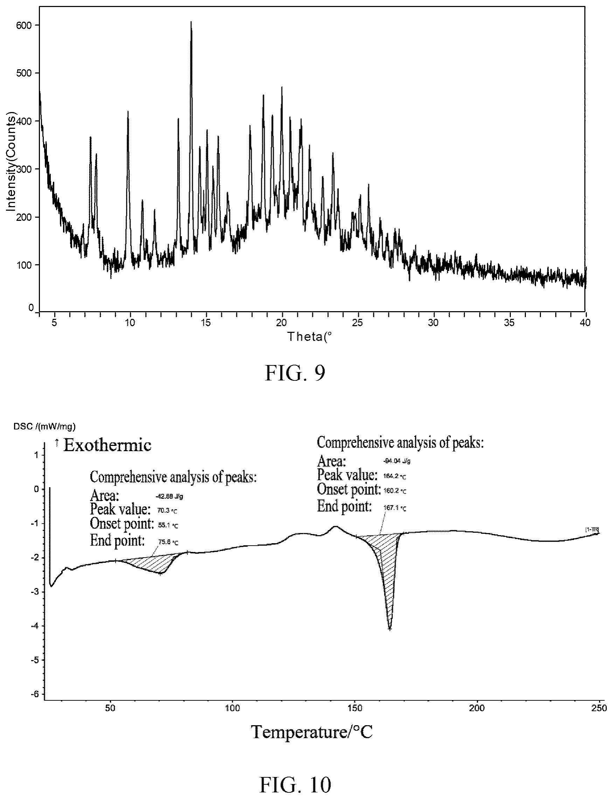

The crystal form D of tromethamine salt of the compound of the present disclosure has an X-ray powder diffraction pattern substantially as shown in FIG. 9, and a DSC pattern substantially as shown in FIG. 10.

In certain embodiments of the present disclosure, the crystal form is the crystal form E of tromethamine salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid. The X-ray powder diffraction pattern of the crystal form E of tromethamine salt has a characteristic peak at 2θ of 4.3±0.2°, or has a characteristic peak at 2θ of 6.3±0.2°, or has a characteristic peak at 2θ of 8.6±0.2°, or has a characteristic peak at 2θ of 9.3±0.2°, or has a characteristic peak at 2θ of 13.6±0.2°, or has a characteristic peak at 2θ of 14.1±0.2°, or has a characteristic peak at 2θ of 17.7+0.2°, or has a characteristic peak at 2θ of 18.5±0.2°, or has a characteristic peak at 2θ of 18.9±0.2°, or has a characteristic peak at 2θ of 20.2±0.2°, or has a characteristic peak at 2θ of 20.5±0.2°, or has a characteristic peak at 2θ of 21.4±0.2°, or has a characteristic peak at 2θ of 21.9±0.2°, or has a characteristic peak at 2θ of 22.4±0.2°, or has a characteristic peak at 2θ of 23.4±0.2°, or has a characteristic peak at 2θ of 23.9±0.2°, or has a characteristic peak at 2θ of 25.2±0.2°; preferably, the X-ray powder diffraction pattern has characteristic peaks at any 2, 4, 6, 8, 10 or 12 of the above-mentioned 2θ.

In certain embodiments of the present disclosure, the X-ray powder diffraction pattern of the crystal form E of tromethamine salt has a characteristic peak at one or more of 2θ of 4.3±0.2°, 6.3±0.2°, 13.6±0.2° or 18.9±0.2°; preferably at 2 to 4 of the above-mentioned 2θ, more preferably at 3 or 4 of the above-mentioned 2θ, and most preferably at 4 of the above-mentioned 2θ; optionally, further, the X-ray powder diffraction pattern can further have a characteristic peak at one or more of 2θ of 8.6±0.2°, 14.1±0.2°, 17.7±0.2°, 20.2±0.2°, 20.5±0.2° or 22.4±0.2°, preferably at 2, 3, 4 or 6 of the above-mentioned 2θ; for example:
4.3±0.2°, 6.3±0.2°, 13.6±0.2°;
4.3±0.2°, 6.3±0.2°, 13.6±0.2°, 18.9±0.2°;
4.3±0.2°, 6.3±0.2°, 8.6±0.2°, 13.6±0.2°;
4.3±0.2°, 6.3±0.2°, 13.6±0.2°, 14.1±0.2°;
4.3±0.2°, 6.3±0.2°, 13.6±0.2°, 17.7±0.2°;
4.3±0.2°, 6.3±0.2°, 13.6±0.2°, 18.9±0.2°;
4.3±0.2°, 8.6±0.2°, 13.6±0.2°, 14.1±0.2°;
6.3±0.2°, 8.6±0.2°, 13.6±0.2°, 14.1±0.2°;
6.3±0.2°, 13.6±0.2°, 14.1±0.2°, 17.7±0.2°;
4.3±0.2°, 6.3±0.2°, 13.6±0.2°, 18.9±0.2°, 20.2±0.2°;
4.3±0.2°, 6.3±0.2°, 8.6±0.2°, 13.6±0.2°, 18.9±0.2°;
4.3±0.2°, 6.3±0.2°, 13.6±0.2°, 14.1±0.2°, 18.9±0.2°;
4.3±0.2°, 6.3±0.2°, 13.6±0.2°, 17.7±0.2°, 18.9±0.2°;
4.3±0.2°, 6.3±0.2°, 13.6±0.2°, 18.9±0.2°, 20.2±0.2°;
4.3±0.2°, 8.6±0.2°, 13.6±0.2°, 14.1±0.2°, 18.9±0.2°;
6.3±0.2°, 8.6±0.2°, 13.6±0.2°, 14.1±0.2°, 18.9±0.2°, 20.2±0.2°;
6.3±0.2°, 13.6±0.2°, 14.1±0.2°, 17.7+0.2°, 18.9±0.2°, 20.2±0.2°;
4.3±0.2°, 6.3±0.2°, 13.6±0.2°, 18.9±0.2°, 20.2±0.2°, 20.5±0.2°;
4.3±0.2°, 6.3±0.2°, 8.6±0.2°, 13.6±0.2°, 18.9±0.2°, 20.5±0.2°;
4.3±0.2°, 6.3±0.2°, 13.6±0.2°, 14.1±0.2°, 18.9±0.2°, 20.5±0.2°;
4.3±0.2°, 6.3±0.2°, 13.6±0.2°, 17.7±0.2°, 18.9±0.2°, 20.2±0.2°;
4.3±0.2°, 6.3±0.2°, 13.6±0.2°, 18.9±0.2°, 20.2±0.2°, 20.5±0.2°;
4.3±0.2°, 8.6±0.2°, 13.6±0.2°, 14.1±0.2°, 18.9±0.2°, 20.2±0.2°;
6.3±0.2°, 8.6±0.2°, 13.6±0.2°, 14.1±0.2°, 18.9±0.2°, 22.4±0.2°;
6.3±0.2°, 13.6±0.2°, 14.1±0.2°, 17.7+0.2°, 18.9±0.2°, 22.4±0.2°;
more preferably, the X-ray powder diffraction pattern thereof optionally also has a diffraction peak at one or more of 2θ of 9.3±0.2°, 14.1±0.2°, 18.5±0.2°, 20.5±0.2°, 21.4±0.2° or 21.9± 0.2°; preferably at any 2 to 4, or any 5 or 6 of the above-mentioned 2θ, and further preferably at any 4 or 6 of the above-mentioned 2θ; for example:
   9.3±0.2°, 14.1±0.2°, 18.5±0.2°, 20.5±0.2°;
   9.3±0.2°, 14.1±0.2°, 18.5±0.2°, 21.4±0.2°;
   9.3±0.2°, 14.1±0.2°, 18.5±0.2°, 21.9±0.2°;
   9.3±0.2°, 18.5±0.2°, 20.5±0.2°, 21.4±0.2°;
   9.3±0.2°, 14.1±0.2°, 18.5±0.2°, 20.5±0.2°, 21.4±0.2°;
   9.3±0.2°, 14.1±0.2°, 18.5±0.2°, 21.4±0.2°, 21.9±0.2°;
   14.1±0.2°, 18.5±0.2°, 20.5±0.2°, 21.4±0.2°, 21.9±0.2°;
   9.3±0.2°, 14.1±0.2°, 18.5±0.2°, 20.5±0.2°, 21.4±0.2°, 21.9±0.2°;

In certain embodiments of the present disclosure, the X-ray powder diffraction pattern of the crystal form E of tromethamine salt has characteristic peaks at 2θ of 6.3±0.2° and 13.6±0.2°; preferably, the X-ray powder diffraction pattern further has characteristic peaks at 2θ of 4.3±0.2° and 18.9±0.2°; more preferably, the X-ray powder diffraction pattern further has characteristic peaks at 2θ of 8.6±0.2°, 17.7±0.2°, 20.2±0.2° and 22.4±0.2°; further preferably, the X-ray powder diffraction pattern further has characteristic peaks at 2θ of 14.1±0.2° and 20.5±0.2°; more further preferably, the X-ray powder diffraction pattern further has a characteristic peak at one or more of 9.3±0.2°, 18.5±0.2°, 21.4±0.2° and 21.9±0.2°.

In certain embodiments of the present disclosure, by using Cu-Kα radiation, the X-ray characteristic diffraction peaks represented by 2θ angles and interplanar spacing d values are shown in Table 5.

**Table 5**

| Serial No. | XRPD ray diffraction data of the crystal form E of tromethamine salt | | | |
|---|---|---|---|---|
| | 2θ (±0.2 °) | d value | Peak height | Proportion (I%) |
| 1 | 4.284 | 20.6084 | 249 | 90.2 |
| 2 | 6.315 | 13.9837 | 268 | 97.1 |
| 3 | 8.579 | 10.2983 | 110 | 39.9 |
| 4 | 9.267 | 9.5358 | 61 | 22.1 |
| 5 | 13.648 | 6.4829 | 276 | 100 |
| 6 | 14.094 | 6.2787 | 95 | 34.4 |
| 7 | 17.722 | 5.0006 | 131 | 47.5 |
| 8 | 18.515 | 4.7882 | 99 | 35.9 |
| 9 | 18.859 | 4.7016 | 192 | 69.6 |
| 10 | 20.177 | 4.3974 | 172 | 62.3 |
| 11 | 20.503 | 4.3281 | 109 | 39.5 |
| 12 | 21.41 | 4.1468 | 85 | 30.8 |
| 13 | 21.878 | 4.0591 | 81 | 29.3 |
| 14 | 22.448 | 3.9573 | 175 | 63.4 |
| 15 | 23.383 | 3.8012 | 88 | 31.9 |
| 16 | 23.872 | 3.7244 | 104 | 37.7 |
| 17 | 25.167 | 3.5357 | 57 | 20.7 |

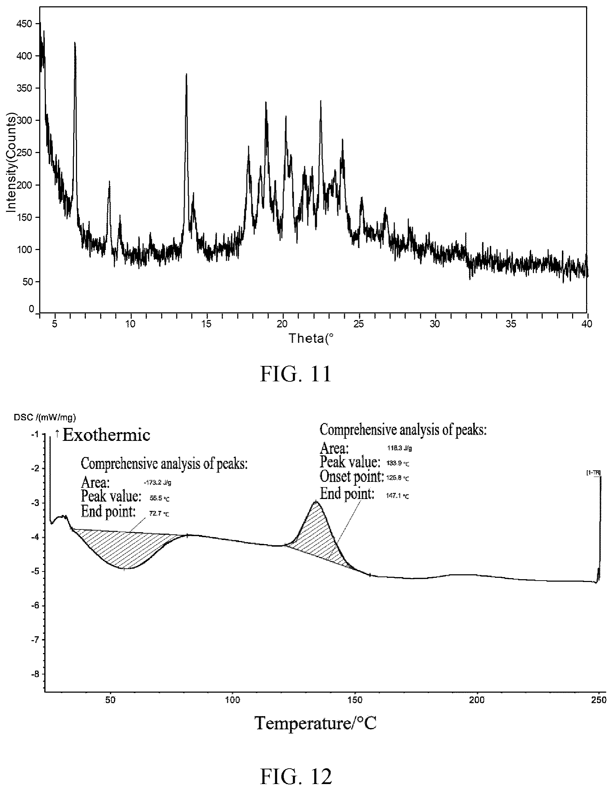

The crystal form E of tromethamine salt of the compound of the present disclosure has an X-ray powder diffraction pattern substantially as shown in FIG. 11, and a DSC pattern substantially as shown in FIG. 12.

In a further preferred embodiment of the present disclosure, the above-mentioned crystal form is a solvent-containing crystal form, wherein the solvent is selected from water, methanol, acetone, ethyl acetate, acetonitrile, ethanol, 88% acetone, 2-methyl-tetrahydrofuran, dichloromethane, 1,4-dioxane, benzene, toluene, isopropanol, n-butanol, isobutanol, N,N-dimethylformamide, N,N-dimethylacetamide, dimethyl sulfoxide, n-propanol, tert-butanol, 2-butanone, 3-pentanone, n-heptane, ethyl formate, isopropyl acetate, cyclohexane, methyl tert-butyl ether or isopropyl ether.

In a further preferred embodiment of the present disclosure, wherein the number of the solvents is 0.2-3, preferably 0.2, 0.5, 1, 1.5, 2, 2.5 or 3, more preferably 0.5, 1, 2 or 3.

The present disclosure further provides a method for preparing the compound as represented by general formula (I) or the stereoisomer and base salt thereof, which specifically comprises the following steps:
1) weighing an appropriate amount of a free acid and dissolving the free acid in a good solvent;
2) weighing an appropriate amount of a counter-ion base and dissolving the counter-ion base in an organic solvent; wherein the amount of the counter-ion base is preferably 1.0 to 1.5 equivalents;
3) combining the above two solutions, and stirring the combined solution to allow precipitation, or dropwise adding a poor solvent followed by the stirring to allow precipitation; and
4) subjecting the mixture to rapid centrifugation or standing to evaporate to dryness so as to obtain a target product;
wherein:
the good solvent is selected from acetone, tetrahydrofuran, ethyl formate, ethyl acetate, 2-methyl-tetrahydrofuran, 2-butanone, n-butanol, 1,4-dioxane, isobutanol, N,N-dimethylformamide, N,N-dimethylacetamide, n-propanol or tert-butanol, preferably 2-methyl-tetrahydrofuran, ethyl acetate, 2-butanone, acetone or ethyl formate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyl-tetrahydrofuran, 2-butanone, 3-pentanone, heptane, methyl tert-butyl ether, isopropyl ether, 1,4-dioxane, tert-butanol, or N,N-dimethylformamide; preferably methanol, ethanol, or acetonitrile; the above good solvent and organic solution must be miscible when used;
the poor solvent is selected from heptane, methyl tert-butyl ether, cyclohexane, toluene, isopropyl ether, and ethyl acetate; preferably methyl tert-butyl ether and isopropyl ether;
the counter-ion base is an organic base or an inorganic base, and the organic base is selected from diethylamine, diethanolamine, triethylamine, triethanolamine, choline hydroxide, tromethamine, meglumine, N-hydroxyethylmorpholine, piperazine, N-hydroxyethylpyrrolidine, N,N-dibenzylethylenediamine, 2-diethylaminoethanol, ethanolamine, betaine, L-arginine, lysine, phenethylbenzylamine, benzathine penicillin, dimethylaminoethanol, imidazole or a mixture thereof, preferably, diethylamine, diethanolamine, triethylamine, triethanolamine, choline hydroxide, tromethamine, or a mixture thereof; further preferably, tromethamine; the inorganic base is selected from sodium hydroxide, potassium hydroxide, calcium hydroxide, magnesium hydroxide, zinc hydroxide, ammonia water or a mixture thereof, preferably sodium hydroxide, potassium hydroxide, calcium hydroxide, magnesium hydroxide or a mixture thereof.

The present disclosure further provides a method for preparing the compound as represented by general formula (I) or the stereoisomer and base salt thereof, which specifically comprises the following steps:
1) weighing an appropriate amount of a free acid, and suspending the free acid with a poor solvent;
2) weighing an appropriate amount of a counter-ion base and dissolving the counter-ion base in an organic solvent; wherein the amount of the counter-ion base is preferably 1.0 to 1.5 equivalents;
3) combining the above two solutions and stirring the combined solution to allow dissolution, followed by continue stirring;
4) subjecting the mixture to rapid centrifugation or standing to evaporate to dryness so as to obtain a target product;
wherein:
the poor solvent is selected from ethanol, ethyl acetate, ethyl formate, isopropanol, isopropyl acetate, methyl tert-butyl ether, dichloromethane, methanol, acetonitrile, chlorobenzene, benzene, toluene, n-butanol, isobutanol or 3-pentanone; preferably, ethanol, ethyl acetate, isopropanol and isopropyl acetate.
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyltetrahydrofuran, 2-butanone, 3-pentanone, heptane, methyl tert-butyl ether, isopropyl ether, 1,4-dioxane, tert-butanol, or N,N-dimethylformamide; preferably methanol, ethanol, or acetonitrile; the above good solvent and organic solution must be miscible when used;
the counter-ion base is an organic base or an inorganic base, and the organic base is selected from diethylamine, diethanolamine, triethylamine, triethanolamine, choline hydroxide, tromethamine, meglumine, N-hydroxyethylmorpholine, piperazine, N-hydroxyethylpyrrolidine, N,N-dibenzylethylenediamine, 2-diethylaminoethanol, ethanolamine, betaine, L-arginine, lysine, phenethylbenzylamine, benzathine penicillin, dimethylaminoethanol, imidazole or a mixture thereof, preferably, diethylamine, diethanolamine, triethylamine, triethanolamine, choline hydroxide, tromethamine, or a mixture thereof; further preferably, tromethamine; the inorganic base is selected from sodium hydroxide, potassium hydroxide, calcium hydroxide, magnesium hydroxide, zinc hydroxide, ammonia water or a mixture thereof, preferably sodium hydroxide, potassium hydroxide, calcium hydroxide, magnesium hydroxide or a mixture thereof.

The present disclosure further provides a method for preparing the compound as represented by general formula (I) or the stereoisomer and base salt thereof, which specifically comprises the following steps:
1) weighing an appropriate amount of the base salt of the compound, and suspending the base salt with a poor solvent preferably at a suspension density of 50-200 mg/mL;
2) shaking the obtained suspension at a certain temperature for a certain time, preferably at a temperature of 25-50°C for preferably 1-15 days;
3) quickly centrifuging the suspension obtained above, removing a supernatant, placing a remaining solid in a vacuum drying oven and drying to a constant weight to afford a target product;
wherein:
the poor solvent is selected from dichloromethane, 1,4-dioxane, acetonitrile, chlorobenzene, benzene, toluene, acetone, ethyl acetate, water, 88% acetone, isopropyl acetate, 3-pentanone, ethyl formate, tetrahydrofuran, 2-methyltetrahydrofuran, isopropanol, n-butanol, isobutanol, n-propanol, methyl tert-butyl ether, n-heptane, tert-butanol or 2-butanone.

Another objective of the present disclosure is to provide a pharmaceutical composition comprising a therapeutically-effective amount of the above-mentioned compound or a stereoisomer and salt thereof, and one or more pharmaceutically acceptable carriers, diluents or excipients.

In the embodiments provided by the present disclosure, the pharmaceutical composition comprises a therapeutically effective amount of the base salt of the crystal form thereof, wherein the therapeutically effective amount is selected from 0.0001-99%, 0.0001-95%, 0.0001-90%, 0.0001-85%, 0.0001-80%, 0.0001-75%, 0.0001-70%, 0.001-60%, 0.001-55%, 0.01-50%, 0.01-40%, 0.01-30%, 0.01-20%, 0.01-10% or 0.01-5%.

The present disclosure further relates to any of the compound of general formula, the stereoisomer or pharmaceutically acceptable salt thereof, or the use of the pharmaceutical composition in the preparation of GLP-1 receptor agonist drugs.

The present disclosure further relates to the use of the compound as represented by the general formula, the stereoisomer or pharmaceutically acceptable salt thereof, or the pharmaceutical composition thereof in the preparation of a drug for treating metabolism related diseases, wherein the metabolism related diseases are selected from diabetes, obesity or non-alcohol steatohepatitis related diseases or other related diseases caused by diabetes, obesity or non-alcoholic steatohepatitis.

The present disclosure further relates to the method of the compound as represented by general formula, or the stereoisomer or pharmaceutically acceptable salt thereof, or the pharmaceutical composition thereof in the preparation of a drug for treating metabolic diseases and related diseases.

The present disclosure further relates to a method for preventing and/or treating metabolism related diseases, which comprises administering to the patient a therapeutically effective dose of the compound as represented by the general formula, the stereoisomers or pharmaceutically acceptable salts thereof, or the pharmaceutical composition thereof.

The present disclosure further provides a method for treating a disease condition with the compound or pharmaceutical composition of the present disclosure. The disease condition includes, but is not limited to, conditions related to GLP-1 receptor regulator.

The present disclosure further relates to a method for treating metabolism related diseases in mammals, comprising administering to the mammal a therapeutically effective amount of the compound of the present disclosure, or the pharmaceutically acceptable salt, ester, prodrug, solvate, hydrate or derivative thereof.

### Brief Description of the Drawings

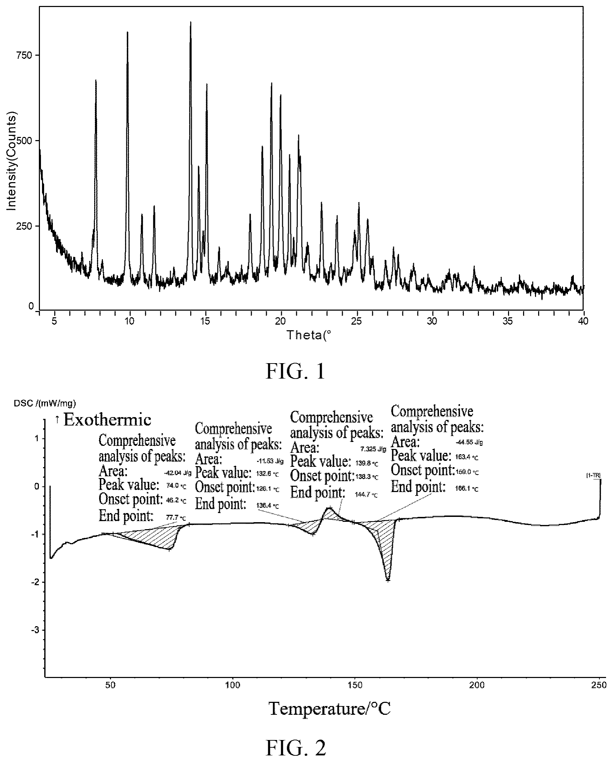

FIG. 1 is an XRPD diagram of crystal form A of tromethamine salt.
FIG. 2 is an DSC diagram of crystal form A of tromethamine salt.
FIG. 3 is an TGA diagram of crystal form A of tromethamine salt.
FIG. 4 is an XRPD diagram of crystal form B of tromethamine salt.
FIG. 5 is an DSC diagram of crystal form B of tromethamine salt.
FIG. 6 is an XRPD diagram of crystal form C of tromethamine salt.
FIG. 7 is an DSC diagram of crystal form C of tromethamine salt.
FIG. 8 is an TGA diagram of crystal form C of tromethamine salt.
FIG. 9 is an XRPD diagram of crystal form D of tromethamine salt.
FIG. 10 is an DSC diagram of crystal form D of tromethamine salt.
FIG. 11 is an XRPD diagram of crystal form E of tromethamine salt.
FIG. 12 is an DSC diagram of crystal form E of tromethamine salt.

### DETAILED DESCRIPTION OF THE INVENTION

Unless stated to the contrary, the terms used in the description and claims have the following meanings.

In the present disclosure, alkyl refers to a saturated aliphatic hydrocarbon group, which is a straight or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc.

Alkyl can be substituted or unsubstituted. When the alkyl is substituted, the substituent can be substituted at any available connection point, and the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group. In the present disclosure, alkyl is preferably methyl, ethyl, isopropyl, tert-butyl, haloalkyl, deuteroalkyl, alkoxy-substituted alkyl and hydroxyl-substituted alkyl; and the hydroxyl-substituted alkyl can be 2-hydroxyisopropyl or 1-hydroxyethyl.

In the present disclosure, alkoxy refers to -O-(alkyl) and -O-(unsubstituted cycloalkyl), wherein alkyl is as defined above, preferably alkyl containing 1 to 8 carbon atoms, more preferably alkyl containing 1 to 6 carbon atoms, and most preferably alkyl containing 1 to 3 carbon atoms. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

Non-limiting examples of alkoxy also include: prop-2-oxy, etc.

In the present disclosure, "haloalkyl" refers to alkyl substituted with one or more halogen, wherein alkyl is defined as above. Non-limiting examples of haloalkyl include: trifluoromethyl and trifluoroethyl.

Non-limiting examples of haloalkyl also include: difluoromethyl, 1,1,2,2-tetrafluoroethyl, perfluoroethyl, etc.

In the present disclosure, haloalkoxy refers to alkoxy substituted with one or more halogen, wherein alkoxy is defined as above.

The haloalkoxy can be fully halogenated or partially halogenated, and the number of halo can be 1, 2, 3, 4, 5, 6, 7, 8, 9, 10, etc. Halogen is preferably F, Cl, Br, or I. For example, haloalkoxy can be trifluoromethoxy, difluoromethoxy, 1,1,2,2-tetrafluoroethoxy, perfluoroethoxy, etc.

In the present disclosure, hydroxyalkyl refers to alkyl substituted with hydroxyl, wherein alkyl is defined as above.

In the present disclosure, haloalkyl refers to alkyl substituted with one or more halogen, wherein alkyl is defined as above.

In the present disclosure, haloalkoxy refers to alkoxy substituted with one or more halogen, wherein alkoxy is defined as above.

"Hydroxyl" refers to an -OH group.

"Halogen" refers to fluorine, chlorine, bromine, or iodine.

"Amino" refers to -NH₂.

"Cyano" refers to -CN.

"Nitro" refers to -NO₂.

"THF" refers to tetrahydrofuran.

"EtOAc" refers to ethyl acetate.

"DMSO" refers to dimethyl sulfoxide.

"LDA" refers to lithium diisopropylamide.

"DMAP" refers to 4-dimethylaminopyridine.

"EtMgBr" refers to ethyl magnesium bromide.

"HOSu" refers to N-hydroxysuccinimide.

"EDC1" refers to 1-(3-dimethylaminopropyl)-3-ethylcarbodiimide hydrochloride.

"IPA" refers to isopropanol.

"MeOH" refers to methanol.

"EtOH" refers to ethanol.

"Acetone" refers to acetone.

"MEK" refers to butanone.

"2-Me-THF" refers to 2-methyltetrahydrofuran.

"BuOH" refers to butanol.

"Dioxane" refers to dioxane.

"DMF" refers to N, N-dimethylformamide.

"DIPEA" refers to N,N-diisopropylethylamine.

"HEPES" refers to 4-hydroxyethylpiperazine ethanesulfonic acid.

"Tris" refers to tromethamine.

Different terms such as "X is selected from A, B, or C", "X is selected from A, B and C", "X is A, B or C", and "X is A, B and C" all express the same meaning, that is, X can be any one or more of A, B, and C.

"Optional" or "optionally" means that the event or circumstance subsequently described may but need not to occur, and the description includes the occasion where the event or circumstance occurs or does not occur.

"Substituted" means that one or more hydrogen atoms, preferably at most 5, more preferably 1-3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort. For example, the amino or a hydroxyl having free hydrogen may be unstable when combined the carbon atoms having an unsaturated (e.g., olefinic) bond.

"Stereoisomerism" comprises three types, geometric isomerism (cis-trans isomerism), optical isomerism, and conformational isomerism.

As used herein, the names of compounds are intended to encompass all possible isomeric forms, including stereoisomers of the compounds (e.g., enantiomers, diastereomers, racemates or racemic mixtures and any mixtures thereof).

The hydrogen atoms of the present disclosure can be replaced with the isotope deuterium thereof, and any hydrogen atom in the example compounds involved in the present disclosure can also be replaced with a deuterium atom.

"Pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

X-ray powder diffraction pattern (XRPD) refers to the experimentally observed diffraction pattern or the parameters derived therefrom. The X-ray powder diffraction pattern is characterized by the peak position (abscissa) and the peak intensity (ordinate). Those skilled in the art will appreciate that the experimental error depends on the conditions of the instrument, the preparation of the sample and the purity of the sample. In particular, it is well known to those skilled in the art that the X-ray diffraction pattern often changes with the conditions of the instrument, and those skilled in the art should understand that the appropriate error tolerance of XRPD can be: 2θ±0.5°; 2θ±0.4°; 2θ±0.3°; or 2θ±0.2°. In particular, it should be noted that the relative intensity of the X-ray diffraction pattern may also change with experimental conditions, and thus the order of peak intensity cannot be used as the only or decisive factor. In addition, due to the influence of experimental factors such as sample height, peak angles may shift as a whole, and a certain shift is usually allowed. Therefore, those skilled in the art will appreciate that any crystal form having the characteristic peak identical or similar to those of the pattern of the present disclosure falls within the scope of the present disclosure.

"TGA" refers to thermogravimetric analysis (TGA) experiment.

"DSC" refers to differential scanning calorimetry (DSC) experiment.

"HPLC" refers to high performance liquid chromatography (HPLC) experiment.

"PK" refers to pharmacokinetic (PK) experiment.

"KF" refers to Karl Fischer moisture measurement (KF) experiment.

The present disclosure will be further described below in conjunction with examples, but these examples are not meant to limit the scope of the present disclosure.

### Example

The following examples are used to explain the present disclosure, but these examples should not be considered to limit the scope of the present disclosure. If the specific conditions of the experimental method are not specifically illustrated in the examples of the present disclosure, the conventional conditions or recommended conditions of the raw material and product manufacturers are generally followed. Reagents without specific source designation are commercially available conventional reagents.

### Intermediate Im-1

### Synthesis of (methyl (S)-2-(chloromethyl)-1-(oxetan-2-ylmethyl)-1H-benzo[d]imidazole-6-carboxylate)

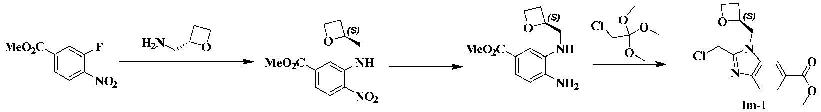

### Step 1

### Methyl (S)-4-nitro-3-((oxetan-2-ylmethyl)amino)benzoate

In a 50 mL reaction bottle, 3-fluoro-4-(1-((6-(piperidin-4-yl)pyridin-2-yl)oxo)cyclopropyl)benzonitrile (2 g, 10.04 mmol), K₂CO₃ (2.78 g, 20.08 mmol) were dissolved in tetrahydrofuran (30 mL), and then (S)-oxetan-2-ylmethylamine (874 mg, 10.04 mmol) was added, and the reaction liquid was stirred at room temperature for 12 h. After the reaction was stopped, water (20 mL) was added to quench the reaction, and the obtained mixture was extracted with ethyl acetate (15 mL × 2). The combined organic phases were washed with saturated sodium chloride (10 mL), dried over anhydrous sodium sulfate, and filtered, and the resulting residue was purified by silica gel column chromatography with petroleum ether and ethyl acetate as an eluent system to obtain the title product methyl (S)-4-nitro-3-((oxetan-2-ylmethyl)amino)benzoate (2.0 mg, yellow solid), yield: 74.8%.

MS m/z (ESI): 267.0 [M+1].

### Step 2

### Methyl (S)-4-amino-3-((oxetan-2-ylmethyl)amino)benzoate

Methyl (S)-4-nitro-3-((oxetan-2-ylmethyl)amino)benzoate (3 g, 11.27 mmol) was dissolved in methanol (30 mL), and then 10% Pd/C (300 mg) was added. The obtained mixture was subjected to hydrogen replacement three times, and reacted under stirring for 3 h. The reaction liquid was filtered, and the organic phase was dried and then dried by rotary evaporation to obtain the title product methyl (*S*)-4-amino-3-((oxetan-2-ylmethyl)amino)benzoate (2.6 g, yellow solid), yield: 97.7%.

MS m/z (ESI): 237.1 [M+1].

### Step 3

### Methyl (S)-2-(chloromethyl)-1-(oxetan-2-ylmethyl)-1H-benzo[d]imidazole-6-carboxylate

In a 50 mL reaction bottle, methyl (*S*)-4-nitro-3-((oxetan-2-ylmethyl)amino)benzoate (2 g, 8.46 mmol) and p-toluenesulfonic acid (86 mg, 499.41 µmol) were dissolved in tetrahydrofuran (100 mL), and then 2-chloro-1,1,1-trimethoxyethane (1.3 g, 8.41 mmol) was added. The reaction liquid was stirred at 60°C for 1 h. After the reaction was stopped, the reaction liquid was cooled to room temperature and concentrated under reduced pressure, and the resulting residue was purified by silica gel column chromatography with petroleum ether and ethyl acetate as an eluent system to obtain the title product methyl (*S*)-2-(chloromethyl)-1-(oxetan-2-ylmethyl)-1H-benzo[d]imidazole-6-carboxylate (Im-1) (1.3 g, yellow solid), yield: 52.0%.

MS m/z (ESI): 295.0 [M+1].

### Intermediate Im-2

### Synthesis of (methyl (S)-2-(chloromethyl)-3-(oxetan-2-ylmethyl)-3H-imidazolo[4,5-d]pyridine-5-carboxylate )

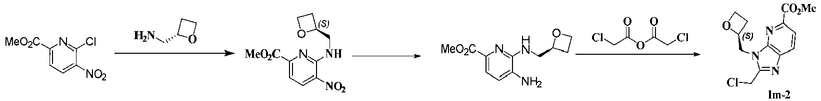

### Step 1

### Methyl (S)-5-nitro-6-((oxetan-2-ylmethyl)amino)2-picolinate

Methyl 6-chloro-5-nitro-2-picolinate was used as the raw material and step 1 of intermediate Im-1 was referenced to obtain the title product methyl (*S*)-5-nitro-6-((oxetan-2-ylmethyl)amino)2-picolinate.

MS m/z (ESI): 268.1 [M+1].

### Step 2

### Methyl (S)-5-amino-6-((oxetan-2-ylmethyl)amino)2-picolinate.

Methyl (*S*)-5-nitro-6-((oxetan-2-ylmethyl)amino)2-picolinate was used as the raw material and step 2 of intermediate Im-1 was referenced to obtain the title product methyl (*S*)-5-amino-6-((oxetan-2-ylmethyl)amino)2-picolinate.

MS m/z (ESI): 238.1 [M+1].

### Step 3

### (methyl (S)-2-(chloromethyl)-3-(oxetan-2-ylmethyl)-3H-imidazolo[4,5-d]pyridine-5-carboxylate

At room temperature, methyl (*S*)-5-amino-6-((oxetan-2-ylmethyl)amino)2-picolinate (340 mg, 1.43 mmol) was dissolved in tetrahydrofuran (5 mL), and then a solution of chloroacetic anhydride (257.27 mg, 1.50 mmol) in tetrahydrofuran (5 mL) was added dropwise. The mixture was stirred at room temperature for 30 minutes, then heated to 60°C, reacted for 2 h, cooled to room temperature, and LCMS indicated that the reaction was completed. The reaction liquid was diluted with ethyl acetate (30 mL), then washed with saturated sodium bicarbonate solution (15 mL × 3), washed with saturated sodium chloride solution (15 mL × 3), and the organic phase was dried over anhydrous sodium sulfate, filtered, and dried by rotary evaporation to obtain the title product (methyl (*S*)-2-(chloromethyl)-3-(oxetan-2-ylmethyl)-3H-imidazolo[4,5-d]pyridine-5-carboxylate (Im-2) (yellow oil, 0.4 g), yield: 94.4%, the crude product was directly used for the next step.

MS m/z (ESI): 296.1 [M+1].

### Intermediate Im-3

### Synthesis of ethyl 2-(chloromethyl)-1-((1-(fluoromethyl)cyclopropyl)methyl)-1H-benzo[d]imidazole-6-carboxylate

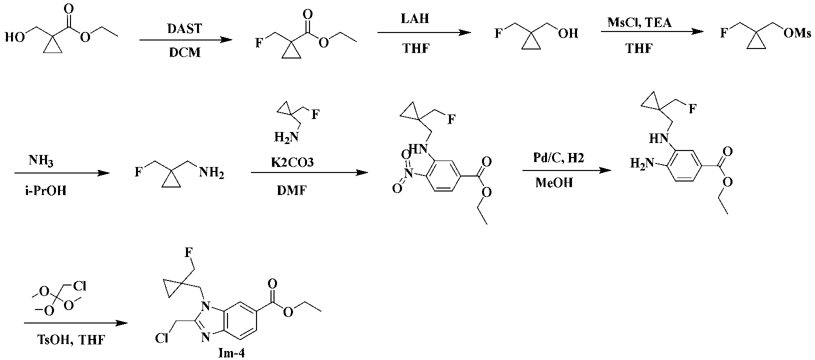

### Step 1

### Ethyl 1-(fluoromethyl)cyclopropane-1-carboxylate

Ethyl 1-(hydroxymethyl)cyclopropylcarboxylate (7 g, 48.55 mmol) was dissolved in DCM (100 mL), and DAST (8.61 g, 53.41 mmol) was added thereto at -78°C. The reaction system was naturally warmed to room temperature and stirred for 16 h. After the reaction was completed, 50 mL of water was added thereto, and the obtained mixture was extracted with dichloromethane (50 mL × 2), washed with saturated sodium chloride solution (30 mL × 2), dried over anhydrous sodium sulfate, and concentrated to obtain the title product ethyl 1-(fluoromethyl)cyclopropane-1-carboxylate (6.5 g, yellow oil), yield: 91.6%.

¹H NMR (400 MHz, CDCl₃): δ 4.52 (dd, 1.5 Hz, 2H), 4.17 (q, 2H), 1.41 - 1.33 (m, 2H), 1.26 (t, 3H), 1.05 - 0.95 (m, 2H).

### Step 2

### 1-(fluoromethyl)cyclopropylmethanol

Ethyl 1-(fluoromethyl)cyclopropane-1-carboxylate (6.5 g, 44.47 mmol) was dissolved in THF (60 mL), and LiAlH4 (2.53 g, 66.71 mmol) was added thereto in an ice-water bath. The reaction system was naturally warmed to room temperature and stirred for 16 h. After the reaction was completed, 15 g of sodium sulfate decahydrate was added thereto to quench the reaction, and the mixture was filtered and concentrated to obtain the title product 1-(fluoromethyl)cyclopropylmethanol (3.5 g, yellow oil), yield: 75.6%.

¹H NMR (400 MHz, CDCl₃): δ 4.36 (d, 2H), 3.58 (s, 2H), 0.60 (m, 4H).

### Step 3

### (1-(fluoromethyl)cyclopropyl)methylmethanesulfonate

1-(fluoromethyl)cyclopropylmethanol (1.3 g, 12.49 mmol) was dissolved in DCM (30 mL), and methanesulfonyl chloride (1.86 g, 16.23 mmol, 1.26 mL) and triethylamine (2.53 g, 24.97 mmol, 3.48 mL) were added dropwise thereto in an ice-water bath. The reaction system was stirred at 20°C. After the reaction was completed, saturated NaHCO₃ (10 mL) was added dropwise to quench the reaction, and the obtained mixture was extracted with dichloromethane (20 mL × 3), washed with saturated sodium chloride solution (20 mL × 2), dried over anhydrous sodium sulfate, and concentrated to obtain the crude product (1-(fluoromethyl)cyclopropyl)methylmethanesulfonate (2.0 g, light yellow oil), yield: 87.0% .

¹H NMR (400 MHz, CDCl₃) δ 4.32 (d, 2H), 4.19 (s, 2H), 3.05 (s, 3H), 0.81 - 0.72 (m, 4H).

### Step 4

### (1-(fluoromethyl)cyclopropyl)methylamine

(1-(fluoromethyl)cyclopropyl)methylmethanesulfonate (1.0 g, 5.49 mmol) was dissolved in NH3/i-PrOH (10 mL), and the reaction system was heated at 60°C under a microwave for 6 h. After the reaction was completed, the mixture was concentrated to obtain the title product (1-(fluoromethyl)cyclopropyl)methylamine (600 mg, yellow oil). The crude product was directly used for the next reaction.

### Step 5

Ethyl 3-(((1-(fluoromethyl)cyclopropyl)methyl)amino)-4-nitrobenzoate Ethyl 3-fluoro-4-nitrobenzoate (1.24 g, 5.82 mmol) was dissolved in DMF (30 mL), and (1-(fluoromethyl)cyclopropyl)methylamine (600 mg, 5.82 mmol) and K₂CO₃ (1.61 g, 11.64 mmol) were added thereto. The reaction system was stirred at 20°C for four h. After the reaction was completed, water (10 mL) was added thereto for dilution, and the obtained mixture was extracted with ethyl acetate (20 mL × 2), washed with saturated sodium chloride solution (20 mL × 2), dried over anhydrous sodium sulfate, and concentrated to obtain a crude product. The resulting residue was purified by silica gel column chromatography with petroleum ether and ethyl acetate as an eluent system to obtain the title product ethyl 3-(((1-(fluoromethyl)cyclopropyl)methyl)amino)-4-nitrobenzoate (1.2 g, light yellow solid), yield: 69.6%.

MS m/z (ESI): 297.1 [M+1].

¹H NMR (400 MHz, CDCl₃): δ 8.22 (d, 1H), 7.56 (s, 1H), 7.25 (d, 1H), 4.41 (q, 2H), 4.30 (d, 2H), 3.42 (d, 2H), 1.41 (t, 3H), 0.76 (m, 4H).

### Step 6

### Ethyl 4-amino-3-(((1-(fluoromethyl)cyclopropyl)methyl)amino)benzoate

Ethyl 3-(((1-(fluoromethyl)cyclopropyl)methyl)amino)-4-nitrobenzoate (1.2 g, 4.05 mmol) was dissolved in MeOH (30 mL), and Pd/C (200 mg, 10% purity) was added thereto. After displacement three times with hydrogen, the reaction system was placed in an 20°C and stirred for 2 h. After the reaction was completed, the mixture was filtered through diatomaceous earth and the filtrate was concentrated to obtain the title product ethyl 4-amino-3-(((1-(fluoromethyl)cyclopropyl)methyl)amino)benzoate (1.0 g, yellow solid), yield: 92.7%.

MS m/z (ESI): 267.1 [M+1].

### Step 7

### Ethyl 2-(chloromethyl)-1-((1-(fluoromethyl)cyclopropyl)methyl)-1H-benzo[d]imidazole-6-carboxylate

Ethyl 4-amino-3-(((1-(fluoromethyl)cyclopropyl)methyl)amino)benzoate (1.0 g, 3.76 mmol) was dissolved in MeCN (30 mL), and p-toluenesulfonic acid (193.99 mg, 1.13 mmol) and 2-chloro-1,1,1-trimethoxyethane (1.16 g, 7.51 mmol) were added thereto. The reaction system was stirred in an oil bath at 60°C for 4 h. After the reaction was completed, the mixture was concentrated to obtain a crude product. The resulting residue was purified by silica gel column chromatography with petroleum ether and ethyl acetate as an eluent system to obtain ethyl 2-(chloromethyl)-1-((1-(fluoromethyl)cyclopropyl)methyl)-1H-benzo[d]imidazole-6-carboxylate (700 mg, yellow solid), yield: 57.4%.

MS m/z (ESI): 325.1 [M+1].

¹H NMR (400 MHz, CDCl₃): δ 8.21 (s, 1H), 8.04(d,1H), 7.81 (d, 1H), 4.97 (s, 2H), 4.47 (s, 2H), 4.44 (q, 2H), 4.03 (d, 2H), 1.43 (t, 3H), 0.92 - 0.82 (m, 4H).

### Intermediate Im-4

### Tert-butyl-4-(3-acetyl-2-hydroxyphenyl)piperidine-1-carboxylate

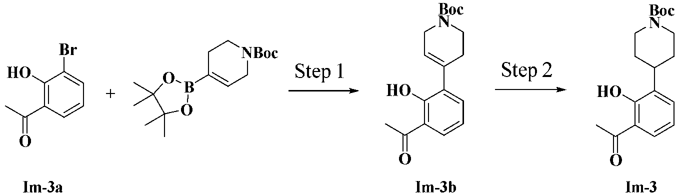

### Step 1

### Tert-butyl 4-(3-acetyl-2-hydroxyphenyl)-3,6-dihydropyridine-1(2H)-carboxylate

1-(3-bromo-2-hydroxyphenyl)ethan-1-one **Im-4a** (20 g, 0.09 mol), tert-butyl-4-(4,4,5,5-tetramethyl-1,3,2-dioxaborolan-2-yl)-3,6-dihydropyridine-1(2H)-carboxylate (32 g, 0.10 mol), Pd(dppf)Cl₂·CH₂Cl₂ (7.6 g, 9.40 mmol), and anhydrous potassium carbonate (39 g, 0.28 mol) were dissolved in 250 mL of a mixed solvent of dioxane and water (4 : 1). The reaction was heated to 100°C and reacted under stirring for 8 h. After the reaction was stopped, the reaction liquid was cooled to ambient temperature. The reaction liquid was filtered through diatomaceous earth, and the filtrate was concentrated under reduced pressure. The resulting residue was purified by silica gel column chromatography with petroleum ether and ethyl acetate as an eluent system to obtain the title product tert-butyl- 4-(3-acetyl-2-hydroxyphenyl)-3,6-dihydropyridine-1(2H)-carboxylate **Im-4b** (26 g, colorless oil), yield: 88.1%.

MS m/z (ESI): 318.1 [M+1].

### Step 2

### Tert-butyl-4-(3-acetyl-2-hydroxyphenyl)piperidine-1-carboxylate

Tert-butyl-4-(3-acetyl-2-hydroxyphenyl)-3,6-dihydropyridine-1(2H)-carboxylate **Im-4b** (26 g, 0.08 mol) and Pd/C (2.6 g, 10% wt.) were dispersed in methanol (300 mL), hydrogen was introduced 3 times, and air was discharged. The reaction liquid was reacted under stirring for 12 h. After the reaction was stopped, the reaction liquid was filtered through diatomaceous earth, and the filtrate was concentrated under reduced pressure. The resulting residue was purified by silica gel column chromatography with petroleum ether and ethyl acetate as an eluent system to obtain the title product tert-butyl-4-(3-acetyl-2-hydroxyphenyl)piperidine-1-carboxylate **Im-4** (25 g, white solid), yield: 95.6%.

MS m/z (ESI): 320.1 [M+1].

### Example 1

### 2-((4-(2-(4-chloro-2-fluorophenyl)-4-fluoro-2H-chromen-8-yl)piperidin-1-yl)methyl)-1-((( S)-oxetan-2-yl)methyl)-1H-benzo[d]imidazole-6-carboxylic acid

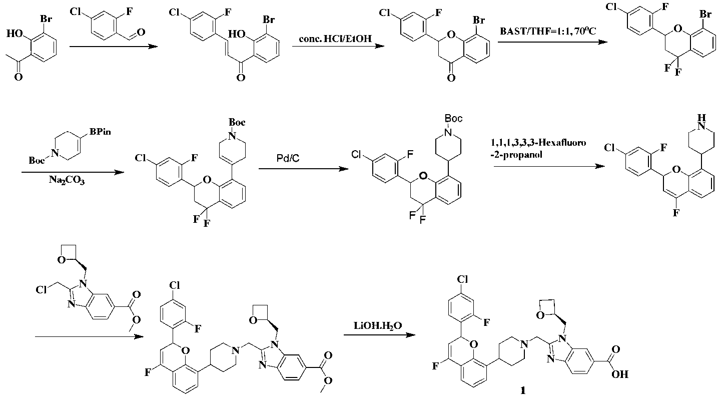

### Step 1

### (E)-1-(3-bromo-2-hydroxy-phenyl)-3-(4-chloro-2-fluoro-phenyl)prop-2-en-1-one

Sodium tetraborate decahydrate (45.97 g, 66.50 mmol) was added to a solution of 1-(3-bromo-2-hydroxy-phenyl)ethyl ketone (13 g, 60.45 mmol) and 4-chloro-2-fluoro-benzaldehyde (10.06 g, 63.48 mmol) in ethanol (125 mL) and water (200 mL), then the mixture was stirred at 90°C for 12 h, cooled, and filtered, and the filter cake was washed with water, and dried to obtain the title product (*E*)-1-(3-bromo-2-hydroxy-phenyl)-3-(4-chloro-2-fluoro-phenyl)prop-2-en-1-one (20 g, yellow solid), yield: 93.0%.
MS m/z (ESI): 354.9 [M+1]

### Step 2

### 8-bromo-2-(4-chloro-2-fluoro-phenyl)chroman-4-one

A solution of (*E*)-1-(3-bromo-2-hydroxy-phenyl)-3-(4-chloro-2-fluorophenyl)prop-2-en-1-one (2 g, 5.62 mmol) and concentrated hydrochloric acid (4 mL) in ethanol (10 mL) was stirred at 110°C under microwave conditions for 20 h, and the mixture was cooled, and dried by rotary evaporation. The resulting residue was purified by silica gel column chromatography with petroleum ether and ethyl acetate as an eluent system to obtain the title product 8-bromo-2-(4-chloro-2-fluorophenyl)chroman-4-one (1.8 g, yellow solid), yield: 90.0%.
MS m/z (ESI): 355.0 [M+1]

### Step 3

### 8-bromo-2-(4-chloro-2-fluoro-phenyl)-4,4-fifluoro-chromane

2-methoxy-N-(2-methoxyethyl)-N-(trifluoro-sulfanyl)ethylamine (10 mL) was dropwise added to a solution of 8-bromo-2-(4-chloro-2-fluoro-phenyl)chroman-4-one (1.8 g, 5.06 mmol) in tetrahydrofuran (10 mL), and then the mixture was stirred at 70°C for 12 h and cooled. Water was added to quench the reaction, and the obtained mixture was extracted with dichloromethane (50 mL × 3). The organic phase was washed with saturated sodium bicarbonate solution (50 mL × 2) and saturated sodium chloride solution (50 mL × 2), dried over anhydrous sodium sulfate, filtered, and dried by rotary evaporation. The resulting residue was purified by silica gel column chromatography with petroleum ether and ethyl acetate as an eluent system to obtain the title product 8-bromo-2-(4-chloro-2-fluoro-phenyl)-4,4-fifluoro-chromane (400 mg, yellow solid), yield: 20.9% .
MS m/z (ESI): 377.0 [M+1]

### Step 4

### Tert-butyl 4-[2-(4-chloro-2-fluoro-phenyl)-4,4-fifluoro-chroman-8-yl]-3,6-dihydro-2H-pyridine-1-carboxylate

A mixture of 8-bromo-2-(4-chloro-2-fluoro-phenyl)-4,4-fifluoro-chromane (400 mg, 1.06 mmol), tert-butyl 4-(4,4,5,5-tetramethyl-1,3,2-dioxaborolan-2-yl)-3,6-dihydro-2H-pyridine-1-carboxylate (360.32 mg, 1.17 mmol), sodium carbonate (280.71 mg, 2.65 mmol), [1,1'-bis(diphenylphosphino)ferrocene]palladium chloride (77.44 mg, 105.94 µmol), 1,4-dioxane (10 mL) and water (2 mL) was stirred at 100°C for 2 h under nitrogen protection, and cooled, and water was added. The obtained mixture was extracted with dichloromethane (10 mL × 2), and the organic phase was washed with saturated sodium chloride solution (20 mL × 2), dried over anhydrous sodium sulfate, filtered, and dried by rotary evaporation. The resulting residue was purified by silica gel column chromatography with petroleum ether and ethyl acetate as an eluent system to obtain the title product tert-butyl 4-[2-(4-chloro-2-fluoro-phenyl)-4,4-fifluoro-chroman-8-yl]-3,6-dihydro-2H-pyridine-1-carboxylate (450 mg, yellow oil), yield 88.5%.
MS m/z (ESI): 480.1 [M+1]

### Step 5

### Tert-butyl 4-[2-(4-chloro-2-fluoro-phenyl)-4,4-fifluoro-chroman-8-yl]piperidine-1-carboxylate

A mixture of tert-butyl 4-[2-(4-chloro-2-fluoro-phenyl)-4,4-fifluoro-chroman-8-yl]-3,6-dihydro-2H-pyridine-1-carboxylate (450 mg, 937.66 µmol), palladium on carbon (80 mg, 10%), and ethyl acetate (30 mL) was subjected to hydrogen replacement three times and reacted under stirring for 5 h. The mixture was filtered and dried by rotary evaporation to obtain the title product tert-butyl 4-[2-(4-chloro-2-fluoro-phenyl)-4,4-fifluoro-chroman-8-yl]piperidine-1-carboxylate (380 mg, light yellow oil), yield: 84.1%
MS m/z (ESI): 482.2 [M+1]

### Step 6

### 4-[2-(4-chloro-2-fluoro-phenyl)-4-fluoro-2H-chromen-8-yl]piperidine

A mixture of tert-butyl 4-[2-(4-chloro-2-fluoro-phenyl)-4,4-fifluoro-chroman-8-yl]piperidine-1-carboxylate (340 mg, 705.49 µmol) and hexafluoroisopropanol (10 mL) was stirred at 80°C under microwave conditions for 4 h and cooled, and the filtrate was concentrated under reduced pressure. The resulting residue was purified by silica gel column chromatography with petroleum ether and ethyl acetate as an eluent system to obtain the title product 4-[2-(4-chloro-2-fluoro-phenyl)-4-fluoro-2H-chromen-8-yl]piperidine (40 mg, colorless oil), yield: 15.7%.
MS m/z (ESI): 362.1 [M+1]

### Step 7

### Methyl 2-[[4-[2-(4-chloro-2-fluoro-phenyl)-4-fluoro-2H-chromen-8-yl]-1-piperidinyl]methyl]-3-[[1-(fluoromethyl)cyclopropyl]methyl]benzimidazole-5-carboxylate

A mixture of 4-[2-(4-chloro-2-fluoro-phenyl)-4-fluoro-2H-chromen-8-yl]piperidine (40 mg, 110.55 µmol), Im-1 (46.68 mg, 143.72 µmol), potassium carbonate (61.03 mg, 442.22 µmol) and acetonitrile (5 mL) was stirred at 50°C for 3 h, and cooled, and 5 mL of water was added. The obtained mixture was extracted with dichloromethane (20 mL × 3), and the organic phase was washed with saturated sodium chloride solution (20 mL × 2), dried over anhydrous sodium sulfate, and filtered, and the filtrate was concentrated under reduced pressure. The resulting residue was purified by silica gel column chromatography with petroleum ether and ethyl acetate as an eluent system to obtain the title product ethyl 2-[[4-[2-(4-chloro-2-fluoro-phenyl)-4-fluoro-2H-chromen-8-yl]-1-piperidinyl]methyl]-3-[[1-(fluoromethyl)cyclopropyl]methyl]benzimidazole-5-carboxylate (30 mg, colorless oil), yield: 43.7%.
MS m/z (ESI): 620.2 [M+1]

### Step 8

### 2-((4-(2-(4-chloro-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid

A mixture of methyl 2-[[4-[2-(4-chloro-2-fluoro-phenyl)-4-fluoro-2H-chromen-8-yl]-1-piperidinyl]methyl]-3-[[1-(fluoromethyl)cyclopropyl]methyl] benzimidazole-5-carboxylate (30 mg, 48.38 µmol), lithium hydroxide monohydrate (20 mg, 476.62 µmol), methanol (2 mL), water (2 mL), and tetrahydrofuran (3 mL) was stirred at room temperature for 12 h, formic acid was added to adjust the pH = 6, and the obtained mixture was dried by rotary evaporation. The resulting residue was purified by silica gel column chromatography with petroleum ether and ethyl acetate as an eluent system to obtain 2-((4-(2-(4-chloro-2-fluorophenyl)-4-fluoro-2H-chromen-8-yl)piperidin-1-yl)methyl)-1-(((*S*)-oxetan-2-yl)methyl)-1H-benzo[d]imidazole-6-carboxylic acid (20 mg, white solid), yield: 68.2%.
MS m/z (ESI): 606.2 [M+1].

2-((4-(2-(4-chloro-2-fluorophenyl)-4-fluoro-2H-chromen-8-yl)piperidin-1-yl)methyl)-1-(((*S*)-oxetan-2-yl)methyl)-1H-benzo[d]imidazole-6-carboxylic acid was subjected to chiral separation to obtain

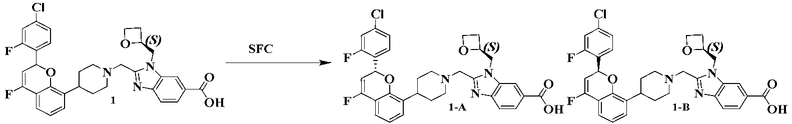

### 2-((4-((R)-2-(4-chloro-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid (1-A)

MS m/z (ESI): 606.2 [M+1].

### 2-((4-((S)-2-(4-chloro-2-fluorophenyl)-4-fluoro-2H-chromen-8-yl)piperidin-1-yl)methyl)-1-(((S)-oxetan-2-yl)methyl)-1H-benzo[d]imidazole-6-carboxylic acid (1-B)

MS m/z (ESI): 606.2 [M+1].

### Example 2

### 2-((4-(2-(4-chloro-2-fluorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid

Im-1 was replaced with Im-2 and example 1 was referenced to obtain 2-((4-(2-(4-chloro-2-fluorophenyl)-4-fluoro-2H-chromen-8-yl)piperidin-1-yl)methyl)-3-(((*S*)-oxetan-2-yl)methyl)-3H-imidazolo[4,5-b]pyridine-5-carboxylic acid

MS m/z (ESI): 607.2 [M+1].

2-((4-(2-(4-chloro-2-fluorophenyl)-4-fluoro-2H-chromen-8-yl)piperidin-1-yl)methyl)-3-(((*S*)-oxetan-2-yl)methyl)-3H-imidazolo[4,5-b]pyridine-5-carboxylic acid was subjected to chiral resolution to obtain

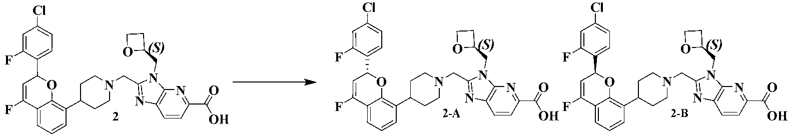

### 2-((4-((R)-2-(4-chloro-2-fluorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (example 2-A)

MS m/z (ESI): 607.2 [M+1].

2-((4-((*S*)-2-(4-chloro-2-fluorophenyl)-4-fluoro-2H-chromen-8-yl)piperidin-1-yl)methyl)-3-(((*S*)-oxetan-2-yl)methyl)-3H-imidazolo[4,5-b]pyridine-5-carboxylic acid (example 2-B)

MS m/z (ESI): 607.2 [M+1].

### Example 3

### 2-((4-(2-(4-chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid

Im-1 was replaced with Im-3 and example 1 was referenced to obtain 2-((4-(2-(4-chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid

MS m/z (ESI): 622.2 [M+1].

2-((4-(2-(4-chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid was subjected to chiral separation to obtain

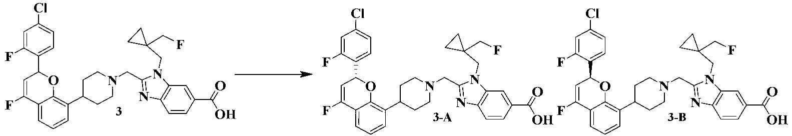

### (R)-2-((4-(2-(4-chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (example 3-A)

MS m/z (ESI): 622.2 [M+1].

### (S)-2-((4-(2-(4-chloro-2-fluorophenyl)-4-fluoro-2H-chromen-8-yl)piperidin-1-yl)methyl)-1-((1-(fluoromethyl)cyclopropyl)methyl)-1H-benzo[d]imidazole-6-carboxylic acid (example 3-B)

MS m/z (ESI): 622.2 [M+1].

### Example 4

### 2-((4-(2-(2,4-dichlorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid

4-(2-(2,4-dichlorophenyl)-4-fluoro-2H-chromen-8-yl)piperidine and Im-2 were used as the raw materials, and example 1 was referenced to obtain 2-((4-(2-(2,4-dichlorophenyl)-4-fluoro-2H-chromen-8-yl)piperidin-1-yl)methyl)-3-(((*S*)-oxetan-2-yl)methyl)-3H-imidazolo[4,5-b]pyridine-5-carboxylic acid

MS m/z (ESI):623.2 [M+1].

2-((4-(2-(2,4-dichlorophenyl)-4-fluoro-2H-chromen-8-yl)piperidin-1-yl)methyl)-3-(((*S*)-oxetan-2-yl)methyl)-3H-imidazolo[4,5-b]pyridine-5-carboxylic acid was subjected to chiral separation to obtain

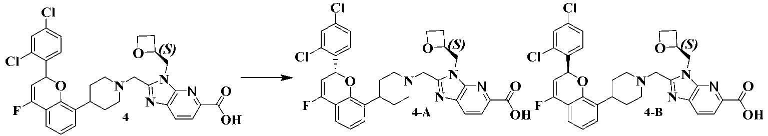

### 2-((4-((R)-2-(2,4-dichlorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (example 4-A)

MS m/z (ESI):623.2 [M+1].

### 2-((4-((S)-2-(2,4-dichlorophenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (example 4-B)

MS m/z (ESI):623.2 [M+1].

### Example 5

### 2-((4-(2-(2,4-dichlorophenyl)-4-fluoro-2H-chromen-8-yl)piperidin-1-yl)methyl)-1-(((S)-oxetan-2-yl)methyl)-1H-benzo[d]imidazole-6-carboxylic acid

4-(2-(2,4-dichlorophenyl)-4-fluoro-2H-chromen-8-yl)piperidine and Im-1 were used as the raw materials, and example 1 was referenced to obtain 2-((4-(2-(2,4-dichlorophenyl)-4-fluoro-2H-chromen-8-yl)piperidin-1-yl)methyl)-1-(((*S*)-oxetan-2-yl)methyl)-1H-benzo[d]imidazole-6-carboxylic acid

MS m/z (ESI):622.2 [M+1].

2-((4-(2-(2,4-dichlorophenyl)-4-fluoro-2H-chromen-8-yl)piperidin-1-yl)methyl)-1-(((*S*)-oxetan-2-yl)methyl)-1H-benzo[d]imidazole-6-carboxylic acid was subjected to chiral separation to obtain

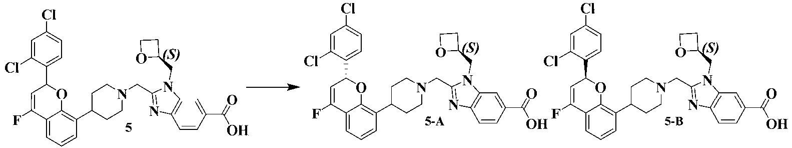

### 2-((4-((R)-2-(2,4-dichlorophenyl)-4-fluoro-2H-chromen-8-yl)piperidin-1-yl)methyl)-1-(((S)-oxetan-2-yl)methyl)-1H-benzo[d]imidazole-6-carboxylic acid (example 5-A)

MS m/z (ESI):622.2 [M+1].

### 2-((4-((S)-2-(2,4-dichlorophenyl)-4-fluoro-2H-chromen-8-yl)piperidin-1-yl)methyl)-1-(((S)-oxetan-2-yl)methyl)-1H-benzo[d]imidazole-6-carboxylic acid (example 5-B)

MS m/z (ESI):622.2 [M+1].

### Example 6

### 2-((4-(4-chloro-2-(4-chloro-2-fluorophenyl)-2H-chromen-8-yl)piperidin-1-yl)methyl)-1-(((S)-oxetan-2-yl)methyl)-1H-benzo[d]imidazole-6-carboxylic acid

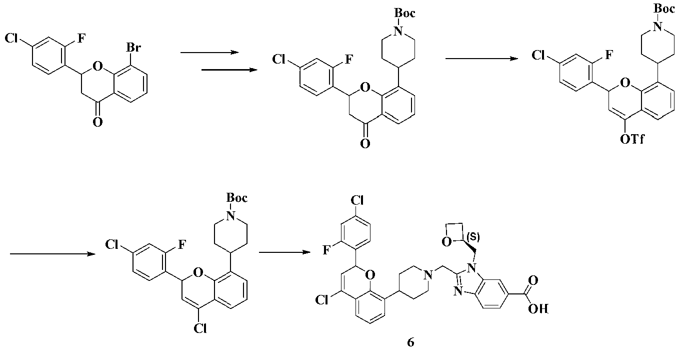

### Step 1

### Tert-butyl 4-(2-(4-chloro-2-fluorophenyl)-4-carboxychroman-8-yl)piperidine-1-carboxylate

8-bromo-2-(4-chloro-2-fluorophenyl)chroman-4-one was used as the raw material, and the step 4 and step 5 of example 1 were referenced to obtain the product tert-butyl 4-(2-(4-chloro-2-fluorophenyl)-4-carboxychroman-8-yl)piperidine-1-carboxylate.

MS m/z (ESI): 460.1[M+1].

### Step 2

### Tert-butyl 4-(2-(4-chloro-2-fluorophenyl)-4-(((trifluoromethyl)sulfonyl)oxo)-2H-chromen-8-yl)piperidine-1-carboxylate

Tert-butyl 4-(2-(4-chloro-2-fluorophenyl)-4-carboxychroman-8-yl)piperidine-1-carboxylate (1 g, 2.17 mmol) was dissolved in 20 mL of THF, and lithium bistrimethylsilylamide (2.4 mL, 2.40 mmol) was added at -78°C and reacted for 1 h. A solution of 2-[N,Nbis(trifluoromethanesulfonyl)amino]-5-chloropyridine (0.94 g, 2.4 mmol) in THF (20 mL) was added at -78°C, and the mixture was slowly warmed to room temperature and stirred for 5 h. Sodium bicarbonate (10 mL) was added to quench the reaction, and the obtained mixture was extracted with ethyl acetate (20 mL × 3). The organic phases were combined, and then washed with saturated sodium chloride solution (20 mL × 2), dried over anhydrous sodium sulfate, and concentrated under reduced pressure to obtain a crude product. The resulting residue was purified by silica gel column chromatography with petroleum ether and ethyl acetate as an eluent system to obtain tert-butyl 4-(2-(4-chloro-2-fluorophenyl)-4-(((trifluoromethyl)sulfonyl)oxo)-2H-chromen-8-yl)piperidine-1-carboxylate (456 mg, yield: 35.5%).

MS m/z (ESI): 592.1[M+1].

### Step 3

### Tert-butyl 4-(4-chloro-2-(4-chloro-2-fluorophenyl)-2H-chromen-8-yl)piperidine-1-carboxylate

Tert-butyl 4-(2-(4-chloro-2-fluorophenyl)-4-(((trifluoromethyl)sulfonyl)oxo)-2H-chromen-8-yl)piperidine-1-carboxylate (0.5 g, 0.84 mmol) was dissolved in 10 mL of DMA/THF (v : v = 1 : 3), and nickel acetate tetrahydrate (24.8 mg, 0.1 mmol), Zn powder (10 mg, 0.16 mmol), 1,5-cyclooctadiene (11 mg, 0.1 mmol) and lithium chloride (53 mg, 1.3 mmol) were added, and the mixture was reacted at room temperature for 16 h under nitrogen protection. Water (10 mL) was added to quench the reaction, and the obtained mixture was extracted with ethyl acetate (20 mL × 3). The organic phases were combined, and then washed with saturated sodium chloride solution (20 mL × 2), dried over anhydrous sodium sulfate, and concentrated under reduced pressure to obtain a crude product. The resulting residue was purified by silica gel column chromatography with petroleum ether and ethyl acetate as an eluent system to obtain tert-butyl 4-(4-chloro-2-(4-chloro-2-fluorophenyl)-2H-chromen-8-yl)piperidine-1-carboxylate (241 mg, yield: 60.0%).

MS m/z (ESI): 478.1[M+1].

### Step 4

### 2-((4-(4-chloro-2-(4-chloro-2-fluorophenyl)-2H-chromen-8-yl)piperidin-1-yl)methyl)-1-(((S)-oxetan-2-yl)methyl)-1H-benzo[d]imidazole-6-carboxylic acid

Tert-butyl 4-(4-chloro-2-(4-chloro-2-fluorophenyl)-2H-chromen-8-yl)piperidine-1-carboxylate and **Im-1** were used as the raw materials, and the step 6 to step 8 of example 1 were referenced to obtain the product 2-((4-(4-chloro-2-(4-chloro-2-fluorophenyl)-2H-chromen-8-yl)piperidin-1-yl)methyl)-1-(((*S*)-oxetan-2-yl)methyl)-1H-benzo[d]imidazole-6-carboxylic acid.

MS m/z (ESI): 622.1[M+1].

2-((4-(4-chloro-2-(4-chloro-2-fluorophenyl)-2H-chromen-8-yl)piperidin-1-yl)methyl)-1-(((*S*)-oxetan-2-yl)methyl)-1H-benzo[d]imidazole-6-carboxylic acid was subjected to resolution to obtain the product

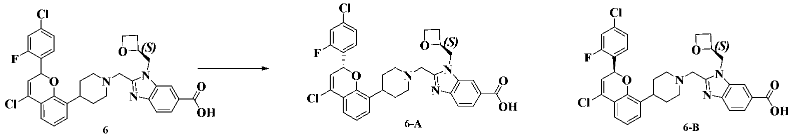

### 2-((4-((R)-4-chloro-2-(4-chloro-2-fluorophenyl)-2H-chromen-8-yl)piperidin-1-yl)methyl)-1-(((S)-oxetan-2-yl)methyl)-1H-benzo[d]imidazole-6-carboxylic acid (example 6-A)

MS m/z (ESI): 622.1[M+1].

### 2-((4-((S)-4-chloro-2-(4-chloro-2-fluorophenyl)-2H-chromen-8-yl)piperidin-1-yl)methyl)-1-(((S)-oxetan-2-yl)methyl)-1H-benzo[d]imidazole-6-carboxylic acid (example 6-B).

MS m/z (ESI): 622.1[M+1].

### Example 7

### 2-((4-(4-chloro-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid

Tert-butyl 4-(4-chloro-2-(4-chloro-2-fluorophenyl)-2H-chromen-8-yl)piperidine-1-carboxylate and Im-2 were used as the raw materials, and the step 6 to step 8 of example 1 were referenced to obtain the product 2-((4-(4-chloro-2-(4-chloro-2-fluorophenyl)-2H-chromen-8-yl)piperidin-1-yl)methyl)-3-(((*S*)-oxetan-2-yl)methyl)-3H-imidazolo[4,5-b]pyridine-5-carboxylic acid.

MS m/z (ESI): 623.1[M+1].

2-((4-(4-chloro-2-(4-chloro-2-fluorophenyl)-2H-chromen-8-yl)piperidin-1-yl)methyl)-3-(((*S*)-oxetan-2-yl)methyl)-3H-imidazolo[4,5-b]pyridine-5-carboxylic acid was subjected to resolution to obtain the product

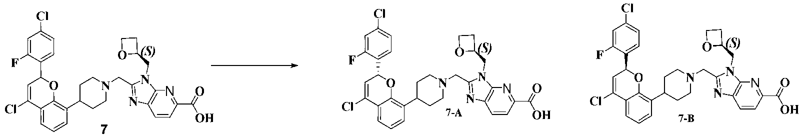

### 2-((4-((R)-4-chloro-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid (example 7-A)

MS m/z (ESI): 623.1[M+1].

2-((4-((*S*)-4-chloro-2-(4-chloro-2-fluorophenyl)-2H-chromen-8-yl)piperidin-1-yl)methyl)-3-(((*S*)-oxetan-2-yl)methyl)-3H-imidazolo[4,5-b]pyridine-5-carboxylic acid (example 7-B).

MS m/z (ESI): 623.1[M+1].

### Example 8

### 2-((4-(2-(4-chloro-2-fluorophenyl)-4-methyl-2H-chromen-8-yl)piperidin-1-yl)methyl)-1-(((S)-oxetan-2-yl)methyl)-1H-benzo[d]imidazole-6-carboxylic acid

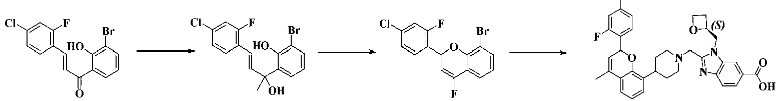

### Step 1

### (E)-2-bromo-6-(4-(4-chloro-2-fluorophenyl)-2-hydroxybut-3-ene-2-yl)phenol

(*E*)-1-(3-bromo-2-hydroxyphenyl)-3-(4-chloro-2-fluorophenyl)prop-2-en-1-one (1 g, 2.81 mmol) was dissolved in 30 mL of THF, methylmagnesium bromide (7 mL, 7 mmol) was added at 0°C, and the mixture was slowly warmed to room temperature and stirred for 3 h. Ammonium chloride (10 mL) was added to quench the reaction, and the obtained mixture was extracted with ethyl acetate (20 mL × 3). The organic phases were combined, and then washed with saturated sodium chloride solution (20 mL × 2), dried over anhydrous sodium sulfate, and concentrated under reduced pressure to obtain a crude product. The resulting residue was purified by silica gel column chromatography with petroleum ether and ethyl acetate as an eluent system to obtain (*E*)-2-bromo-6-(4-(4-chloro-2-fluorophenyl)-2-hydroxybut-3-ene-2-yl)phenol (831 mg, yield: 79.6%).

MS m/z (ESI): 370.9 [M+1].

### Step 2

### 8-bromo-2-(4-chloro-2-fluorophenyl)-4-fluoro-2H-chromene

(*E*)-2-bromo-6-(4-(4-chloro-2-fluorophenyl)-2-hydroxybut-3-ene-2-yl)phenol (0.5 g, 1.34 mmol) was dissolved in 15 mL of nitromethane, (2,3,4,5-tetrafluorophenyl)boric acid (52 mg, 0.26 mmol)was added, and the mixture was stirred at 60°C for 16 h. Water (10 mL) was added to quench the reaction, and the obtained mixture was extracted with ethyl acetate (20 mL × 3), washed with saturated sodium chloride solution (20 mL × 2), dried over anhydrous sodium sulfate, and concentrated under reduced pressure to obtain a crude product. The resulting residue was purified by silica gel column chromatography with petroleum ether and ethyl acetate as an eluent system to obtain 8-bromo-2-(4-chloro-2-fluorophenyl)-4-fluoro-2*H*-chromene (326 mg, yield: 68.0%).

MS m/z (ESI): 356.9 [M+1].

### Step 3

### 2-((4-(2-(4-chloro-2-fluorophenyl)-4-methyl-2H-chromen-8-yl)piperidin-1-yl)methyl)-1-(((S)-oxetan-2-yl)methyl)-1H-benzo[d]imidazole-6-carboxylic acid

8-bromo-2-(4-chloro-2-fluorophenyl)-4-fluoro-2H-chromene was used as the raw material, and the step 4, step 5, step 7 and step 8 of example 1 were referenced to obtain the product 2-((4-(2-(4-chloro-2-fluorophenyl)-4-methyl-2*H*-chromen-8-yl)piperidin-1-yl)methyl)-1-(((*S*)-oxetan-2-yl)methyl)-1*H*-benzo[d]imidazole-6-carboxylic acid.

MS m/z (ESI): 602.2 [M+1].

2-((4-(2-(4-chloro-2-fluorophenyl)-4-methyl-2*H*-chromen-8-yl)piperidin-1-yl)methyl)-1-(((*S*)-oxetan-2-yl)methyl)-1H-benzo[d]imidazole-6-carboxylic acid was subjected to resolution to obtain the product

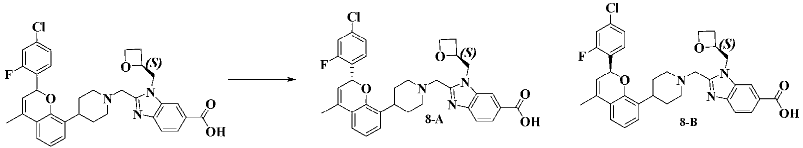

### 2-((4-((R)-2-(4-chloro-2-fluorophenyl)-4-methyl-2H-chromen-8-yl)piperidin-1-yl)methyl)-1-(((S)-oxetan-2-yl)methyl)-1H-benzo[d]imidazole-6-carboxylic acid (example 8-A)

MS m/z (ESI): 602.2 [M+1].

### 2-((4-((S)-2-(4-chloro-2-fluorophenyl)-4-methyl-2H-chromen-8-yl)piperidin-1-yl)methyl)-1-(((S)-oxetan-2-yl)methyl)-1H-benzo[d]imidazole-6-carboxylic acid (example 8-B).

MS m/z (ESI): 602.2 [M+1].

### Example 9

### 2-((4-(2-(4-chloro-2-(methoxy-d3)phenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid

4-chloro-2-(methoxy-d3)benzaldehyde was used as the raw material and example 2 was referenced to obtain the product 2-((4-(2-(4-chloro-2-(methoxy-d3)phenyl)-4-fluoro-2H-chromen-8-yl)piperidin-1-yl)methyl)-3-(((*S*)-oxetan-2-yl)methyl)-3H-imidazolo[4,5-b]pyridine-5-carboxylic acid.

MS m/z (ESI): 622.2 [M+1].

2-((4-(2-(4-chloro-2-(methoxy-d3)phenyl)-4-fluoro-2H-chromen-8-yl)piperidin-1-yl)methyl)-3-(((*S*)-oxetan-2-yl)methyl)-3H-imidazolo[4,5-b]pyridine-5-carboxylic acid was subjected to chiral separation to obtain

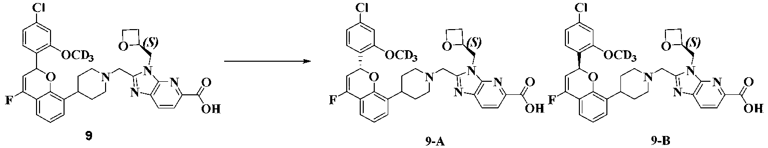

### 2-((4-((R)-2-(4-chloro-2-(methoxy-d3)phenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (example 9-A)

MS m/z (ESI): 622.2 [M+1].

### 2-((4-((S)-2-(4-chloro-2-(methoxy-d3)phenyl)-4-fluoro-2H-chromen-8-yl)piperidin-1-yl)methyl)-3-(((S)-oxetan-2-yl)methyl)-3H-imidazolo[4,5-b]pyridine-5-carboxylic acid (example 9-B)

MS m/z (ESI): 622.2 [M+1].

### Example 10

### 2-((4-(2-(4-chloro-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid

4-chloro-2-(methoxy-d3)benzaldehyde was used as the raw material and example 1 was referenced to obtain the product 2-((4-(2-(4-chloro-2-(methoxy-d3)phenyl)-4-fluoro-2H-chromen-8-yl)piperidin-1-yl)methyl)-1-(((*S*)-oxetan-2-yl)methyl)-1H-benzo[d]imidazole-6-carboxylic acid.

MS m/z (ESI): 621.2[M+1].

2-((4-(2-(4-chloro-2-(methoxy-d3)phenyl)-4-fluoro-2H-chromen-8-yl)piperidin-1-yl)methyl)-1-(((*S*)-oxetan-2-yl)methyl)-1H-benzo[d]imidazole-6-carboxylic acid was subjected to chiral separation to obtain

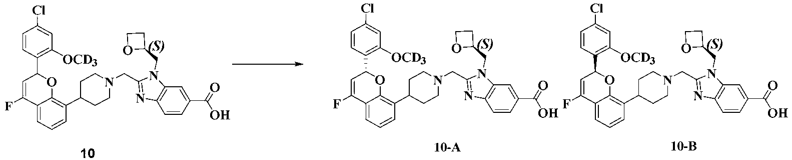

### 2-((4-((R)-2-(4-chloro-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid (example 10-A)

MS m/z (ESI): 621.2 [M+1].

### 2-((4-((S)-2-(4-chloro-2-(methoxy-d3)phenyl)-4-fluoro-2H-chromen-8-yl)piperidin-1-yl)methyl)-1-(((S)-oxetan-2-yl)methyl)-1H-benzo[d]imidazole-6-carboxylic acid (example 10-B)

MS m/z (ESI): 621.2 [M+1].

### Example 11

### 2-((4-(2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid

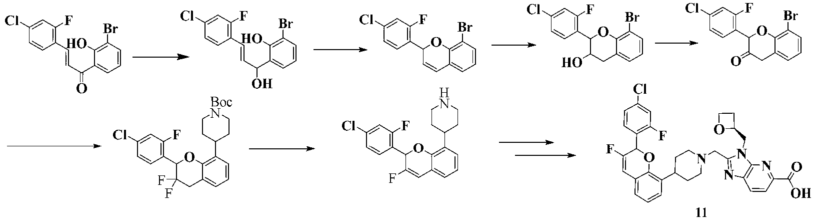

### Step 1

### (E)-2-bromo-6-(3-(4-chloro-2-fluorophenyl)-1-hydroxyallyl)phenol

(*E*)-1-(3-bromo-2-hydroxyphenyl)-3-(4-chloro-2-fluorophenyl)prop-2-en-1-one (500 mg, 1.41 mmol) was dissolved in methanol (15 mL), sodium borohydride (64 mg, 1.69 mmol) was added, and the mixture was stirred at room temperature for 2 h. Water was added to quench the reaction, and the obtained mixture was extracted with dichloromethane (30 mL × 3), washed with saturated sodium chloride solution (20 mL × 2), dried over anhydrous sodium sulfate, and filtered, and the filtrate was concentrated under reduced pressure. The resulting residue was purified by silica gel column chromatography with petroleum ether and ethyl acetate as an eluent system to obtain (*E*)-2-bromo-6-(3-(4-chloro-2-fluorophenyl)-1-hydroxyallyl)phenol (454 mg, yield: 90.0%)

MS m/z (ESI): 356.9[M+1].

### Step 2

### 8-bromo-2-(4-chloro-2-fluorophenyl)-2H-chromene

(E)-2-bromo-6-(3-(4-chloro-2-fluorophenyl)-1-hydroxyallyl)phenol (300 mg, 0.84 mmol) was dissolved in dichloromethane (10 mL), p-toluenesulfonic acid (29 mg, 0.17 mmol) was added, and the mixture was stirred at room temperature for 2 h. Water was added to quench the reaction, and the obtained mixture was extracted with dichloromethane (30 mL × 3), washed with saturated sodium chloride solution (20 mL × 2), dried over anhydrous sodium sulfate, and filtered, and the filtrate was concentrated under reduced pressure. The resulting residue was purified by silica gel column chromatography with petroleum ether and ethyl acetate as an eluent system to obtain (8-bromo-2-(4-chloro-2-fluorophenyl)-2H-chromene (200 mg, yield: 70.1%).

MS m/z (ESI): 338.9[M+1].

### Step 3

### 8-bromo-2-(4-chloro-2-fluorophenyl)chroman-3-ol

(8-bromo-2-(4-chloro-2-fluorophenyl)-2H-chromene (200 mg, 0.59 mmol) was dissolved in THF (10 mL), a solution of borane in THF (0.71 mL, 0.71 mmol) was added, and the mixture was stirred at 0°C for 1 h. 2 mL of water was added, then NaOH (71 mg, 1.77 mmol) and 0.1 mL of hydrogen peroxide solution were added, and the mixture was stirred at 40°C for 2 h. 10 mL of water was added to quench the reaction, and the obtained mixture was extracted with dichloromethane (30 mL × 3), washed with saturated sodium chloride solution (20 mL × 2), dried over anhydrous sodium sulfate, and filtered, and the filtrate was concentrated under reduced pressure. The resulting residue was purified by silica gel column chromatography with petroleum ether and ethyl acetate as an eluent system to obtain 8-bromo-2-(4-chloro-2-fluorophenyl)chroman-3-ol (63 mg, yield: 29.8%)

MS m/z (ESI): 356.9[M+1].

### Step 4

### 8-bromo-2-(4-chloro-2-fluorophenyl)chroman-3-one

8-bromo-2-(4-chloro-2-fluorophenyl)chroman-3-ol (500 mg, 1.40 mmol) was dissolved in DCM (20 mL), Dess-Martin oxidant (0.71 mL, 1.67 mmol) was added, and the mixture was stirred at 0°C for 1 h. 10 mL of water was added to quench the reaction, and the obtained mixture was extracted with dichloromethane (30 mL × 3), washed with saturated sodium chloride solution (20 mL × 2), dried over anhydrous sodium sulfate, and filtered, and the filtrate was concentrated under reduced pressure. The resulting residue was purified by silica gel column chromatography with petroleum ether and ethyl acetate as an eluent system to obtain 8-bromo-2-(4-chloro-2-fluorophenyl)chroman-3-one (448 mg, yield: 89.9%)

MS m/z (ESI): 354.9[M+1].

### Step 5

### Tert-butyl 4-(2-(4-chloro-2-fluorophenyl)-3,3-difluorochroman-8-yl)piperidine-1-carboxylate

8-bromo-2-(4-chloro-2-fluorophenyl)chroman-3-one was used as the raw material, and the step 3 to step 5 of example 1 were referenced to obtain the product tert-butyl 4-(2-(4-chloro-2-fluorophenyl)-3,3-difluorochroman-8-yl)piperidine-1-carboxylate

MS m/z (ESI): 482.2[M+1].

### Step 6

### 4-(2-(4-chloro-2-fluorophenyl)-3-fluoro-2H-chromen-8-yl)piperidine

Tert-butyl 4-(2-(4-chloro-2-fluorophenyl)-3,3-difluorochroman-8-yl)piperidine-1-carboxylate was used as the raw materials, and the step 6 of example 1 was referenced to obtain the product 4-(2-(4-chloro-2-fluorophenyl)-3-fluoro-2H-chromen-8-yl)piperidine

MS m/z (ESI): 362.1[M+1].

### Step 7

### 2-((4-(2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid

4-(2-(4-chloro-2-fluorophenyl)-3-fluoro-2H-chromen-8-yl)piperidine was used as the raw material, and the step 7 and step 8 of example 1 were referenced to obtain the product 2-((4-(2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid

MS m/z (ESI): 607.1[M+1].

2-((4-(2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid was subjected to chiral resolution

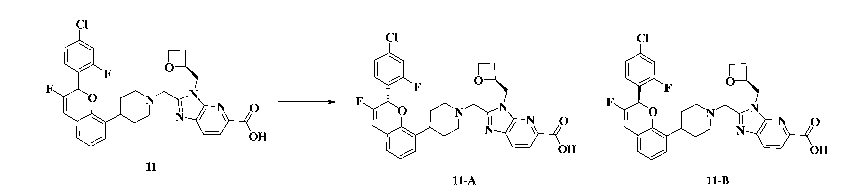

### 2-((4-((S)-2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid (example 11-A)

MS m/z (ESI): 607.1 [M+1].

### 2-((4-((R)-2-(4-chloro-2-fluorophenyl)-3-fluoro-2H-chromen-8-yl)piperidin-1-yl)methyl)-3-(((S)-oxetan-2-yl)methyl)-3H-imidazolo[4,5-b]pyridine-5-carboxylic acid (example 11-B)

MS m/z (ESI): 607.1 [M+1].

### Example 12

### 2-((4-((R)-2-(4-chloro-2-(methoxy-d3)phenyl)-4-fluoro-2-methyl-2H-chromen-8-yl)piperidin-1-yl)methyl)-3-(((S)-oxetan-2-yl)methyl)-3H-imidazolo[4,5-b]pyridine-5-carboxylic acid

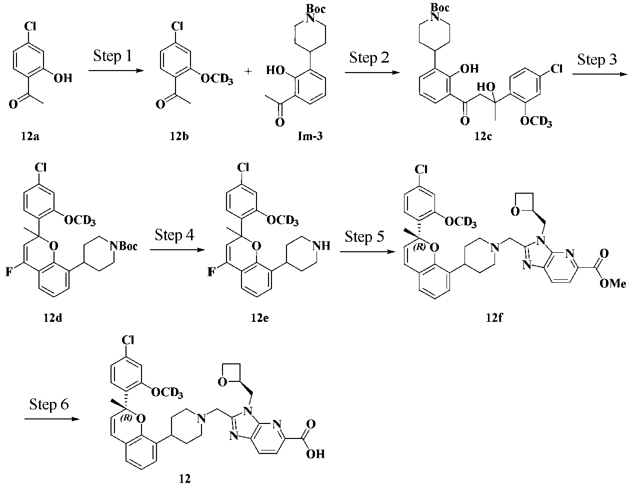

### Step 1

### 1-(4-chloro-2-(methoxy-d3)phenyl)ethan-1-one

1-(4-chloro-2-hydroxyphenyl)ethan-1-one **12a** (15 g, 87.93 mmol), deuterated iodomethane (16.57 g, 114.31 mmol), and anhydrous potassium carbonate (36.46 g, 0.26 mol) were dispersed in DMF (150 mL). The reaction liquid was heated to 50°C and reacted under vigorous stirring for 12 h. After the reaction was stopped, the reaction liquid was filtered through diatomaceous earth, and the filtrate was concentrated under reduced pressure. The resulting residue was purified by silica gel column chromatography with petroleum ether and ethyl acetate as an eluent system to obtain the title product 1-(4-chloro-2-(methoxy-d3)phenyl)ethan-1-one **12b** (15 g), yield: 90.9%.

MS m/z (ESI): 188.1 [M+1].

### Step 2

### Tert-butyl-4-(3-(3-(4-chloro-2-(methoxy-d3)phenyl)-3-hydroxybutyryl)-2-hydroxyphenyl)piperidine-1-carboxylate

Tert-butyl-4-(3-acetyl-2-hydroxyphenyl)piperidine-1-carboxylate **Im-4** (25 g, 79.94 mmol) was dissolved in tetrahydrofuran (300 mL). The solution was cooled in a dry ice/ethanol bath, and LiHMDS (18.4 mL, 0.184 mol, 1 M solution in THF) was slowly added thereto. After the addition, the mixture was reacted under stirring for 30 minutes. 1-(4-chloro-2-(methoxy-d3)phenyl)ethan-1-one **12b** (15 g, 79.94 mmol) was dissolved in THF (30 mL), and the solution was slowly added dropwise to the aforementioned reaction liquid. The mixture was reacted under stirring for 40 minutes while maintaining the dry ice/ethanol bath temperature. After the reaction was stopped, the dry ice/ethanol bath was removed, saturated ammonium chloride solution was added to the reaction liquid to quench the reaction, and the obtained mixture was extracted with ethyl acetate (300 mL × 2). The organic phases were combined, washed with saturated sodium chloride (500 mL), dried over anhydrous sodium sulfate, and filtered, and the filtrate was concentrated under reduced pressure. The resulting residue was purified by silica gel column chromatography with petroleum ether and ethyl acetate as an eluent system to obtain the title product tert-butyl-4-(3-(3-(4-chloro-2-(methoxy-d3)phenyl)-3-hydroxybutyryl)-2-hydroxyphenyl)piperidine-1-carboxylate **12c** (31 g), yield: 76.5%.

MS m/z (ESI): 507.2 [M+1].

### Step 3

### Tert-butyl-4-(2-(4-chloro-2-(methoxy-d3)phenyl)-4-fluoro-2-methyl-2H-chromen-8-yl)piperidine-1-carboxylate

Tert-butyl-4-(3-(3-(4-chloro-2-(methoxy-d3)phenyl)-3-hydroxybutyryl)-2-hydroxyphenyl)piperidine-1-carboxylate **12c** (2.00 g, 4.07 mmol) and BAST (10 mL) were dissolved in DMF (10 mL), then ethanol (20 µL) was added to the reaction liquid, and the mixture was reacted under stirring for 4 h. After the reaction was stopped, the reaction liquid was slowly added into ice water to quench the reaction, and the obtained mixture was extracted with ethyl acetate (50 mL × 2). The organic phases were combined, washed with water (100 mL), washed with saturated sodium bicarbonate (100 mL), dried over anhydrous sodium sulfate, and filtered, and the filtrate was concentrated under reduced pressure. The resulting residue was purified by silica gel column chromatography with petroleum ether and ethyl acetate as an eluent system to obtain the title product tert-butyl-4-(2-(4-chloro-2-(methoxy-d3)phenyl)-4-fluoro-2-methyl-2H-chromen-8-yl)piperidine-1-carboxylate **12d** (750 mg), yield: 38.7%.

MS m/z (ESI): 491.2 [M+1].

### Step 4

### 4-(2-(4-chloro-2-(methoxy-d3)phenyl)-4-fluoro-2-methyl-2H-chromen-8-yl)piperidine

Tert-butyl-4-(2-(4-chloro-2-(methoxy-d3)phenyl)-4-fluoro-2-methyl-2H-chromen-8-yl)piperidine-1-carboxylate **12d** (750 mg, 1.53 mmol), boron trifluoride diethyl etherate (543 mg, 3.83 mmol), and powdered 4 Å molecular sieve (750 mg) were dispersed in dichloromethane (10 mL), and the mixture was reacted under stirring for 3 h while maintaining 0°C. After the reaction was stopped, saturated sodium bicarbonate solution was added to the reaction liquid to quench the reaction. The mixture was filtered through diatomaceous earth, and the filtrate was allowed to stand. The organic layer was separated, and the aqueous layer was extracted with dichloromethane (20 mL × 2). The organic phases were combined, dried over anhydrous sodium sulfate, and filtered, and the filtrate was concentrated under reduced pressure. The resulting residue was purified by silica gel column chromatography with dichloromethane and methanol as an eluent system to obtain the title product 4-(2-(4-chloro-2-(methoxy-d3)phenyl)-4-fluoro-2-methyl-2H-chromen-8-yl)piperidine **12e** (490 mg), yield: 82.1%.

MS m/z (ESI): 391.2 [M+1].

### Step 5

### Methyl 2-((4-((R)-2-(4-chloro-2-(methoxy-d3)phenyl)-2-methyl-2H-chromen-8-yl)piperidin-1-yl)methyl)-3-(((S)-oxetan-2-yl)methyl)-3H-imidazolo[4,5-b]pyridine-5-carboxylate

4-(2-(4-chloro-2-(methoxy-d3)phenyl)-4-fluoro-2-methyl-2H-chromen-8-yl)piperidine **12e** (490 mg, 1.25 mmol), methyl (S)-2-(chloromethyl)-3-(oxetan-2-ylmethyl)-3H-imidazolo[4,5-b]pyridine-5-carboxylate **Im-2** (371 mg, 1.25 mmol), and anhydrous potassium carbonate (518 mg, 3.75 mmol) were dispersed in acetonitrile (8 mL). The reaction liquid was heated to 50°C and reacted under vigorous stirring for 3 h. After the reaction was stopped, the reaction liquid was cooled to room temperature and filtered through diatomaceous earth, and the filtrate was concentrated under reduced pressure. The resulting residue was purified by silica gel column chromatography with ethyl acetate as an eluent system, and then the purified resulting sample was sent to chiral preparative HPLC separation to obtain the title product methyl 2-((4-((R)-2-(4-chloro-2-(methoxy-d3)phenyl)-2-methyl-2H-chromen-8-yl)piperidin-1-yl)methyl)-3-(((S)-oxetan-2-yl)methyl)-3H-imidazolo[4,5-b]pyridine-5-carboxylate **12f** (210 mg), yield: 26.5%.

MS m/z (ESI): 632.2 [M+1].

### Step 6

### 2-((4-((R)-2-(4-chloro-2-(methoxy-d3)phenyl)-2-methyl-2H-chromen-8-yl)piperidin-1-yl)methyl)-3-(((S)-oxetan-2-yl)methyl)-3H-imidazolo[4,5-blpyridine-5-carboxylic acid

Methyl 2-((4-((R)-2-(4-chloro-2-(methoxy-d3)phenyl)-2-methyl-2H-chromen-8-yl)piperidin-1-yl)methyl)-3-(((S)-oxetan-2-yl)methyl)-3H-imidazolo[4,5-b]pyridine-5-carboxylate **12f** (210 mg, 332.19 µmol) and lithium hydroxide (133 mg, 3.32 mmol) were dissolved in 9 mL of a mixed solvent of THF, water and methanol (4 : 4 : 1), and the mixture was reacted under stirring for 3 h. After the reaction was stopped, formic acid was added to adjust the pH = 6, and the reaction liquid was concentrated under reduced pressure. The resulting residue was purified by preparative HPLC to obtain the title product 2-((4-((R)-2-(4-chloro-2-(methoxy-d3)phenyl)-2-methyl-2H-chromen-8-yl)piperidin-1-yl)methyl)-3-(((S)-oxetan-2-yl)methyl)-3H-imidazolo[4,5-b]pyridine-5-carboxylic acid **12** (170 mg), yield: 82.8%.

MS m/z (ESI): 618.2 [M+1].

¹H NMR (400 MHz, MeOD) δ 8.16 - 7.97 (m, 2H), 7.29 (d, J = 8.2 Hz, 1H), 7.08 - 6.98 (m, 2H), 6.94 - 6.85 (m, 2H), 6.81 (t, J = 7.5 Hz, 1H), 6.57 (d, J = 9.8 Hz, 1H), 5.78 (d, J = 9.8 Hz, 1H), 5.25 (dd, J = 7.5, 3.0 Hz, 1H), 4.96 (dd, J = 14.9, 6.7 Hz, 1H), 4.83 (dd, J = 14.9, 3.1 Hz, 1H), 4.66 - 4.53 (m, 1H), 4.48 - 4.35 (m, 1H), 4.25 (d, J = 14.2 Hz, 1H), 4.16 (d, J = 14.2 Hz, 1H), 3.25 (d, J = 11.5 Hz, 1H), 3.12 (d, J = 11.5 Hz, 1H), 2.91 (s, 1H), 2.83 - 2.70 (m, 1H), 2.61 - 2.41 (m, 3H), 1.85 (q, J = 3.9 Hz, 2H), 1.78 (s, 3H), 1.68 (d, J = 3.7 Hz, 1H), 1.59 (d, J = 14.9 Hz, 1H).

### Example 13

### 2-((4-((R)-2-(4-chloro-2-fluorophenyl)-4-fluoro-2-methyl-2H-chromen-8-yl)piperidin-1-yl)methyl)-3-(((S)-oxetan-2-yl)methyl)-3H-imidazolo[4,5-b]pyridine-5-carboxylic acid

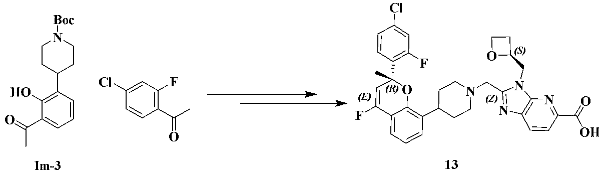

Tert-butyl 4-(3-acetyl-2-hydroxyphenyl)piperidine-1-carboxylate **Im-4** and 1-(4-chloro-2-fluorophenyl)ethan-1-one were used as the raw materials, and the step 2 to step 6 of example 12 were referenced to obtain 2-((4-((R)-2-(4-chloro-2-fluorophenyl)-4-fluoro-2-methyl-2H-chromen-8-yl)piperidin-1-yl)methyl)-3-(((S)-oxetan-2-yl)methyl)-3H-imidazolo[4,5-b]pyridine-5-carboxylic acid **13.**

MS m/z (ESI):621.2 [M+1].

### Example 14

### 2-((4-((R)-2-(4-chloro-2-fluorophenyl)-2-methyl-2H-chromen-8-yl)piperidin-1-yl)methyl)-3-(((S)-oxetan-2-yl)methyl)-3H-imidazolo[4,5-b]pyridine-5-carboxylic acid

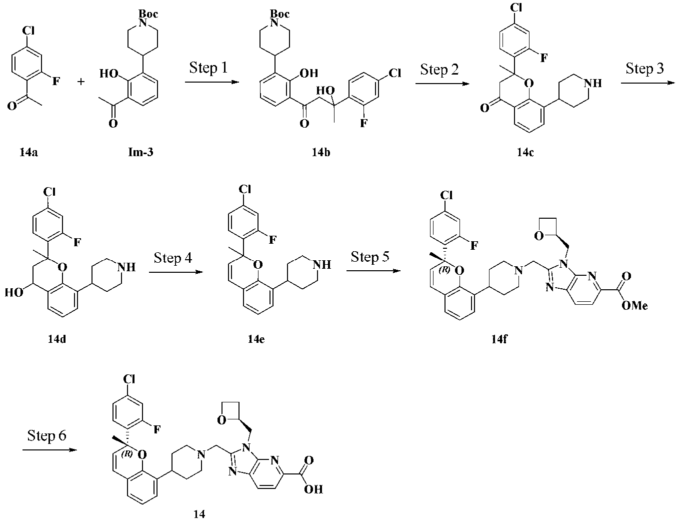

### Step 1

### Tert-butyl-4-(3-(3-(4-chloro-2-fluorophenyl)-3-hydroxybutyryl)-2-hydroxyphenyl)piperidine-1-carboxylate

Referring to the synthesis method of example **13,** 1-(4-chloro-2-fluorophenyl)ethan-1-one **14a** (5 g, 28.97 mmol), Im-4 (9.25 g, 28.97 mmol), LiHMDS (66.6 mL, 66.63 mmol), and THF (100 mL) were dosed to obtain the title product tert-butyl-4-(3-(3-(4-chloro-2-fluorophenyl)-3-hydroxybutyryl)-2-hydroxyphenyl)piperidine-1-carboxylate **17b** (7.60 g), yield: 53.3%.

MS m/z (ESI): 492.2 [M+1].

### Step 2

### 2-(4-chloro-2-fluorophenyl)-2-methyl-8-(piperidin-4-yl)chroman-4-one

Tert-butyl-4-(3-(3-(4-chloro-2-fluorophenyl)-3-hydroxybutyryl)-2-hydroxyphenyl)piperidine-1-carboxylate **14b** (5 g, 10.16 mmol) and p-toluenesulfonic acid (5.25 g, 30.48 mmol) were dissolved in toluene (50 mL). The reaction liquid was heated to 100°C and reacted under stirring for 5 h. After the reaction was stopped, the reaction liquid was cooled to room temperature, and 2 M sodium hydroxide solution was added thereto to quench the reaction. The organic layer was separated, and the aqueous layer was extracted with dichloromethane (50 mL × 3). The organic phases were combined, dried over anhydrous sodium sulfate, and filtered, and the filtrate was concentrated under reduced pressure. The resulting residue was purified by silica gel column chromatography with dichloromethane and methanol as an eluent system to obtain the title product 2-(4-chloro-2-fluorophenyl)-2-methyl-8-(piperidin-4-yl)chroman-4-one **14c** (2.36 g), yield: 62.1%.

MS m/z (ESI): 374.1 [M+1].

### Step 3

### 2-(4-chloro-2-fluorophenyl)-2-methyl-8-(piperidin-4-yl)chroman-4-ol

2-(4-chloro-2-fluorophenyl)-2-methyl-8-(piperidin-4-yl)chroman-4-one **14c** (2 g, 5.35 mmol) was dissolved in methanol (30 mL). Sodium borohydride (396 mg, 10.70 mmol) was slowly added to the solution in portions under an ice bath. After the addition, the ice bath was removed and the reaction liquid was allowed to naturally warm to ambient temperature and reacted for 2 h. After the reaction was stopped, saturated ammonium chloride solution was added to the reaction liquid to quench the reaction, and the obtained mixture was adjusted to pH = 10 with 1 M sodium hydroxide solution. The obtained mixture was extracted with dichloromethane (50 mL × 3). The organic phases were combined, dried over anhydrous sodium sulfate, and filtered, and the filtrate was concentrated under reduced pressure to obtain the title product 2-(4-chloro-2-fluorophenyl)-2-methyl-8-(piperidin-4-yl)chroman-4-ol **14d** (1.88 g), yield: 93.5%, the compound was directly used for the next reaction without purification.

MS m/z (ESI): 376.1 [M+1].

### Step 4

### 4-(2-(4-chloro-2-fluorophenyl)-2-methyl-2H-chromen-8-yl)piperidine

2-(4-chloro-2-fluorophenyl)-2-methyl-8-(piperidin-4-yl)chroman-4-ol **14d** (1.50 g, 3.99 mmol) and p-toluenesulfonic acid (2.06 g, 11.97 mmol) were dissolved in toluene (20 mL). The reaction liquid was heated to 100°C and reacted under stirring for 30 minutes. After the reaction was stopped, the reaction liquid was cooled to room temperature, and 2 M sodium hydroxide solution was added thereto to quench the reaction. The organic layer was separated, and the aqueous layer was extracted with dichloromethane (30 mL × 3). The organic phases were combined, dried over anhydrous sodium sulfate, and filtered, and the filtrate was concentrated under reduced pressure to obtain the title product 4-(2-(4-chloro-2-fluorophenyl)-2-methyl-2H-chromen-8-yl)piperidine **14e** (1.02 g), yield: 71.4%, the compound was directly used for the next reaction without purification.

MS m/z (ESI): 358.1 [M+1].

### Step 5

### Methyl 2-((4-((R)-2-(4-chloro-2-fluorophenyl)-2-methyl-2H-chromen-8-yl)piperidin-1-yl)methyl)-3-(((S)-oxetan-2-yl)methyl)-3H-imidazolo[4,5-blpyridine-5-carboxylate

Referring to the synthesis method of the step 5 of example **13,** 4-(2-(4-chloro-2-fluorophenyl)-2-methyl-2H-chromen-8-yl)piperidine **14e** (500 mg, 1.40 mmol), methyl (S)-2-(chloromethyl)-3-(oxetan-2-ylmethyl)-3H-imidazolo[4,5-b]pyridine-5-carboxylate **Im-2** (413 mg, 1.40 mmol), anhydrous potassium carbonate (580 mg, 4.20 mmol), and acetonitrile (10 mL) were dosed to obtain the title product methyl 2-((4-((R)-2-(4-chloro-2-fluorophenyl)-2-methyl-2H-chromen-8-yl)piperidin-1-yl)methyl)-3-(((S)-oxetan-2-yl)methyl)-3H-imidazolo[4,5-b]pyridine-5-carboxylate **17f** (263 mg), yield: 30.5%.

MS m/z (ESI): 617.2 [M+1].

### Step 6

### 2-((4-((R)-2-(4-chloro-2-fluorophenyl)-2-methyl-2H-chromen-8-yl)piperidin-1-yl)methyl)-3-(((S)-oxetan-2-yl)methyl)-3H-imidazolo[4,5-b]pyridine-5-carboxylic acid

Referring to the synthesis method of the step 6 of example **13,** methyl 2-((4-((R)-2-(4-chloro-2-fluorophenyl)-2-methyl-2H-chromen-8-yl)piperidin-1-yl)methyl)-3-(((S)-oxetan-2-yl)methyl)-3H-imidazolo[4,5-b]pyridine-5-carboxylate **14f** (100 mg, 162 µmol) and lithium hydroxide (39 mg, 1.62 mmol) were dosed to obtain the title product 2-((4-((R)-2-(4-chloro-2-fluorophenyl)-2-methyl-2H-chromen-8-yl)piperidin-1-yl)methyl)-3-(((S)-oxetan-2-yl)methyl)-3H-imidazolo[4,5-b]pyridine-5-carboxylic acid 14 (76 mg), yield: 77.8%.

MS m/z (ESI): 603.2 [M+1].

### Example 15

### 2-((4-((R)-2-(4-chloro-2-(methoxy-d3)phenyl)-2-methyl-2H-chromen-8-yl)piperidin-1-yl)methyl)-3-(((S)-oxetan-2-yl)methyl)- 1H-benzo[d]imidazole-6-carboxylic acid

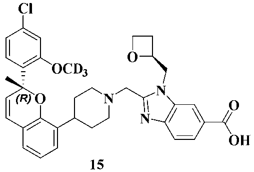

Reference to the synthesis method of example **12, Im-1** was used as the starting material to obtain the title product 2-((4-((R)-2-(4-chloro-2-(methoxy-d3)phenyl)-2-methyl-2H-chromen-8-yl)piperidin-1-yl)methyl)-3-(((S)-oxetan-2-yl)methyl)- 1H-benzo[d]imidazole-6-carboxylic acid.

MS m/z (ESI): 617.3 [M+1].

### Example 16

### 2-((4-((R)-2-(4-chloro-2-fluorophenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

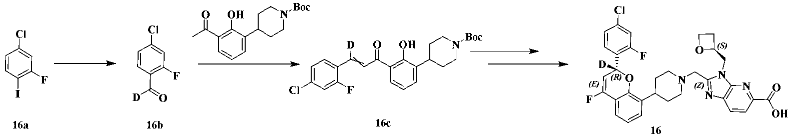

### Step 1

### 4-chloro-2-fluoro<α-2H>benzaldehyde

4-chloro-2-fluoro-1-iodo-benzene (5 g, 19.50 mmol) and toluene (50 mL) were added to a 100 mL flask, and isopropanol magnesium chloride (12.9 mL, 3 M, 38.99 mmol) was added at -30°C, and the reaction liquid was reacted at -20°C for 2h. Then N,N-dimethylformamide-D7 (3.12 g, 38.99 mmol) was added to the reaction liquid, and the reaction was continued at 0°C for 1 h. After the reaction was stopped, the reaction liquid was quenched with a saturated ammonium chloride aqueous solution. Water (50 mL) was added, and the obtained mixture was extracted with ethyl acetate (20 mL × 3). The organic phase was washed with saturated sodium chloride solution (20 mL), dried over anhydrous sodium sulfate, filtered, and dried by rotary evaporation. The resulting residue was purified by silica gel column chromatography with petroleum ether and ethyl acetate as an eluent system to obtain the title product 4-chloro-2-fluoro<α-2H>benzaldehyde **16b** (2.7 g), yield: 86.79%.

MS m/z (ESI): 160.0 [M+1].

### Step 2

### Tert-butyl 4-(3-(3-(4-chloro-2-fluorophenyl)acryloyl-3-d-3-d)-2-hydroxyphenyl)piperidine-1-carboxylate

4-chloro-2-fluoro<α-2H>benzaldehyde **16b** (1.5 g, 9.40 mmol) , tert-butyl 4-(3-acetyl-2-hydroxyphenyl)piperidine-1-carboxylate **Im-4** (3.00 g, 9.40 mmol) and tetrahydrofuran (40 mL) were added to a 100 mL flask, and sodium hydride (1.13 g, 28.20 mmol, 60% purity) was added at 0°C, then the reaction liquid was reacted at 25°C for 3h. After the reaction was stopped, the reaction liquid was quenched with a saturated ammonium chloride aqueous solution. Water (20 mL) was added, and the obtained mixture was extracted with ethyl acetate (20 mL × 3). The organic phase was washed with saturated sodium chloride solution (20 mL), dried over anhydrous sodium sulfate, filtered, and dried by rotary evaporation. The resulting residue was purified by silica gel column chromatography with petroleum ether and ethyl acetate as an eluent system to obtain the title product tert-butyl 4-(3-(3-(4-chloro-2-fluorophenyl)acryloyl-3-d-3-d)-2-hydroxyphenyl)piperidine-1-carboxylate **16c** (3 g), yield: 69.23%.

MS m/z (ESI): 461.1 [M+1].

### Step 3

### 2-((4-((R)-2-(4-chloro-2-fluorophenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

Tert-butyl 4-(3-(3-(4-chloro-2-fluorophenyl)acryloyl-3-d-3-d)-2-hydroxyphenyl)piperidine-1-carboxylate **16c** was used as the raw material, and the synthesis method of the step 3 to step 6 of example 36 was referenced to obtain 2-((4-((R)-2-(4-chloro-2-fluorophenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid **16.**

MS m/z (ESI):608.1 [M+1].

¹H NMR (400 MHz, DMSO-d6) δ 8.10 (d, 1H), 7.97 (d, 1H), 7.54 (dd, 1H), 7.41 (t, 1H), 7.29 (dd, 1H), 7.24 - 7.15 (m, 2H), 6.96(t, 1H), 5.67 (d, 1H), 5.20 - 5.11 (m, 1H), 4.88 - 4.79 (m, 1H), 4.74 - 4.65 (m, 1H), 4.53 - 4.43 (m, 1H), 4.41 - 4.31 (m, 1H), 3.97 (d, 1H), 3.88 (d, 1H), 2.98 - 2.91 (m, 1H), 2.87 - 2.79 (m, 1H), 2.75 - 2.62 (m, 2H), 2.49 - 2.41 (m, 1H), 2.25 - 2.07 (m, 2H), 1.73 - 1.59 (m, 2H), 1.54 - 1.41 (m, 1H), 1.37 - 1.29 (m, 1H).

### Example 17

### 2-((4-((R)-2-(4-chloro-2-(methoxy-d3)phenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

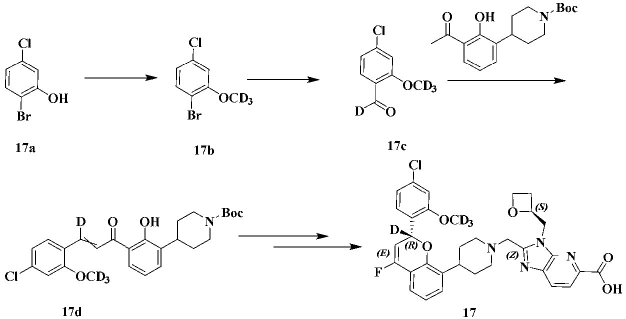

### Step 1

### 1-bromo-4-chloro-2-(methoxy-d3)benzene

2-bromo-5-chlorophenol **17a** was used as the raw material, and the synthesis method of the step 1 of example 36 was referenced to obtain 1-bromo-4-chloro-2-(methoxy-d3)benzene **17b.**

MS m/z (ESI):223.9 [M+1].

### Step 2

### 4-chloro-2-(methoxy-d3) <α-2H>benzaldehyde

1-bromo-4-chloro-2-(methoxy-d3)benzene **17b** was used as the raw material, and the synthesis method of the step 1 of example 16 was referenced to obtain 4-chloro-2-(methoxy-d3) <α-2H>benzaldehyde **17c.**

MS m/z (ESI):175.0 [M+1].

### Step 3

### Tert-butyl 4-(3-(3-(4-chloro-2-(methoxy-d3)phenyl)acryloyl-3-d-3-d)-2-hydroxyphenyl)piperidine-1-carboxylate

4-chloro-2-(methoxy-d3) <α-2H>benzaldehyde **17c** was used as the raw material, and the synthesis method of the step 2 of example 16 was referenced to obtain tert-butyl 4-(3-(3-(4-chloro-2-(methoxy-d3)phenyl)acryloyl-3-d-3-d)-2-hydroxyphenyl)piperidine-1-carboxylate **17d.**

MS m/z (ESI):476.2 [M+1].

### Step 4

### 2-((4-((R)-2-(4-chloro-2-(methoxy-d3)phenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid

Tert-butyl 4-(3-(3-(4-chloro-2-(methoxy-d3)phenyl)acryloyl-3-d-3-d)-2-hydroxyphenyl)piperidine-1-carboxylate **17d** was used as the raw material, and the synthesis method of the step 3 to step 6 of example 12 was referenced to obtain 2-((4-((R)-2-(4-chloro-2-(methoxy-d3)phenyl)-4-fluoro-2H-chromen-8-yl-2-d)piperidin-1-yl)methyl)-3-(((S)-oxetan-2-yl)methyl)-3H-imidazolo[4,5-b]pyridine-5-carboxylic acid **17.**

MS m/z (ESI):623.2 [M+1].

¹H NMR (400 MHz, Methanol-d4) δ 8.14 - 8.04 (m, 2H), 7.31 (d, 1H), 7.20 - 7.13 (m, 2H), 7.05 (d, 1H), 6.96 - 6.87 (m, 2H), 5.36 (d, 1H), 5.29 - 5.24 (m, 1H), 5.03 - 4.93 (m, 1H), 4.82 - 4.75 (m, 1H), 4.65 - 4.55 (m, 1H), 4.45 - 4.36 (m, 1H), 4.20 (d, 1H), 4.10 (d, 1H), 3.23 - 3.15 (m, 1H), 3.09 - 3.02 (m, 1H), 2.91 - 2.86 (m, 1H), 2.81 - 2.72 (m, 1H), 2.57 - 2.37 (m, 3H), 1.88 - 1.78 (m, 2H), 1.71 - 1.63 (m, 1H), 1.59 - 1.52 (m, 1H).

### Example 18

### 2-((4-((R)-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylic acid

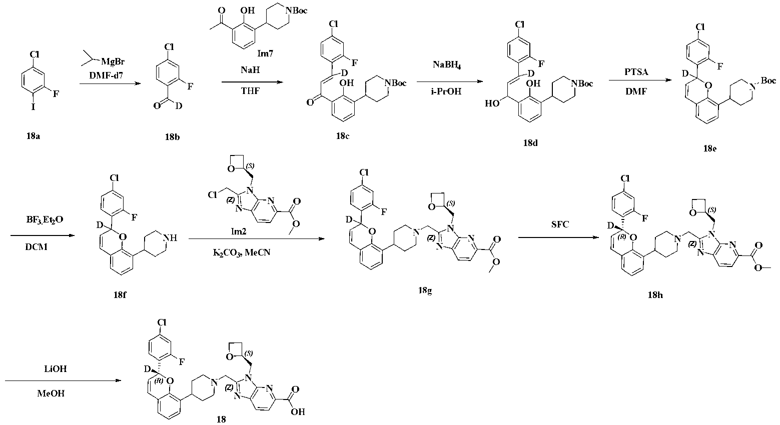

### Step 1

### 4-chloro-2-fluoro<α-2H>benzaldehyde

4-chloro-2-fluoro-1-iodo-benzene **18a** (5 g, 19.50 mmol) and toluene (50 mL) were added to a 100 mL flask, and isopropanol magnesium chloride (12.9 mL, 3 M, 38.99 mmol) was added at -30°C, and the reaction liquid was reacted at -20°C for 2 h. Then N,N-dimethylformamide-D7 (3.12 g, 38.99 mmol) was added to the reaction liquid, and the reaction was continued at 0°C for 1 h. After the reaction was stopped, the reaction liquid was quenched with a saturated ammonium chloride aqueous solution. Water (50 mL) was added, and the obtained mixture was extracted with ethyl acetate (20 mL × 3). The organic phase was washed with saturated sodium chloride solution (20 mL), dried over anhydrous sodium sulfate, filtered, and dried by rotary evaporation. The resulting residue was purified by silica gel column chromatography with petroleum ether and ethyl acetate as an eluent system to obtain the title product 4-chloro-2-fluoro<α-2H>benzaldehyde **18b** (2.7 g), yield: 86.79%.

MS m/z (ESI): 160.0 [M+1].

### Step 2

### Tert-butyl 4-(3-(3-(4-chloro-2-fluorophenyl)acryloyl-3-d-3-d)-2-hydroxyphenyl)piperidine-1-carboxylate

4-chloro-2-fluoro<α-2H>benzaldehyde **18b** (1.5 g, 9.40 mmol) , tert-butyl 4-(3-acetyl-2-hydroxyphenyl)piperidine-1-carboxylate **Im-4** (3.00 g, 9.40 mmol) and tetrahydrofuran (40 mL) were added to a 100 mL flask, and sodium hydride (1.13 g, 28.20 mmol, 60% purity) was added at 0°C, then the reaction liquid was reacted at 25°C for 3 h. After the reaction was stopped, the reaction liquid was quenched with a saturated ammonium chloride aqueous solution. Water (20 mL) was added, and the obtained mixture was extracted with ethyl acetate (20 mL × 3). The organic phase was washed with saturated sodium chloride solution (20 mL), dried over anhydrous sodium sulfate, filtered, and dried by rotary evaporation. The resulting residue was purified by silica gel column chromatography with petroleum ether and ethyl acetate as an eluent system to obtain the title product tert-butyl 4-(3-(3-(4-chloro-2-fluorophenyl)acryloyl-3-d-3-d)-2-hydroxyphenyl)piperidine-1-carboxylate **18c** (3 g), yield: 69.23%.

MS m/z (ESI): 461.1 [M+1].

### Step 3

### Tert-butyl 4-(3-(3-(4-chloro-2-fluorophenyl)-1-hydroxyallyl-3-d)-2-hydroxyphenyl)piperidine-1-carboxylate

Tert-butyl 4-(3-(3-(4-chloro-2-fluorophenyl)acryloyl-3-d-3-d)-2-hydroxyphenyl)piperidine-1-carboxylate **18c** (3 g, 6.51 mmol) and isopropanol (50 mL) were added to a 250 mL flask, and sodium borohydride (369.33 mg, 9.76 mmol) was added at 0°C. The reaction liquid was then reacted at 20°C for 3 h. After the reaction was stopped, the reaction liquid was quenched with water (10 mL), and the obtained mixture was extracted with ethyl acetate (20 mL × 3). The organic phase was washed with saturated sodium chloride solution (20 mL), dried over anhydrous sodium sulfate, filtered, and dried by rotary evaporation to obtain the title product tert-butyl 4-(3-(3-(4-chloro-2-fluorophenyl)-1-hydroxyallyl-3-d)-2-hydroxyphenyl)piperidine-1-carboxylate **18d** (3 g), yield: 99.56%.

MS m/z (ESI): 463.1 [M+1].

### Step 4

### Tert-butyl 4-(2-(4-chloro-2-fluorophenyl)-2H-chromen-8-yl-2-d)piperidine-1-carboxylate

Tert-butyl 4-(3-(3-(4-chloro-2-fluorophenyl)-1-hydroxyallyl-3-d)-2-hydroxyphenyl)piperidine-1-carboxylate **18d** (3 g, 6.48 mmol) and N,N-dimethylformamide (30 mL) were added to a 100 mL flask, and p-toluenesulfonic acid (1.67 g, 9.72 mmol) was added at 25°C. The reaction liquid was then reacted at 50°C for 4 h. After the reaction was stopped, the reaction liquid was quenched with water (20 mL), and the obtained mixture was extracted with ethyl acetate (20 mL × 3). The organic phase was washed with saturated sodium chloride solution (20 mL), dried over anhydrous sodium sulfate, filtered, and dried by rotary evaporation. The resulting residue was purified by silica gel column chromatography with petroleum ether and ethyl acetate as an eluent system to obtain the title product tert-butyl 4-(2-(4-chloro-2-fluorophenyl)-2H-chromen-8-yl-2-d)piperidine-1-carboxylate **18e** (1.7 g), yield: 58.96%.

MS m/z (ESI): 445.1 [M+1].

### Step 5

### 4-(2-(4-chloro-2-fluorophenyl)-2H-chromen-8-yl-2-d)piperidine

Tert-butyl 4-(2-(4-chloro-2-fluorophenyl)-2H-chromen-8-yl-2-d)piperidine-1-carboxylate **18e** (1.7 g, 3.82 mmol) and dichloromethane (25 mL) were added to a 100 mL flask, and 4A molecular sieve (1.7 g) and boron trifluoride diethyl etherate (1.36 g, 9.55 mmol) were added at 0°C. The reaction liquid was then reacted at 0°C for 2 h. After the reaction was stopped, the reaction liquid was quenched with saturated sodium bicarbonate aqueous solution (10 mL). Water (10 mL) was added, and the obtained mixture was extracted with dichloromethane (20 mL × 3). The organic phase was washed with saturated sodium chloride solution (20 mL), dried over anhydrous sodium sulfate, filtered, and dried by rotary evaporation to obtain the title product 4-(2-(4-chloro-2-fluorophenyl)-2H-chromen-8-yl-2-d)piperidine **18f** (1.3 g), yield: 98.67%.

MS m/z (ESI): 345.1 [M+1]

### Step 6

### Methyl 2-((4-(-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate

4-(2-(4-chloro-2-fluorophenyl)-2H-chromen-8-yl-2-d)piperidine **18f** (1.3 g, 3.77 mmol) and acetonitrile (15 mL) were added to a 100 mL flask, and potassium carbonate (1.04 g, 7.54 mmol) and methyl (S)-2-(chloromethyl)-3-(oxetan-2-ylmethyl)-3H-imidazolo[4,5-b]pyridine-5-carboxylate **Im2** (1.11 g, 3.77 mmol) were added at 25°C. The reaction liquid was then reacted at 25°C for 10 h. After the reaction was stopped, the reaction liquid was quenched with aqueous solution (10 mL), and the obtained mixture was extracted with dichloromethane (10 mL × 3). The organic phase was washed with saturated sodium chloride solution (10 mL), dried over anhydrous sodium sulfate, filtered, and dried by rotary evaporation. The resulting residue was purified by silica gel column chromatography with petroleum ether and ethyl acetate as an eluent system to obtain the title product methyl 2-((4-(-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate **18g** (1.7 g), yield: 74.65%.
MS m/z (ESI): 604.2 [M+1]

### Step 7

### Methyl 2-((4-((R)-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate

Methyl 2-((4-(-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate **18g** was subjected to chiral resolution to obtain methyl 2-((4-((R)-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate **18h.**
**Resolution conditions:** D-H 4.6*250
Hexane : EtOH : MeOH : DEA = 70 : 15 : 15 : 0.1%
F=1mL T = 35°C

### Step 8

### 2-((4-((R)-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylic acid

Methyl 2-((4-((R)-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylate **18h** (100 mg, 165.54 µmol) and methanol (2 mL) were added to a 25 mL flask, and lithium hydroxide (39.64 mg, 1.66 mmol) was dissolved in water (1 mL), and the mixture was added dropwise to the reaction liquid at 25°C. The reaction liquid was then reacted at 25°C for 1 h. After the reaction was stopped, the reaction liquid was quenched with formic acid (0.1 mL). Water (2 mL) was added, and the obtained mixture was extracted with dichloromethane (2 mL × 3). The organic phase was washed with saturated sodium chloride solution (2 mL), dried over anhydrous sodium sulfate, filtered, and dried by rotary evaporation. The resulting residue was purified by prep-HPLC to obtain the title product 2-((4-((R)-2-(4-chloro-2-fluorophenyl)-2H-chromen-8-yl-2-d)piperidin-1-yl)methyl)-3-(((S)-oxetan-2-yl)methyl)-3H-imidazolo[4,5-b]pyridine-5-carboxylic acid **18** (70 mg), yield: 71.66%.
MS m/z (ESI): 590.2 [M+1]

¹H NMR (400 MHz, DMSO) δ 8.08 (d, 1H), 7.96 (d, 1H), 7.52 (dd, 1H), 7.34 (t, 1H), 7.27 (dd, 1H), 7.05 (dd, 1H), 6.98 (dd, 1H), 6.85 (t, 1H), 6.75 (d, 1H), 5.91 (d, 1H), 5.18 - 5.11 (m, 1H), 4.87 - 4.80 (m, 1H), 4.72 - 4.65 (m, 1H), 4.53 - 4.43 (m, 1H), 4.39 - 4.31 (m, 1H), 3.96 (d, 1H), 3.87 (d, 1H), 3.01 - 2.90 (m, 1H), 2.89 - 2.79 (m, 1H), 2.77 - 2.61 (m, 2H), 2.48 - 2.45 (m, 1H), 2.27 - 2.08 (m, 2H), 1.73 - 1.61 (m, 2H), 1.54 - 1.42 (m, 1H), 1.42 - 1.34 (m, 1H).

### Example 19

### 2-((4-((R)-2-(4-chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylic acid

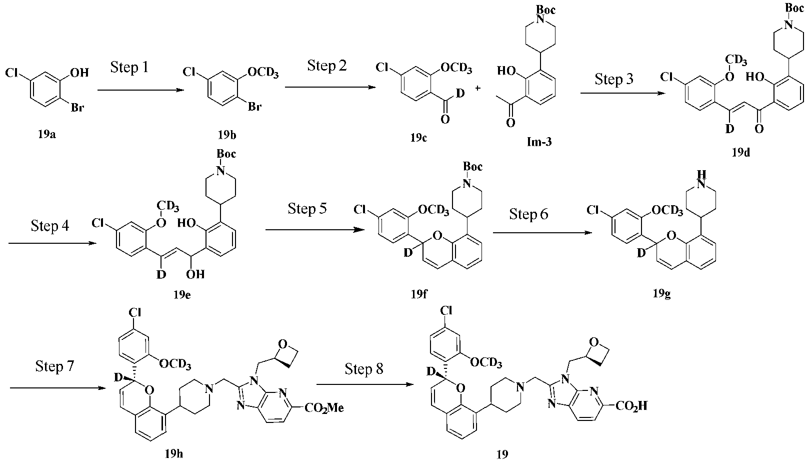

### Step 1

### 1-bromo-4-chloro-2-(methoxy-d3)benzene

2-bromo-5-chlorophenol **19a** (10 g, 48.20 mmol), deuterated iodomethane (10.48 g, 72.30 mmol), anhydrous potassium carbonate (13.33 g, 96.41 mol) were dispersed in DMF (100 mL). The reaction liquid was heated to 50°C and reacted under vigorous stirring for 12 h. After the reaction was stopped, the reaction liquid was filtered through diatomaceous earth, and the filtrate was concentrated under reduced pressure. The resulting residue was purified by silica gel column chromatography with petroleum ether and ethyl acetate as an eluent system to obtain the title product 1-bromo-4-chloro-2-(methoxy-d3)benzene **19b** (10 g), yield: 92.5%.

MS m/z (ESI): 223.9 [M+1].

### Step 2

### 4-chloro-2-(methoxy-d3)benzaldehyde-d

1-bromo-4-chloro-2-(methoxy-d3)benzene **19b** (1.6 g, 7.13 mmol) was dissolved in tetrahydrofuran (30 mL), and after nitrogen displacement, n-BuLi (2.5 M, 3.42 mL) was slowly added dropwise thereto at -78°C. After the dropwise addition was completed, stirring was maintained at -78°C for 1 h, and then N,N-dimethylformamide-D7 (856.71 mg, 10.69 mmol) was added dropwise thereto. The reaction system was further stirred for 3 h and naturally warmed to room temperature. Saturated ammonium chloride solution (20 mL) was slowly added dropwise thereto to quench the reaction, and then the obtained mixture was extracted with ethyl acetate (30 mL x 3), washed with saturated sodium chloride aqueous solution (30 mL x 2), dried over anhydrous sodium sulfate, filtered, and concentrated to obtain the 4-chloro-2-(methoxy-d3)-benzaldehyde-d **19c** (1.2 g, 6.87 mmol) as white solid, yield: 96.42%.

MS m/z (ESI): 175.0 [M+1].

### Step 3

### Tert-butyl (E)-4-(3-(3-(4-chloro-2-(methoxy-d3)phenyl)acryloyl-3-d-3-d)-2-hydroxyphenyl)piperidine-1-carboxylate

4-chloro-2-(methoxy-d3)-benzaldehyde-d **19c** (700 mg, 4.01 mmol) and tert-butyl-4-(3-acetyl-2-hydroxyphenyl)piperidine-1-carboxylate **Im-4** (1.28 g, 4.01 mmol) were dissolved in tetrahydrofuran (40 mL), and sodium hydride (481.05 mg, 12.03 mmol, 60% purity) was added thereto in portions in an ice-water bath. The reaction system was stirred in an ice-water bath for 0.5 h, and then placed at room temperature 20°C and stirred for 2.5 h. After the reaction was completed, the reaction was quenched by dropwise adding water (20 mL), extracted with ethyl acetate (30 mL × 3), washed with a saturated sodium chloride aqueous solution (30 mL × 2), dried over anhydrous sodium sulfate, filtered, and concentrated to obtain a crude product. The crude product was purified by silica gel column chromatography with petroleum ether and ethyl acetate as an eluent system to obtain the title product tert-butyl (E)-4-(3-(3-(4-chloro-2-(methoxy-d3)phenyl)acryloyl-3-d-3-d)-2-hydroxyphenyl)piperidine-1-carboxylate **19d** (1.5 g). Yield: 78.6%

MS m/z (ESI): 476.2 [M+1].

### Step 4

### Tert-butyl (E)-4-(3-(3-(4-chloro-2-(methoxy-d3)phenyl)-1-hydroxyallyl-3-d)-2-hydroxyphenyl)piperidine-1-carboxylate

Tert-butyl (E)-4-(3-(3-(4-chloro-2-(methoxy-d3)phenyl)acryloyl-3-d-3-d)-2-hydroxyphenyl)piperidine-1-carboxylate **19d** (1.4 g, 2.94 mmol) was dissolved in tetrahydrofuran (30 mL), and sodium borohydride (333.82 mg, 8.82 mmol) was added thereto in portions at room temperature. The reaction system was stirred for 2 h at room temperature. After the reaction was completed, water (20 mL) was added dropwise to quench the reaction, and the obtained mixture was extracted with ethyl acetate (30 mL x 3), washed with saturated sodium chloride aqueous solution (30 mL x 2), dried over anhydrous sodium sulfate, filtered, and concentrated to obtain the title product tert-butyl (E)-4-(3-(3-(4-chloro-2-(methoxy-d3)phenyl)-1-hydroxyallyl-3-d)-2-hydroxyphenyl)piperidine-1-carboxylate **19e** (1.4 g).

MS m/z (ESI): 478.2 [M+1].

### Step 5

### Tert-butyl 4-(2-(4-chloro-2-(methoxy-d3)phenyl)-2H-chromen-8-yl-2-d)piperidine-1-carboxylate

Tert-butyl (E)-4-(3-(3-(4-chloro-2-(methoxy-d3)phenyl)-1-hydroxyallyl-3-d)-2-hydroxyphenyl)piperidine-1-carboxylate **19e** (1.40 g, 2.93 mmol) was dissolved in dichloromethane (20 mL), and p-toluenesulfonic acid (151.62 mg, 880.49 µmol) was added thereto. The reaction system was stirred for 3 h at room temperature. After the reaction was completed, the solvent was directly concentrated to obtain a crude product. The crude product was purified by silica gel column chromatography with petroleum ether and ethyl acetate as an eluent system to obtain the title product tert-butyl 4-(2-(4-chloro-2-(methoxy-d3)phenyl)-2H-chromen-8-yl-2-d)piperidine-1-carboxylate **19f** (600 mg, 1.30 mmol), yield 44.4%.

MS m/z (ESI): 460.2 [M+1].

### Step 6

### 4-(2-(4-chloro-2-(methoxy-d3)phenyl)-2H-chromen-8-yl-2-d)piperidine

Tert-butyl 4-(2-(4-chloro-2-(methoxy-d3)phenyl)-2H-chromen-8-yl-2-d)piperidine-1-carboxylate **19f** (260 mg, 565.22 µmol) was dissolved in dichloromethane (10 mL), and 4A° molecular sieve (260 mg) and boron trifluoride diethyl etherate (240.66 mg, 1.70 mmol) were added thereto in an ice-water bath. The reaction system was further stirred in the ice-water bath for 1 h. After the reaction was completed, saturated sodium bicarbonate solution (10 mL) was added dropwise to quench the reaction, and the obtained mixture was extracted with dichloromethane (20 mL x 3), washed with saturated sodium chloride aqueous solution (20 mL x 2), dried over anhydrous sodium sulfate, filtered, and concentrated to obtain the crude product 4-(2-(4-chloro-2-(methoxy-d3)phenyl)-2H-chromen-8-yl-2-d)piperidine **19g** (200 mg).

MS m/z (ESI): 360.2 [M+1].

### Step 7

### Methyl 2-((4-((R)-2-(4-chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylate

4-(2-(4-chloro-2-(methoxy-d3)phenyl)-2H-chromen-8-yl-2-d)piperidine **19g** (200 mg, 555.74 µmol, crude), methyl (S)-2-(chloromethyl)-3-(oxetan-2-ylmethyl)-3H-imidazolo[4,5-b]pyridine-5-carboxylate **Im-2** (180.17 mg, 611.31 µmol) and potassium carbonate (230.42 mg, 1.67 mmol) were dissolved in acetonitrile (10 mL). The reaction system was placed in an oil bath at 60°C and stirred for 4 h. After the reaction was completed, the reaction liquid was diluted by adding water (10 mL), extracted with ethyl acetate (20 mL × 3), washed with a saturated sodium chloride aqueous solution (20 mL × 2), dried over anhydrous sodium sulfate, filtered, and concentrated to obtain a crude product. The crude product was purified by silica gel column chromatography with petroleum ether and ethyl acetate as an eluent system, and then the purified resulting sample was sent to chiral preparative HPLC separation to obtain the title product methyl 2-((4-((R)-2-(4-chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylate **19h** (100 mg, 161.5 µmol), yield: 29.1%.

MS m/z (ESI): 619.2 [M+1].

### Step 8

### 2-((4-((R)-2-(4-chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylic acid

Methyl 2-((4-((R)-2-(4-chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylate **19h** (100 mg, 161.5 µmol) and lithium hydroxide (38.8 mg, 1.62 mmol) were dissolved in 9 mL of a mixed solvent of THF, water and methanol (4 : 4 : 1), and the mixture was reacted under stirring for 3 h. After the reaction was stopped, formic acid was added to adjust the pH = 6, and the reaction liquid was concentrated under reduced pressure. The resulting residue was purified by preparative HPLC to obtain the title product 2-((4-((R)-2-(4-chloro-2-(methoxy-d3)phenyl)-2H-chromen-8-yl-2-d)piperidin-1-yl)methyl)-3-(((S)-oxetan-2-yl)methyl)-3H-imidazolo[4,5-b]pyridine-5-carboxylic acid **19** (60 mg), yield: 61.5%.

MS m/z (ESI): 605.2 [M+1].

¹H NMR (400 MHz, DMSO) δ 8.08 (d, 1H), 7.96 (d, 1H), 7.22 (d, 1H), 7.14 (d, 1H), 7.02 (d, 1H), 6.99 - 6.91 (m, 2H), 6.82 (t, 1H), 6.66 (d, 1H), 5.86 (d, 1H), 5.17 - 5.11 (m, 1H), 4.86 - 4.77 (m, 1H), 4.73 - 4.65 (m, 1H), 4.51 - 4.45 (m, 1H), 4.38 - 4.30 (m, 1H), 3.97 - 3.84 (m, 2H), 2.98 - 2.91 (m, 1H), 2.87 - 2.81 (m, 1H), 2.76 - 2.64 (m, 2H), 2.47 - 2.45 (m, 1H), 2.24 - 2.09 (m, 2H), 1.70 - 1.64 (m, 2H), 1.50 - 1.38 (m, 2H).

### Example 20

### 2-((4-((S)-2-(4-chloro-2-fluorophenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

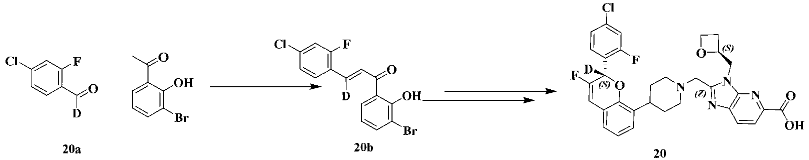

### Step 1

### 1-(3-bromo-2-hydroxyphenyl)-3-(4-chloro-2-fluorophenyl)prop-2-en-1-one-3-d

4-chloro-2-fluoro<α-2H>benzaldehyde**20a** and 1-(3-bromo-2-hydroxyphenyl)ethan-1-one were used as the raw materials, and the synthesis method of the step 1 of example 1 was referenced to obtain 1-(3-bromo-2-hydroxyphenyl)-3-(4-chloro-2-fluorophenyl)prop-2-en-1-one-3-d **20b.**

MS m/z (ESI):355.9 [M+1].

### Step 2

### 2-((4-((S)-2-(4-chloro-2-fluorophenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

1-(3-bromo-2-hydroxyphenyl)-3-(4-chloro-2-fluorophenyl)prop-2-en-1-one-3-d **20b** was used as the raw material, and the synthesis method of example 11 was referenced to obtain 2-((4-((S)-2-(4-chloro-2-fluorophenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid **20.**

MS m/z (ESI):608.1 [M+1].

### Example 21

### 2-((4-((S)-2-(4-chloro-2-(methoxy-d3)phenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

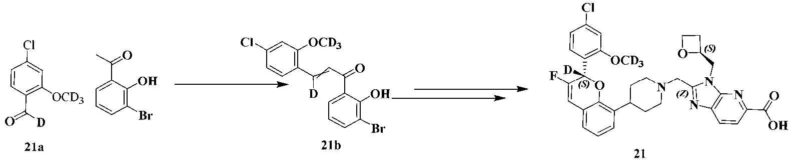

### Step 1

### 1-(3-bromo-2-hydroxyphenyl)-3-(4-chloro-2-(methoxy-d3)phenyl)prop-2-en-1-one-3-d

4-chloro-2-(methoxy-d3) <α-2H>benzaldehyde **21a** and 1-(3-bromo-2-hydroxyphenyl)ethan-1-one were used as the raw materials, and the synthesis method of the step 1 of example 1 was referenced to obtain 1-(3-bromo-2-hydroxyphenyl)-3-(4-chloro-2-(methoxy-d3)phenyl)prop-2-en-1-one-3-d **21b.**

MS m/z (ESI):371.0 [M+1].

### Step 2

### 2-((4-((S)-2-(4-chloro-2-(methoxy-d3)phenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid

1-(3-bromo-2-hydroxyphenyl)-3-(4-chloro-2-(methoxy-d3)phenyl)prop-2-en-1-one-3-d **21b** was used as the raw material, and the synthesis method of example 11 was referenced to obtain 2-((4-((S)-2-(4-chloro-2-(methoxy-d3)phenyl)-3-fluoro-2H-chromen-8-yl-2-d)piperidin-1-yl)methyl)-3-(((S)-oxetan-2-yl)methyl)-3H-imidazolo[4,5-b]pyridine-5-carboxylic acid **21.**

MS m/z (ESI):623.2 [M+1].

### Example 22

### 2-((4-((R)-2-(4-chloro-2-fluorophenyl)-4-fluoro-2H-chromen-8-yl-2-d)piperidin-1-yl)methyl)-1-(((S)-oxetan-2-yl)methyl)-1H-benzo[d]imidazole-6-carboxylic acid

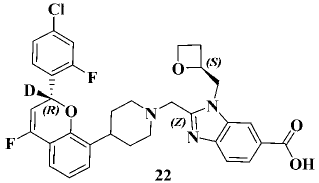

Intermediate Im1 was used as the raw material and example 16 was referenced to obtain the product 2-((4-((R)-2-(4-chloro-2-fluorophenyl)-4-fluoro-2H-chromen-8-yl-2-d)piperidin-1-yl)methyl)-1-(((S)-oxetan-2-yl)methyl)-1H-benzo[d]imidazole-6-carboxylic acid.

MS m/z (ESI): 607.2 [M+1].

### Example 23

### 2-((4-((R)-2-(4-chloro-2-fluorophenyl)-2H-chromen-8-yl-2-d)piperidin-1-yl)methyl)-1-(((S)-oxetan-2-yl)methyl)-1H-benzo[d]imidazole-6-carboxylic acid

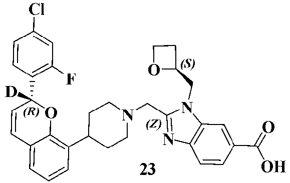

Intermediate Im1 was used as the raw material and example 18 was referenced to obtain the product 2-((4-((R)-2-(4-chloro-2-fluorophenyl)-2H-chromen-8-yl-2-d)piperidin-1-yl)methyl)-1-(((S)-oxetan-2-yl)methyl)-1H-benzo[d]imidazole-6-carboxylic acid.

MS m/z (ESI): 589.2 [M+1].

### Example 24

### 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid Method I

Example 18 was referenced to obtain the product 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid.

### Method II

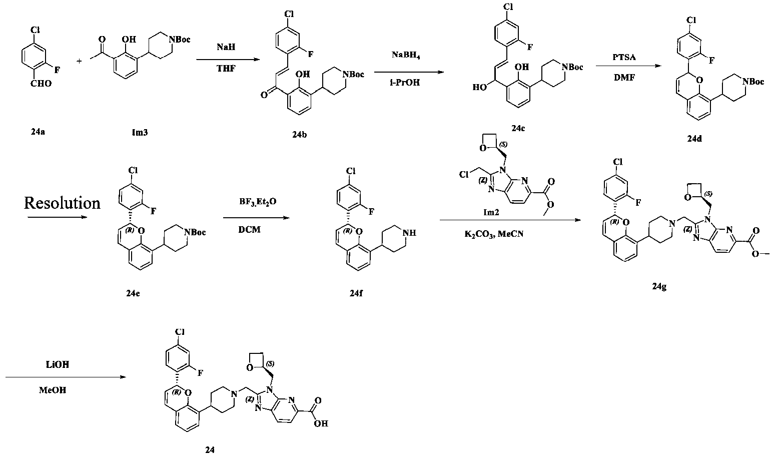

### Step 1

### Tert-butyl 4-(3-(3-(4-chloro-2-fluorophenyl)acryloyl)-2-hydroxyphenyl)piperidine-1-carboxylate

Tert-butyl 4-(3-acetyl-2-hydroxyphenyl)piperidine-1-carboxylate **Im4** (20.3 g, 63.56 mmol) and tetrahydrofuran (300 mL) were added to a 1000 mL flask, the mixture was cooled at 0°C for 10 minutes, and sodium hydride (7.63 g, 190.67 mmol, 60% purity) was added. Then 4-chloro-2-fluorobenzaldehyde **24a** (10.08 g, 63.56 mmol) was dissolved in THF (100 mL), and the mixture was slowly added dropwise into the reaction liquid through a constant pressure dropping funnel over 20 minutes. After the addition, the mixture was warmed to 25°C and reacted for 2 h. After the reaction was stopped, the reaction liquid was quenched with saturated ammonium chloride aqueous solution (200 mL). Water (50 mL) was added, and the obtained mixture was extracted with ethyl acetate (200 mL × 3). The organic phase was washed with saturated sodium chloride solution (200 mL), dried over anhydrous sodium sulfate, filtered, and dried by rotary evaporation. Ethyl acetate (60 mL) was added, and the mixture was slurried (stirred for 15 minutes) and filtered. The filter cake was washed with petroleum ether (30 mL) and dried to obtain the title product tert-butyl 4-(3-(3-(4-chloro-2-fluorophenyl)acryloyl)-2-hydroxyphenyl)piperidine-1-carboxylate **24b** (15.2 g), yield: 52.00%.

MS m/z (ESI): 460.1 [M+1].

### Step 2

### Tert-butyl 4-(3-(3-(4-chloro-2-fluorophenyl)-1-hydroxyallyl)-2-hydroxyphenyl)piperidine-1-carboxylate

Tert-butyl 4-(3-(3-(4-chloro-2-fluorophenyl)acryloyl)-2-hydroxyphenyl) piperidine-1-carboxylate **24b** (25 g, 54.36 mmol) and isopropanol (500 mL) were added to a 1000 mL flask, and sodium borohydride (3.08 g, 81.53 mmol) was added at 0°C. The reaction liquid was then reacted at 20°C for 3 h. After the reaction was stopped, the reaction liquid was quenched with water (100 mL), and the obtained mixture was extracted with ethyl acetate (200 mL × 3). The organic phase was washed with saturated sodium chloride solution (200 mL), dried over anhydrous sodium sulfate, filtered, and dried by rotary evaporation to obtain the title product tert-butyl 4-(3-(3-(4-chloro-2-fluorophenyl)-1-hydroxyallyl)-2-hydroxyphenyl) piperidine-1-carboxylate **24c** (25 g), yield: 99.56%.

MS m/z (ESI): 462.1 [M+1].

### Step 3

### Tert-butyl 4-(2-(4-chloro-2-fluorophenyl)-2H-chromen-8-yl)piperidine-1-carboxylate

Tert-butyl 4-(3-(3-(4-chloro-2-fluorophenyl)-1-hydroxyallyl)-2-hydroxyphenyl) piperidine-1-carboxylate **24c** (25 g, 54.12 mmol) and N,N-dimethylformamide (300 mL) were added to a 500 mL flask, and p-toluenesulfonic acid (13.98 g, 81.18 mmol) was added at 25°C. The reaction liquid was then reacted at 50°C for 4 h. After the reaction was stopped, the reaction liquid was quenched with water (200 mL), and the obtained mixture was extracted with ethyl acetate (200 mL × 3). The organic phase was washed with saturated sodium chloride solution (200 mL), dried over anhydrous sodium sulfate, filtered, and dried by rotary evaporation. The resulting residue was purified by silica gel column chromatography with petroleum ether and ethyl acetate as an eluent system to obtain the title product tert-butyl 4-(2-(4-chloro-2-fluorophenyl)-2H-chromen-8-yl)piperidine-1-carboxylate **24d** (13 g), yield: 54.11%.

MS m/z (ESI): 444.1 [M+1].

### Step 4

### Tert-butyl (R)-4-(2-(4-chloro-2-fluorophenyl)-2H-chromen-8-yl)piperidine-1-carboxylate

Tert-butyl 4-(2-(4-chloro-2-fluorophenyl)-2H-chromen-8-yl)piperidine-1-carboxylate **24d** was subjected to chiral separation to obtain tert-butyl (R)-4-(2-(4-chloro-2-fluorophenyl)-2H-chromen-8-yl)piperidine-1-carboxylate **24e.**
Resolution conditions
OD-H 4.6*150
Hexane: IPA : DEA =90 : 10 : 0.1%
F = 1mL T=35°C
MS m/z (ESI): 444.1 [M+1].

### Step 5

### (R)-4-(2-(4-chloro-2-fluorophenyl)-2H-chromen-8-yl)piperidine

Tert-butyl (R)-4-(2-(4-chloro-2-fluorophenyl)-2H-chromen-8-yl)piperidine-1-carboxylate **24e** (2.55 g, 5.74 mmol) and dichloromethane (50 mL) were added to a 100 mL flask, and 4A molecular sieve (2.55 g) and boron trifluoride diethyl etherate (2.04 g, 14.36 mmol) were added at 0°C. The reaction liquid was then reacted at 0°C for 2 h. After the reaction was stopped, the reaction liquid was quenched with saturated sodium bicarbonate aqueous solution (10 mL). Water (10 mL) was added, and the obtained mixture was extracted with dichloromethane (20 mL × 3). The organic phase was washed with saturated sodium chloride solution (20 mL), dried over anhydrous sodium sulfate, filtered, and dried by rotary evaporation to obtain the title product (R)-4-(2-(4-chloro-2-fluorophenyl)-2H-chromen-8-yl)piperidine **24f** (1.97 g, yield: 100%).
MS m/z (ESI): 344.1 [M+1]

### Step 6

### Methyl 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylate

(R)-4-(2-(4-chloro-2-fluorophenyl)-2H-chromen-8-yl)piperidine **24f** (1.97 g, 5.73 mmol) and acetonitrile (50 mL) were added to a 100 mL flask, and potassium carbonate (1.58 g, 11.46 mmol) and methyl (S)-2-(chloromethyl)-3-(oxetan-2-ylmethyl)-3H-imidazolo[4,5-b]pyridine-5-carboxylate **Im2** (1.69 g, 5.73 mmol) were added at 25°C. The reaction liquid was then reacted at 25°C for 10 h. After the reaction was stopped, the reaction liquid was quenched with aqueous solution (20 mL), and the obtained mixture was extracted with dichloromethane (20 mL × 3). The organic phase was washed with saturated sodium chloride solution (20 mL), dried over anhydrous sodium sulfate, filtered, and dried by rotary evaporation. The resulting residue was purified by silica gel column chromatography with petroleum ether and ethyl acetate as an eluent system to obtain the title product methyl 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylate **24g** (3.1 g), yield: 89.71%.
MS m/z (ESI): 603.2 [M+1]

### Step 7

### 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid

Methyl 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylate **24g** (3.1 g, 5.14 mmol) and methanol (25 mL) were added to a 100 mL flask, and lithium hydroxide (1.23 g, 51.40 mmol) was dissolved in water (10 mL), and the mixture was added dropwise to the reaction liquid at 25°C. The reaction liquid was then reacted at 25°C for 1 h. After the reaction was stopped, the reaction liquid was quenched with formic acid (1 mL). Water (20 mL) was added, and the obtained mixture was extracted with dichloromethane (20 mL × 3). The organic phase was washed with saturated sodium chloride solution (20 mL), dried over anhydrous sodium sulfate, filtered, and dried by rotary evaporation. The resulting residue was purified by prep-HPLC to obtain the title product 2-((4-((R)-2-(4-chloro-2-fluorophenyl)-2H-chromen-8-yl)piperidin-1-yl)methyl)-3-(((S)-oxetan-2-yl)methyl)-3H-imidazolo[4,5-b]pyridine-5-carboxylic acid **24** (2.1 g), yield: 69.35%.
MS m/z (ESI): 589.1 [M+1]

¹H NMR (400 MHz, MeOD) δ 8.14 - 8.03 (m, 2H), 7.40 (t, 1H), 7.25 (dd, 1H), 7.15 (dd, 1H), 7.05 (dd, 1H), 6.92 (dd, 1H), 6.84 (t, 1H), 6.68 (dd, 1H), 6.21 (dd, 1H), 5.82 (dd, 1H), 5.30 - 5.24 (m, 1H), 5.03 - 4.93 (m, 1H), 4.85 - 4.81 (m, 1H),4.64 - 4.54 (m, 1H), 4.45 - 4.35 (m, 1H), 4.23 (d, 1H), 4.13 (d, 1H), 3.25 - 3.18 (m, 1H), 3.12 - 3.05 (m, 1H),2.95 - 2.85 (m, 1H), 2.83 - 2.70 (m, 1H), 2.57 - 2.38 (m, 3H), 1.88 - 1.78 (m, 2H), 1.78 - 1.63 (m, 1H), 1.59 - 1.51 (m, 1H).

### Example 25

### 2-((4-((R)-2-(4-chloro-2-(methoxy-d3)phenyl)-4-fluoro-2H-chromen-8-yl-2-d)piperidin-1-yl)methyl)-1-(((S)-oxetan-2-yl)methyl)-1H-benzo[d]imidazole-6-carboxylic acid

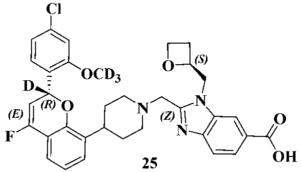

Example 17 was referenced to obtain the product 2-((4-((R)-2-(4-chloro-2-(methoxy-d3)phenyl)-4-fluoro-2H-chromen-8-yl-2-d)piperidin-1-yl)methyl)-1-(((S)-oxetan-2-yl)methyl)-1H-benzo[d]imidazole-6-carboxylic acid.

MS m/z (ESI): 622.2 [M+1].

### Example 26

### 2-((4-((R)-2-(4-chloro-2-(methoxy-d3)phenyl)-2H-chromen-8-yl-2-d)piperidin-1-yl)methyl)-1-(((S)-oxetan-2-yl)methyl)-1H-benzo[d]imidazole-6-carboxylic acid

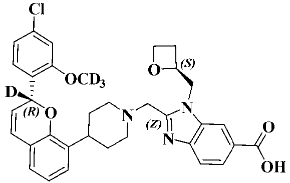

Example 19 was referenced to obtain the product 2-((4-((R)-2-(4-chloro-2-(methoxy-d3)phenyl)-2H-chromen-8-yl-2-d)piperidin-1-yl)methyl)-1-(((S)-oxetan-2-yl)methyl)-1H-benzo[d]imidazole-6-carboxylic acid.

MS m/z (ESI): 604.2 [M+1].

### Example 27

### 2-((4-((R)-2-(4-cyano-2-fluorophenyl)-2H-chromen-8-yl)piperidin-1-yl)methyl)-1-(((S)-oxetan-2-yl)methyl)- -1H-benzo[d]imidazole-6-carboxylic acid

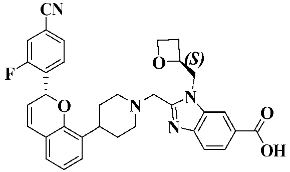

4-cyano-2-fluorobenzaldehyde and intermediate Im-1 were used as the raw materials, and Example 18 was referenced to obtain the product 2-((4-((R)-2-(4-cyano-2-fluorophenyl)-2H-chromen-8-yl)piperidin-1-yl)methyl)- 1-(((S)-oxetan-2-yl)methyl)- -1H-benzo[d]imidazole-6-carboxylic acid.

MS m/z (ESI): 579.2 [M+1].

¹H NMR (400 MHz, DMSO) δ 8.25 (d, 1H), 7.95 (dd, 1H), 7.79 (dd, 1H), 7.70 (dd, 1H), 7.62 (d, 1H), 7.52 (t, 1H), 7.07 (dd, 1H), 6.99 (dd, 1H), 6.87 (t, 1H), 6.75 (dd, 1H), 6.33 (dd, 1H), 5.93 (dd, 1H), 5.11 - 5.00 (m, 1H), 4.85 - 4.71 (m, 1H), 4.67 - 4.58 (m, 1H), 4.52 - 4.44 (m, 1H), 4.42 - 4.31 (m, 1H), 3.92 (d, 1H), 3.75 (d, 1H), 3.04 - 2.92 (m, 1H), 2.83 - 2.62 (m, 3H), 2.46 - 2.38 (m, 1H), 2.23 - 2.02 (m, 2H), 1.73 - 1.56 (m, 2H), 1.48 - 1.34 (m, 2H).

### Example 28

### 2-((4-((R)-2-(4-chloro-2-fluorophenyl)-2H-chromen-8-yl)piperidin-1-yl)methyl)-3-(((S)-oxetan-2-yl)methyl)- 1H-benzo[d]imidazole-6-carboxylic acid

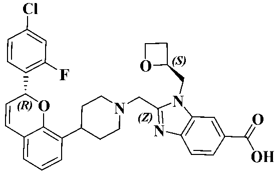

Im-1 was used as the raw materials and example 24 was referenced to obtain the product 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid.

MS m/z (ESI): 588.2 [M+1].

¹H NMR (400 MHz, MeOD) δ 8.30 (s, 1H), 7.96 (dd, 1H), 7.66 (d, 1H), 7.40 (t, 1H), 7.25 (s, 1H), 7.16 (d, 1H), 7.04 (dd, 1H), 6.92 (dd, 1H), 6.83 (d, 1H), 6.68 (dd, 1H), 6.23 - 6.19 (m, 1H), 5.83 (dd, 1H), 5.27 - 5.20 (m, 1H), 4.85 - 4.80 (m, 1H), 4.74 - 4.62 (m, 2H), 4.48 - 4.41 (m, 1H), 4.05 (d, 1H), 3.95 (d, 1H), 3.12 - 3.05 (m, 1H), 2.94 - 2.77 (m, 3H), 2.55 - 2.46 (m, 1H), 2.39 - 2.25 (m, 2H), 1.82 - 1.73 (m, 2H), 1.66 - 1.50 (m, 2H).

### Biological assay and evaluation

The present disclosure will be further described and explained below in conjunction with test examples, and these examples are not meant to limit the scope of the present disclosure.

### 1. Determination of the ability of the compounds of the present disclosure to stimulate the production of cAMP in human GLP1 receptor stably transfected cell line

### 1. Experimental objective:

The objective of this test example is to test the ability of a compound to activate human GLP-1 receptors on the cell surface. EC₅₀ of the generation of AMP due to stimulatation after agonism characterizes the ability of the compound to activate the human GLP-1 receptor.

### 2. Experimental reagents and instruments:

### 2.1 Experimental instruments:

Microplate reader (BioTek Synergy H1);
Pipette (Eppendorf & Rainin).

### 2.2 Experimental reagents:

DMEM/F12 medium was purchased from Gibco, with a catalog number of 11330032;
Casein was purchased from Sigma, with a catalog number of C3400;
384-well plate was purchased from Sigma, with a catalog number of CLS4514;
IBMX was purchased from Sigma, with a catalog number of 17018;
Cisbio cAMP - Gs Dynamic kit was purchased from Cisbio, with a catalog number of 62AM4PEC.

### 3. Experimental method:

The frozen human GLP1 receptor stably transfected cell line CHO-K1/GLP-1R/CRE-1uc was taken out from the liquid nitrogen tank, placed in a 37°C water bath kettle to thaw quickly, and resuspended with DMEM/F12 medium. The cells were washed once after centrifugation, resuspended with experimental buffer, that is, DMEM/F12 medium containing 0.1% casein, and the cell density was adjusted with experimental buffer, and the cells were plated onto a 384-well plate at a density of 2500 cells/5 µL/well, then 2.5 µL of IBMX working solution prepared with buffer was added to each well, the final concentration of IBMX was 0.5 mM, and 2.5 µL of serially diluted compound samples (starting from 1000 nM, 3-fold dilution, 11 concentrations) were also added, centrifuged at 1000 rpm for 1min, shaken for 30 seconds to mix uniformly, and stood at room temperature and incubated for 30 minutes. Cisbio cAMP - Gs Dynamic kit was used for detection, and cAMP-d2 and Anti-cAMP-Eu³⁺-Cryptate were respectively diluted 20 folds with cAMP Lysis & Detection Buffer and mixed uniformly. 5 µL of diluted cAMP-d2 solution was added to each well, then 5 µL of diluted Anti-cAMP- Eu3⁺-Cryptate solution was added, shaken for 30 seconds to mix uniformly, and incubated at room temperature in the dark for 1 h. Biotek Synergy H1 microplate reader was used to read HTRF signals, with an excitation wavelength of 320 nm and emission wavelengths of 620 nm and 665 nm.

### 4. Experimental data processing method:

The signal ratio (665 nm/620 nm*10,000) was calculated, and the nonlinear fitting of the signal ratio and sample concentration was performed using a fourparameter equation in GraphPad Prism 6 to obtain the EC₅₀ value.

### 5. Experimental results:

**Table 1**

| Example | EC50 (nM) |
|---|---|
| 1 | 0.13 |
| 2-A | 0.07 |
| 9-A | 0.07 |
| 10-A | 0.05 |
| 16 | 0.1 |
| 17 | 0.04 |
| 18 | 0.07 |
| 19 | 0.05 |
| 23 | 0.08 |
| 24 | 0.03 |
| 25 | 0.08 |
| 27 | 0.05 |
| 28 | 0.06 |

### 6. Experimental conclusion

According to the above scheme, the example compound of the present disclosure showed good biological activity in the experiment of stimulating the human GLP1 receptor stably transfected cell line to produce cAMP.

### II. Effects of single administration of the compound of the present disclosure on intraperitoneal glucose tolerance of GLP-1R humanized mice

1. Experimental objective:
   To evaluate the effects of a single administration of the compound of the present disclosure on blood glucose changes in the intraperitoneal glucose tolerance (ipGTT) experiment of GLP-1R humanized C57BL/6 mice.
2. Experiment materials:
   C57BL/6_*hGLP-1R,* male, 5-8 weeks; Ultra-clean bench; Electronic balance; Active blood glucose meter; Glucose.
3. Experimental operations and data processing:
   3.1 One day before the experiment, the animals were randomly divided into groups according to their body weights, with 5 animals in each group. All animals were deprived of food to fast overnight and fasted for more than 16 h before administration.
   3.2 A 0.2 g/mL glucose solution was prepared with pure water and filtered through a 0.22 µm filter membrane for later use.
   3.3 On the day of the test, the blood glucose level of each animal was measured in turn by tail-cutting method before administration, and recorded as the Baseline value; Blood glucose test method: The mouse was placed in a restrainer, the tail tip was disinfected with alcohol cotton balls, then a little of the tail tip was cut with scissors. The first drop of blood was discarded, and the second drop of blood was dropped onto the prepared blood glucose test paper to test the blood glucose value.
   3.4 Administration was performed according to the body weights of the animals and the administration time of each animal was recorded. 1 h after administration, the blood glucose level of each animal was measured in turn and recorded as the blood glucose level at 0 min;
   3.5 Subsequently, according to the body weight of the day, pure water or glucose solution was intraperitoneally injected immediately, with a volume of 10 mL/kg and a glucose dose of 2 g/kg;
   3.6 The blood glucose level of each mouse was respectively measured at 15, 30, 60, 90 and 120 min after the injection of pure water or glucose solution, and the time and data was recorded;
   3.7 After the test was completed, all animals resumed feeding.
   3.8 Data processing:
      The blood glucose (BG)-time curve was plotted, and the area under the blood glucose-time curve was calculated. The calculation formula was:
      AUC (mmol/L.hr)=(BG0+BG15)×0.25/2+(BG15+BG30)×0.25/2+(BG30+ BG60)×0.5/2 + (BG60+BG90)×0.5/2+ (BG90+BG120)×0.5/2.
      Note: BG0, BG15, BG30, BG60, BG90 and BG120 represent the blood glucose values before sugar administration (0 min), 15, 30, 60, 90 and 120 min after sugar administration, respectively.

The blood glucose reduction rate at each time point and AUC was calculated according to the average blood glucose value and blood glucose AUC at each time point. The calculation formula was: blood glucose reduction rate = (blood glucose in the administration group/AUC-blood glucose in the model control group/AUC)/blood glucose in the model control group/AUC×100%.

### 4 Experimental results:

| Compound | Dose (mg/kg) | Number of animals | Blood glucose AUC reduction rate% |
|---|---|---|---|
| Vehicle | - | 5 | - |
| Example 17 | 0.3 | 5 | 53.09 |
| Example 18 | 0.3 | 5 | 56.79 |
| Example 19 | 0.3 | 5 | 46.57 |
| Example 24 | 0.3 | 5 | 62.89 |

### 5. Experimental conclusion

According to the above experimental results, it can be seen that the example compounds of the present disclosure can effectively reduce blood glucose in mice.

### III. Effects of long-term administration of the compound of the present disclosure on the body weight and food intake of GLP-1R humanized mice fed with a high-fat diet

1. Experimental objective:
   The purpose of this test is to evaluate the effects of long-term administration of the compound on the body weight and food intake of GLP-1R humanized C57BL/6 mice fed with a high-fat diet.
2. Experimental reagents and instruments
   *C57BL*/*6_hGLP-1R,* male, 5-8 weeks; 60% high-fat diet (HFD); Ultra-clean bench; Electronic balance.
3. Experimental method
   3.1 On the day of starting high-fat diet feeding, C57BL/6 mice were randomly divided into two groups according to body weight. The first group of 7 mice was Blank, fed with normal control diet, and the remaining animals were modeling group, fed with high-fat diet until the end of the experiment.
   3.2 At the 8th week of HFD feeding, the animals of the modeling group were randomly divided into groups according to body weight, with 7 mice in each group. The first group was Vehicle group (Vehicle: 0.5% CMC-Na + 1% Tween 80), which was administered vehicle; The remaining group were administration group; Administration regimen: The corresponding compound was administered orally, with an administration cycle of 14 days, once a day, an administration dosage of 10 mg/kg, and an administration volume of 10 mL/kg. The Blank group continued to feed normal diet, and no administration operation was performed.
   3.3 The day of administration was defined as Day 0.
   3.4 At each administration, the animals were weighed and the data were recorded, and oral administration was performed according to body weight, with an administration volume of 10 mL/kg.
   3.5 Starting from the day 0 of the experiment, the food intake of each group of mice was measured every three days. Specifically, the feed was changed after each weighing and administration, and the added and remaining amounts were recorded.
   3.6 On Day 14, the end of the experiment, all mice were euthanized and dissected according to the grouping order, and the livers were removed and weighed.
4. Experimental data processing and statistical analysis
   The body weight and body weight change rate of mice after administration were summarized and statistically analyzed. Calculation of body weight change rate: (BWt - BW0)/ BW0 × 100%. BWt represents the body weight of mice on the day t of the experiment, and BW0 represents the body weight of mice on the day 0 of the experiment.

Calculation of food intake: (Added amount (g) - Remaining amount (g))/Number of animals per cage, the cumulative food intake was the sum of the daily food intake of each animal during the administration.

Experimental data were analyzed using GraphPad Prism software. The comparison between two groups was tested using t-test method. Comparisons among three or more groups were tested using one-way ANOVA method.

### 5. Experimental results

| **Compound** | **Dose (mg/kg)** | **Weight (g)-D0** | | | **Weight (g)-D10** | | | **Weight change (%)-D10** |
|---|---|---|---|---|---|---|---|---|
| | | **Mean** | ± | **SEM** | **Mean** | ± | **SEM** | **Mean** |
| Vehicle | - | 28.52 | | 0.54 | 28.28 | | 0.58 | -0.84% |
| Example 17 | 10 | 28.54 | ± | 0.37 | 26.20 | ± | 0.36 | -8.19% |
| Example 16 | 10 | 28.40 | ± | 0.67 | 26.01 | ± | 0.85 | -8.49% |
| Example 18 | 10 | 28.54 | ± | 0.46 | 26.04 | ± | 0.42 | -8.75% |
| Vehicle | - | 28.09 | | 0.81 | 28.23 | | 0.75 | 0.55% |
| Example 19 | 10 | 28.37 | ± | 1.06 | 26.57 | ± | 1.14 | -6.37% |
| Example 24 | 10 | 28.23 | ± | 0.37 | 25.85 | ± | 0.44 | -8.44% |
| Example 28 | 10 | 28.34 | ± | 0.46 | 25.65 | ± | 0.77 | -9.53% |

### 6. Experimental conclusion

According to the above experimental results, it can be seen that long-term administration of the example compound of the present disclosure has a good weight-reducing effect on GLP-1R humanized C57BL/6 mice fed with a high-fat diet.

### IV. Pharmacokinetic determination in SD rat

1. Study objective:
SD rats were used as test animals to study the pharmacokinetic behavior of the following compound examples in plasma of rat in vivo after oral administration at a dose of 50 mg/kg.
2. Experimental scheme
2.1 Experimental drugs:
Vehicle formula: 0.5% CMC-Na (1% Tween 80);
the examples of the present disclosure, made in house.

2.2 Experimental animals:
3 SD rats per group, male.
2.3 Administration:
3 SD rats per group, male, PO respectively at a dose of 50 mg/kg and an administration volume of 10 mL/kg after the rats were fasted overnight.
2.4 Sample collection:
Before and after administration, 0.2 mL of blood was collected from the jugular vein of rats at 0, 0.25, 0.5, 1, 2, 4, 6, 8 and 24 h and placed in EDTA-K₂ test tubes, which were centrifuged at 6000 rpm at 4°C for 6 min to separate plasma, and the plasma was stored at -80°C. The mice were fed at 4 h after administration.
2.5 Sample treatment:
1) 160 uL of acetonitrile was added to 40 uL of plasma sample for precipitation, and same was mixed and centrifuged at 3500 × g for 5 to 20 min.
2) 100 uL of the treated supernatant solution was taken and subjected to LC/MS/MS to analyze the concentrations of the test compound.

2.6 Liquid phase analysis
• Liquid phase conditions: Shimadzu LC-20AD pump
• Mass spectrometry conditions: AB Sciex API 4000 mass spectrometer
• Chromatographic column: Phenomenex Gemiu 5 um C18 50 × 4.6 mm
• Mobile phase: liquid A was 0.1% aqueous formic acid solution, and liquid B was methanol
• Flow rate: 1.0 mL/min
• Elution time: 0-4.0 min, the eluent is as follows:

| Time/min | Liquid A | Liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Experimental results and analysis
The main pharmacokinetic parameters were calculated using WinNonlin 8.2.
4. Experimental results

| Compound | tmax(h) | Cmax(ng/mL) | AUC0-t(ng/mL*h) | AUC0-∞ (ng/ml*h) | t1/2(h) |
|---|---|---|---|---|---|
| Example 2-A | 2.0 | 7157 | 35755 | 35838 | 3.1 |
| Example 16 | 1.0 | 2303 | 10311 | 10831 | 2.0 |
| Example 24 | 2.0 | 6403 | 19084 | 19114 | 3.8 |
| Example 27 | 1.0 | 9397 | 12550 | 12555 | 1.6 |
| Example 28 | 0.5 | 7980 | 21532 | 21556 | 2.0 |

5. Experimental conclusion:
As could be seen from the results of the pharmacokinetic experiment on rats in the table, the example compounds of the present disclosure, at a dose of 50 mg/kg, exhibited good metabolic properties and performed well on both exposure (AUC) and maximum blood drug concentration Cₘₐₓ.

### Study on salt and crystal form

### 1.1 Experimental instruments

### 1.1.1 Some parameters of physical and chemical testing instruments

| | | |
|---|---|---|
| XRPD | Instrument model | BRUKER D8 ADVANCE |
| | Diffracted ray | CuK (40 kV, 25 mA) |
| | Scan rate | 0.02°/S (2θ value) |
| | Scan range | 4° - 40°(2θ value) |
| DSC | Instrument model | NETZSCH DSC 214 polyma |
| | Purge gas | Nitrogen |
| | Purge rate | 40 mL/min |
| | Heating rate | 10°C/min |
| | Temperature range | 25°C-350°C |
| | Tray type | Aluminum tray |
| TGA | Instrument model | NETZSCH TG 209 Tarsus |
| | Purge gas | Nitrogen |
| | Purge rate | 40 mL/min |
| | Heating rate | 10°C/min |
| | Temperature range | Room temperature-400°C |
| | Tray type | Al₂O₃ |

### 1.2 Instrument and liquid phase analysis conditions

### 1.2.1 Instruments and equipment

| Name of instrument | Model |
|---|---|
| Analytical balance | METTLER TOLEDO XA105 |
| Water purifier | Milli-Q Plus, Millipore |
| High performance liquid chromatograph | Agilent1260 |
| Pump | Agilent G1311B |
| Sample injector | G1329B |
| Column oven | G1316A |
| Detector | G1315D |

### 1.2.2 Chromatographic conditions

| Instrument | Agilent 1260 | |
|---|---|---|
| Mobile phase A | A: 0.05% TFA in H2O | |
| Mobile phase B | B: 0.05%TFA in ACN | |
| Flow rate | 1.0 mL/min | |
| Injection volume | 3µL | |
| Chromatographic column | Waters X-bridge C18 (150*4.6mm,3.5um) | |
| Column temperature | 25°C | |
| Detection wavelength | 225 nm | |
| Gradient (min) | A% | B% |
| 0.00 | 60 | 40 |
| 8.00 | 20 | 80 |
| 12.00 | 20 | 80 |
| 12.01 | 60 | 40 |
| 15.00 | 60 | 40 |

### Preparation of salt form of compound

### 2. Screening of salt form and crystal form of compound

### 2.1 Screening of salt form of compound

### 2.1.1 Experimental objective:

Different counter-ion bases were selected, and which counter-ion bases can form a salt of the compound were detected through appropriate crystallization methods.

### 2.1.2 Experimental steps:

1) Instruments and equipments

| Name | Model | Source |
|---|---|---|
| Analytical balance | XA105 | METTLER TOLEDO |
| Ultrasonic cleaner | SK5200LHC | Shanghai Kudos Ultrasonic Instrument |
| Pipette | Eppendorf (50 mL, 100 µL) | Eppendorf |

2) Operation procedure

### 3. Screening of salt form of free acid

### 3.1 Screening of salt form using solvent ACN system

A total of 181.82 mg of compound 28 was weighed, and 2.4 ml of ACN was added. A ball was formed, adhered to the wall, and not fully dissolved after ultrasonication and heating. 400 ul of water was added, and the mixture was subjected to ultrasonication. The solid adhered to the wall was gradually dissolved, resulting in a stock solution that was slightly turbid with minor mechanical impurities. Each aliquot (245 ul, equivalent to 15.91 mg of compound) was further treated by adding 1.1 equivalents of base solution (29.8 ul), as detailed in the table below. After overnight at room temperature, a solid was precipitated. The detection XRD of the solid was obtained.

The obtained 2-3 had good crystallinity. DSC showed that 2-3 might be a solvate or a hydrate. TGA further illustrated that 2-3 was dehydrated or desolvated at low temperature.

### Results of screening of salt form using ACN system

| Serial No. | Counter-ion base | Stock/ul | Counter-ion acid/ul | Phenomenon after adding base | Room temperature overnight | ACN was added | Evaporated to dryness | EA was added and slurried | XRD |
|---|---|---|---|---|---|---|---|---|---|
| 2-1 | 1 mol/L sodium hydroxideaqueous solution | 245 | 29.8 | Turn red, dissolved clarification | Dissolved clarification | Oil formation | Oil formation | Solid | |
| 2-2 | 1 mol/L potassium hydroxideaqueous solution | 245 | 29.8 | Turn red, dissolved clarification | Dissolved clarification | Oil formation | Oil formation | Solid | |
| 2-3 | 1 mol/L tromethamin e-aqueous solution | 245 | 29.8 | Dissolved clarification | Solid | / | / | / | Form A |

The experimental results showed that the results of screening of salt form using ACN system afforded crystal form A of tromethamine salt.

H-NMR analysis was performed on samples 2-3 to determine whether salt (Tris) was formed. The results showed that because the chemical shift of the methylene of Tris may overlap with the chemical shift of water, and the H of the hydroxyl did not produce a peak in H-NMR, it was impossible to determine whether Tris salt was formed by H-NMR.

The free form content was measured by HPLC-DAD to determine whether the salt was formed. As shown in the table below, the average free form content percentage of the two Tris salt samples was 80.58%, which was closest to the theoretical free form content percentage when binding 1 molecule of Tris and 1 molecule of water. Therefore, it was preliminarily determined that the Tris salt was a hydrate binding 1 molecule of water.

### HPLC-DAD quantitative results of Tris salt of compound 28

| Sample | sample 1 | sample 2 |
|---|---|---|
| Determination of free base content percentage in the compound | 81.91 | 79.25 |
| Theoretical free form content percentage when binding 1 molecule of Tris | 82.93 | |
| Theoretical free form content percentage when binding 1 molecule of Tris and 1 molecule of water | 80.88 | |
| Theoretical free form content percentage when binding 1 molecule of Tris and 1 molecule of acetonitrile | 78.40 | |
| Theoretical free form content percentage when binding 1 molecule of Tris, 1 molecule of acetonitrile and 1 molecule of water | 76.56 | |
| Theoretical water content percentage when binding 1 molecule of Tris and 1 molecule of water | 2.48 | |

| | | |
|---|---|---|
| Remarks: sample 1: ACN-H₂O-Tris (crystal form A) sample 2: MeOH- Tris-Evap-EA Slurry (crystal form A) | | |

The free form content was further measured using HPLC-ELSD to determine the salt formation ratio. The method was shown in the table below. The results showed that the average Tris content percentage of the two Tris salt samples was 16.13%, which was closest to the theoretical Tris content percentage when binding 1 molecule of Tris and 1 molecule of water.

### Methods and conditions of HPLC-ELSD quantitative determination

| Mobile phase | A: 75 mM ammonium acetate (pH 4.80); B: Acetonitrile |
|---|---|
| Chromatographic column | ZIC HILIC (150*4.6mm, 5um) |
| Column temperature | 40°C |
| ELSD temperature | 40°C |
| Pressure | 3.5bar |
| Proportion | 70%B (v/v) |
| Run time | 10min |

### HPLC-ELSD quantitative results of Tris salt of compound 28

| Sample | sample 1 | sample 2 |
|---|---|---|
| Determination of Tris amine content percentage in the compound | 15.76 | 16.57 |
| Theoretical Tris content percentage in a salt when binding 1 molecule of Tris | 17.07 | |
| Theoretical Tris content percentage in a salt when binding 1 molecule of Tris and 1 molecule of water | 16.64 | |

| | | |
|---|---|---|
| Remarks: sample 1: ACN-H₂O-Tris (crystal form A) sample 2: MeOH- Tris-Evap-EA Slurry (crystal form A) | | |

Combining the DSC pattern and TGA pattern, the weight loss (ACN-TRIS) before 80 degrees was about 2%, which was equivalent to the theoretical water content percentage in the Tris salt when binding 1 molecule of Tris and 1 molecule of water. It was further determined that the crystal form A of Tris salt was a monohydrate.

### 3.2 Screening of salt form using solvent DCM system

A total of 147.31 mg of compound 28 was weighed, and 2.2 ml of DCM was added to formulate a stock solution. After the dissolution was completed, each aliquot (200 ul, equivalent to 13.39 mg of compound) was evaporated to dryness at 40 degrees, then the following solvents (200 ul each) were respectively added for dissolution, then 1.1 equivalents of 1 mol/L tromethamine aqueous solution (25.1 uL) was respectively added, holes were pierced for evaporation to dryness. The phenomenon was shown in the following table.

### Results of screening of salt form using DCM system

| Serial No. | Solvent | Phenomenon after adding solvent | Phenomenon after adding Tris | Evaporation to dryness at room temperature | EA was added and slurried | XRD |
|---|---|---|---|---|---|---|
| 3-4 | Acetone | Dissolved clarification | Dissolved clarification | Oil formation | Solid | Crystal form C |
| 3-5 | MEK | Dissolved clarification | Dissolved clarification | Oil formation | Solid | Crystal form C |
| 3-6 | 2-Me-THF | Dissolved clarification | Dissolved clarification | Oil formation | Solid | Crystal form C |
| 3-8 | I-BuOH | Dissolved clarification | Dissolved clarification | Oil formation | Solid | Crystal form C |
| 3-9 | 2-BuOH | Dissolved clarification | Turbid, oil formation | Oil formation | Solid | Crystal form C |
| 3-10 | Dioxane | Dissolved clarification | Turbid, oil formation | Oil formation | Solid | Crystal form E |

The experimental results showed that the results of screening of salt form using DCM system afforded crystal forms C and E of tromethamine salt.

### 3.3 Screening of salt form using solvent ACN-H₂O system

27.69 mg of compound 28 was weighed, and ACN : H₂O = 6 : 1 (v/v) (400 ul) was added, the oil was adhered to the wall and gradually dissolved at 40 degrees. 1.1 eq of 1 mol/L tromethamine aqueous solution (51.8 ul) was added. Dissolved clarification occurred, the solution was kept at 40 degrees for 3 h, with no solid precipitation observed. Stirring at room temperature also yielded no precipitation. After ultrasonication for 1 to 2 min, violent precipitation occurred followed by solidification. 200 ul of ACN : H₂O = 6 : 1 (v/v) was supplemented, and the mixture was stirred at room temperature for 3 h, filtered, oven-dried in vacuo at 40 degrees overnight, and then corresponding characterization was performed. The XRD pattern of the sample showed that the crystal form D of tromethamine salt was obtained, and was a monohydrate similar to the crystal form A of Tris salt as showed by the DSC pattern.

### 4. Preparation methods of different salt forms

### (1) Preparation of crystal form A of tromethamine salt

15.91 mg of free acid was weighed and 245 uL of acetonitrile : water = 6 : 1 (v/v) was added. The mixture was rendered slightly turbid with minor mechanical impurities after ultrasonication. 1.1 equivalents of 1 mol/L tromethamine aqueous solution (29.8 ul) was added. After overnight at room temperature, solid was precipitated, and centrifuged quickly. The supernatant was removed, and the solid was dried under vacuum at 40°C to constant weight to obtain the crystal form A of tromethamine salt. Upon detection and analysis, it had the XRPD graph as shown in FIG. 1, the DSC graph as shown in FIG. 2, and the TGA graph as shown in FIG. 3.

### (2) Preparation of crystal form B of tromethamine salt

An appropriate amount of the crystal form A of tromethamine salt was taken, and the sample was heated to 88 degrees with TGA for 2 minutes. The obtained mixture was cooled to room temperature, and the crystal form B of tromethamine salt was obtained. Upon detection and analysis, it had the XRPD graph as shown in FIG. 4 and the DSC graph as shown in FIG. 5.

### (3) Preparation of crystal form C of tromethamine salt

229.54 mg of free form was weighed, and 0.4 ml of acetone was added. Dissolved clarification occurred after ultrasonication, and 1.6 ml of methanol was added. Dissolved clarification occurred at room temperature, and 0.527 ml (1.1 eq) of 0.815 mol/L Tris ethanol-water (18.44% water by mass fraction) solution was added at 40 degrees. Dissolved clarification occurred. 5 ml of isopropyl ether was added, producing slight turbidity. After being maintained at 40 degrees for 30 min, substantial precipitation occurred. 3 ml of isopropyl ether was supplemented, and the mixture was held at 40°C overnight, yielding a well-defined solid. The mixture was cooled to room temperature, and centrifuged quickly. The supernatant was removed, and the solid was dried under vacuum at 40°C to constant weight to obtain the crystal form C of tromethamine salt. Upon detection and analysis, it had the XRPD graph as shown in FIG. 6, the DSC graph as shown in FIG. 7, and the TGA graph as shown in FIG. 8.

### (4) Preparation of crystal form D of tromethamine salt

27.69 mg of free form was weighed, and ACN : H2O = 6 : 1 (v/v) (400 ul) was added, the oil was adhered to the wall and gradually dissolved at 40 degrees. 1.1 eq of 1 mol/L tromethamine aqueous solution (51.8 ul) was added. Dissolved clarification occurred, the solution was kept at 40 degrees for 3 h, with no solid precipitation observed. Stirring at room temperature also yielded no precipitation. After ultrasonication for 1 to 2 min, violent precipitation occurred followed by solidification. 200 ul of ACN : H2O = 6 : 1 (v/v) was supplemented, and the mixture was stirred at room temperature for 3 h and centrifuged quickly. The supernatant was removed, and the solid was dried under vacuum at 40°C to constant weight to obtain the crystal form D of tromethamine salt. Upon detection and analysis, it had the XRPD graph as shown in FIG. 9, and the DSC graph as shown in FIG. 10.

### (5) Preparation of crystal form E of tromethamine salt

13.39 mg of free form was weighed, and 200 uL of dioxane was added. Dissolution was performed, and 1.1 equivalents of 1 mol/L tromethamine aqueous solution (25.1 uL) was added, resulting in turbidity and an oily appearance. Holes were pierced for evaporation to dryness, yielding an oil. 200 ul of ethyl acetate was added, and slurrying was conducted at 40 degrees for 2 days, converting the material to a solid. After cooling to room temperature, rapid centrifugation was performed, and the supernatant was removed. The solid was dried under vacuum at 40°C to constant weight to obtain the crystal form E of tromethamine salt. After detection and analysis, the crystal form had an XRPD diagram as shown in FIG. 11 and a DSC diagram as shown in FIG. 12 as follows.

### 5. Thermodynamic stability experiment of crystal form

### 5.1 Experimental objective:

Through polycrystalline screening test, the releatively thrmodynamically stable crystal form of salt was found.

### 5.2 Experimental scheme:

### 5.2.1 The crystal form C of tromethamine salt was used as the starting raw material

10 mg of the crystal form C of tromethamine salt was taken, and 200 ul of solvent was respectively added. The mixture was slurried at 40 degrees for 2 days, and centrifuged, and the solid was oven-dried in vacuo at 40 degrees overnight, and then XRD and other corresponding characterizations were performed. The results were shown in the table below. The XRD of the obtained solid showed that it was crystal form C, indicating that the crystal form C of tromethamine salt was very stable.

| Serial No. | Solvent | Solvent volume ul | Phenomenon after slurrying for 2 days | XRD |
|---|---|---|---|---|
| 13-1 | EA | 200 | Solid | Crystal form C |
| 13-2 | IPAC | 200 | Solid | Crystal form C |
| 13-3 | MTBE | 200 | Solid | Crystal form C |
| 13-4 | IPE | 200 | Solid | Crystal form C |
| 13-6 | Heptane | 200 | Solid | Crystal form C |
| 13-7 | hexane | 200 | Solid | Crystal form C |
| 13-8 | EtOH | 200 | Solid | Crystal form C |
| 13-9 | IPA | 200 | Solid | Crystal form C |
| 13-10 | water | 200 | Dissolved clarification (Dissolved clarification after supplement of sample) after | |

### 6. Solubility determination of crystal form C of tromethamine salt

1 to 2 mg of compound 28 was weighed into a 1.5 mL liquid phase vial, then 1 mL of pH buffer, artificial simulated gastric fluid (FaSSGF), artificial fasted-state simulated intestinal fluid (FaSSIF), artificial non-fasted-state simulated intestinal fluid (FeSSIF), and pure water were respectively added, and the mixture was placed on a constant temperature oscillator overnight at a temperature of 37 degrees. After 24 h, the sample solution was filtered with a 0.45 µm mixed water fiber filter membrane and then the filtrate was taken. The content was tested by HPLC. For the HPLC detection method, please refer to the solution stability analysis method in Section 1.2. The solubility of the compound in the buffer solution medium was shown in the following table.

### Results of solubility of crystal form C of tromethamine salt in different pH buffers

| Tromethamine salt | Medium | Solubility mg/ml |
|---|---|---|
| Crystal form C | USP Buffer pH 7.4 | 0.2539 |
| | FaSSIF | 0.4782 |
| | FeSSIF | 0.7560 |
| | H₂O | 0.0664 |

## Claims

1. A base salt of a compound as represented by general formula (I) or a stereoisomer thereof, **characterized in that** the compound has a structure as follows: 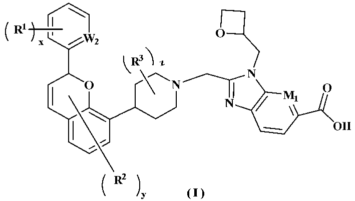 wherein:
each R¹ is independently selected from hydrogen, deuterium, fluorine, chlorine, cyano, C₁₋₃ alkyl, C₁₋₃ deuteroalkyl, C₁₋₃ haloalkyl, C₁₋₃ alkoxy, C₁₋₃ deuteroalkoxy or C₁₋₃ haloalkoxy;
each R² is independently selected from hydrogen, deuterium, fluorine, chlorine, cyano, C₁₋₃ alkyl, C₁₋₃ deuteroalkyl, C₁₋₃ haloalkyl, C₁₋₃ alkoxy, C₁₋₃ deuteroalkoxy or C₁₋₃ haloalkoxy;
each R³ is independently selected from hydrogen, deuterium or halogen;
M₁ is N or CH;
W₂ is N or CH;
x, y and z are each independently 0, 1 or 2;
wherein the base is an organic base or an inorganic base, and the organic base is selected from diethylamine, diethanolamine, triethylamine, triethanolamine, choline hydroxide, tromethamine, meglumine, N-hydroxyethylmorpholine, piperazine, N-hydroxyethylpyrrolidine, N,N-dibenzylethylenediamine, 2-diethylaminoethanol, ethanolamine, betaine, L-arginine, lysine, phenethylbenzylamine, benzathine penicillin, dimethylaminoethanol, imidazole or a mixture thereof; and the inorganic base is selected from sodium hydroxide, potassium hydroxide, calcium hydroxide, magnesium hydroxide, zinc hydroxide, ammonia water or a mixture thereof.

2. The base salt of the compound or the stereoisomer thereof according to claim 1, **characterized in that**
each R₁ is independently selected from hydrogen, deuterium, fluorine, chlorine, methoxy or -OCD₃;
each R² is independently selected from hydrogen, deuterium, fluorine, chlorine or methyl;
each R³ is independently hydrogen;
M₁ is CH;
W₂ is CH; and
x, y and z are each independently 0, 1 or 2.

3. The base salt of the compound or the stereoisomer thereof according to claim 1, **characterized in that** the compound is further as represented by the following general formula (I-1) to (I-4):

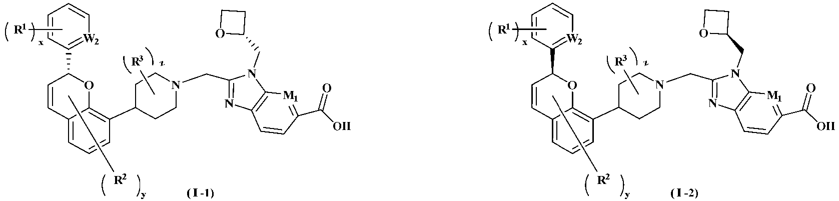

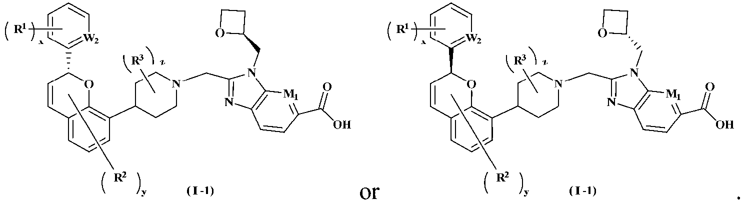

4. The base salt of the compound or the stereoisomer thereof according to claims 1-3, **characterized in that** the general formula is selected from the following compounds: 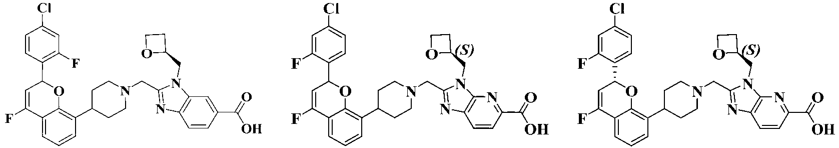 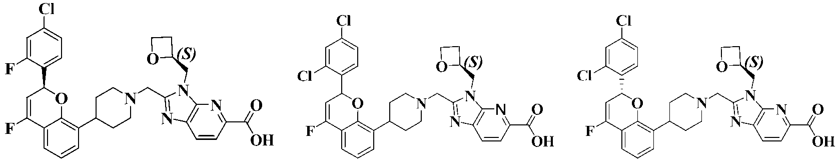 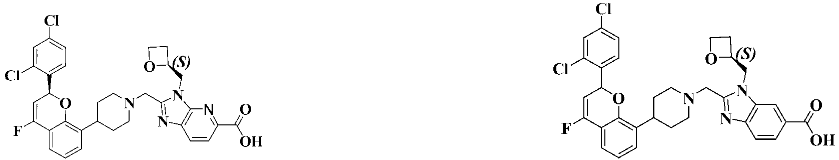 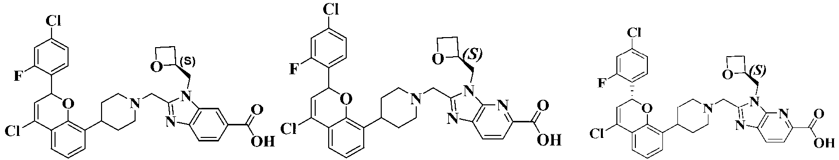 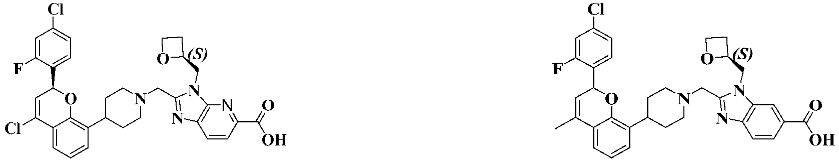 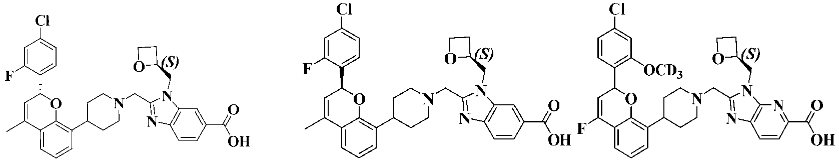 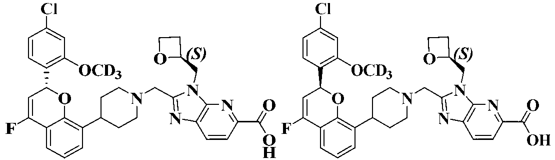 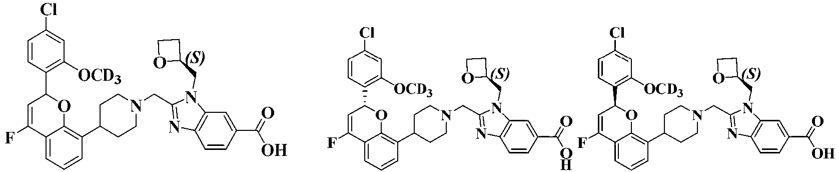 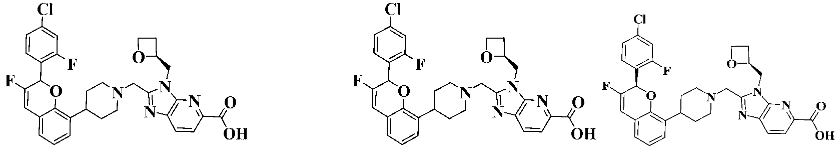 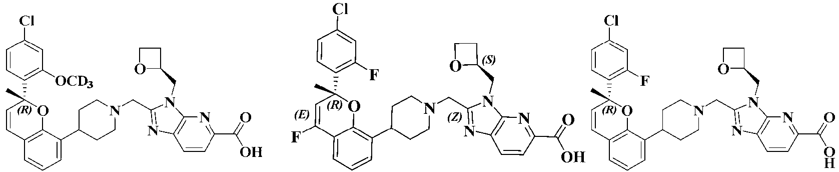 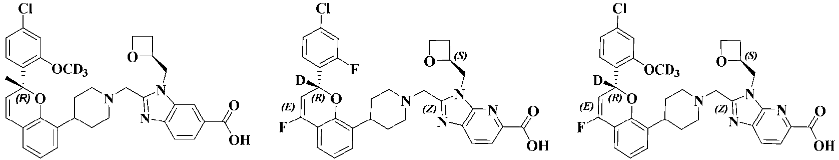 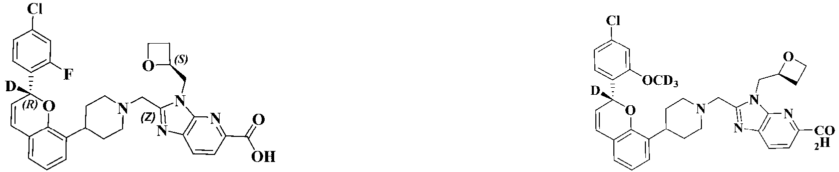 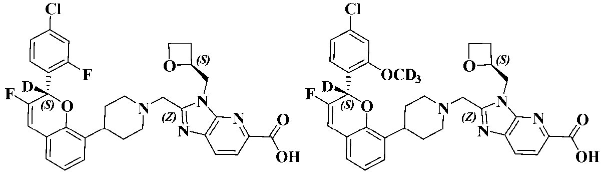 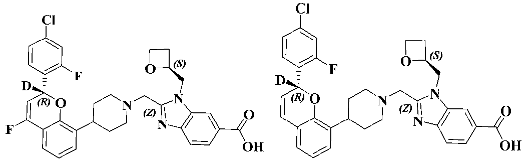 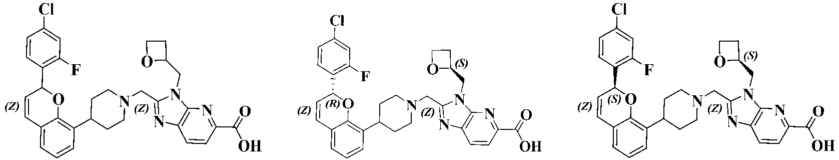 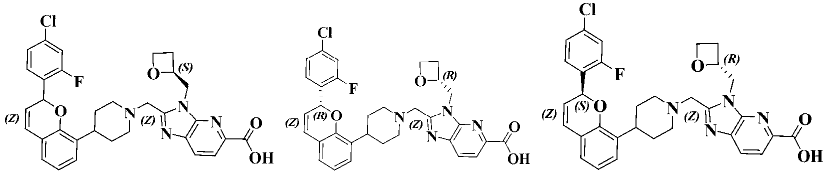 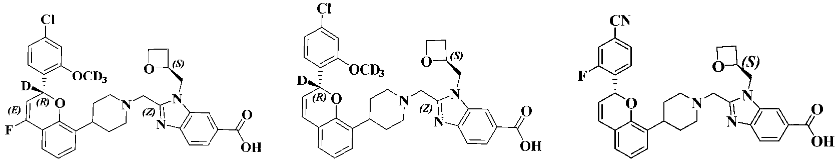 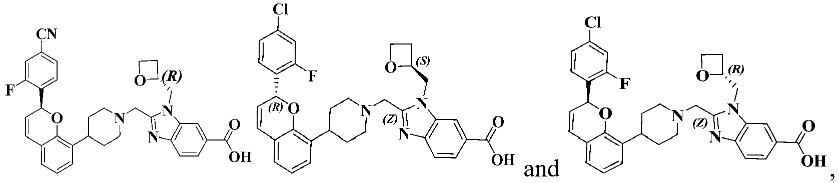 wherein the base is an organic base or an inorganic base, and the organic base is selected from diethylamine, diethanolamine, triethylamine, triethanolamine, choline hydroxide, tromethamine, or a mixture thereof; and the inorganic base is selected from sodium hydroxide, potassium hydroxide, calcium hydroxide, magnesium hydroxide, or a mixture thereof.

5. The base salt of the compound according to claim 4, **characterized in that** the compound is 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid, wherein the organic base is selected from diethylamine, diethanolamine, triethylamine, triethanolamine, choline hydroxide, tromethamine or a mixture thereof; and the inorganic base is selected from sodium hydroxide, potassium hydroxide, calcium hydroxide, magnesium hydroxide, or a mixture thereof.

6. The base salt of the compound according to any one of claims 1-5, **characterized in that** the number of the base is 0.5-3, preferably 0.5, 1, 1.5, 2, 2.5 or 3, further preferably 0.5, 1, 2 or 3, and even further preferably 1.

7. The base salt of the compound according to any one of claims 1-5, **characterized in that** the base salt is a hydrate or an anhydrate, preferably an anhydrate;
when the base salt is a hydrate, the number of water molecules is 0.2-3, preferably 0.2, 0.5, 1, 1.5, 2, 2.5 or 3, and more preferably 0.5, 1, 2 or 3.

8. The base salt of the compound according to claim 4, **characterized in that** the base salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid is in a crystal form.

9. The base salt of the compound according to claim 8, **characterized in that** the crystal form of the base salt of 2-((4-((R)-2-(4-chloro-2-fluorophenyl)-2H-chromen-8-yl)piperidin-1-yl)methyl)-3-(((S)-oxetan-2-yl)methyl)-1H-benzo[d]imidazole-6-carboxylic acid is selected from crystal forms A to E of tromethamine salt, wherein:
the X-ray powder diffraction pattern of the crystal form A of tromethamine salt has a characteristic peak at 2θ of 7.7±0.2°, or has a characteristic peak at 2θ of 9.8±0.2°, or has a characteristic peak at 2θ of 10.8±0.2°, or has a characteristic peak at 20 of 11.6±0.2°, or has a characteristic peak at 2θ of 14.0±0.2°, or has a characteristic peak at 2θ of 14.5±0.2°, or has a characteristic peak at 2θ of 14.8±0.2°, or has a characteristic peak at 2θ of 15.1±0.2°, or has a characteristic peak at 2θ of 15.9±0.2°, or has a characteristic peak at 2θ of 18.0±0.2°, or has a characteristic peak at 2θ of 18.7±0.2°, or has a characteristic peak at 2θ of 19.3±0.2°, or has a characteristic peak at 2θ of 20.0±0.2°, or has a characteristic peak at 2θ of 20.5±0.2°, or has a characteristic peak at 2θ of 21.1±0.2°, or has a characteristic peak at 2θ of 22.6±0.2°, or has a characteristic peak at 2θ of 23.7+0.2°, or has a characteristic peak at 2θ of 25.1±0.2°; preferably, the X-ray powder diffraction pattern has characteristic peaks at any 2, 4, 6, 8, 10 or 12 of the above-mentioned 2θ;
the X-ray powder diffraction pattern of the crystal form B of tromethamine salt has a characteristic peak at 2θ of 8.2±0.2°, or has a characteristic peak at 2θ of 10.1±0.2°, or has a characteristic peak at 2θ of 12.3±0.2°, or has a characteristic peak at 2θ of 14.4±0.2°, or has a characteristic peak at 2θ of 14.8±0.2°, or has a characteristic peak at 2θ of 16.0±0.2°, or has a characteristic peak at 2θ of 16.2±0.2°, or has a characteristic peak at 2θ of 17.5±0.2°, or has a characteristic peak at 2θ of 17.7±0.2°, or has a characteristic peak at 2θ of 18.3±0.2°, or has a characteristic peak at 2θ of 18.7±0.2°, or has a characteristic peak at 2θ of 19.7+0.2°, or has a characteristic peak at 2θ of 20.5±0.2°, or has a characteristic peak at 2θ of 20.9+0.2°, or has a characteristic peak at 2θ of 21.9±0.2°, or has a characteristic peak at 2θ of 22.1±0.2°, or has a characteristic peak at 2θ of 22.4±0.2°, or has a characteristic peak at 2θ of 24.7±0.2°; preferably, the X-ray powder diffraction pattern has characteristic peaks at any 2, 4, 6, 8, 10 or 12 of the above-mentioned 2θ;
the X-ray powder diffraction pattern of the crystal form C of tromethamine salt has a characteristic peak at 2θ of 3.6±0.2°, or has a characteristic peak at 2θ of 7.1±0.2°, or has a characteristic peak at 2θ of 9.7±0.2°, or has a characteristic peak at 2θ of 10.6±0.2°, or has a characteristic peak at 2θ of 13.5±0.2°, or has a characteristic peak at 2θ of 14.1±0.2°, or has a characteristic peak at 2θ of 15.0±0.2°, or has a characteristic peak at 2θ of 16.0±0.2°, or has a characteristic peak at 2θ of 16.5±0.2°, or has a characteristic peak at 2θ of 17.1±0.2°, or has a characteristic peak at 2θ of 17.6±0.2°, or has a characteristic peak at 2θ of 19.0±0.2°, or has a characteristic peak at 2θ of 19.7+0.2°, or has a characteristic peak at 2θ of 20.8±0.2°, or has a characteristic peak at 2θ of 21.8±0.2°, or has a characteristic peak at 2θ of 22.3±0.2°, or has a characteristic peak at 2θ of 23.1±0.2°, or has a characteristic peak at 2θ of 26.4±0.2°, or has a characteristic peak at 2θ of 28.3±0.2°; preferably, the X-ray powder diffraction pattern has characteristic peaks at any 2, 4, 6, 8, 10 or 12 of the above-mentioned 2θ;
the X-ray powder diffraction pattern of the crystal form D of tromethamine salt has a characteristic peak at 2θ of 7.4±0.2°, or has a characteristic peak at 2θ of 7.7±0.2°, or has a characteristic peak at 2θ of 9.8±0.2°, or has a characteristic peak at 2θ of 10.8±0.2°, or has a characteristic peak at 2θ of 11.6±0.2°, or has a characteristic peak at 2θ of 13.1±0.2°, or has a characteristic peak at 2θ of 14.0±0.2°, or has a characteristic peak at 2θ of 14.5±0.2°, or has a characteristic peak at 2θ of 15.1±0.2°, or has a characteristic peak at 2θ of 15.4±0.2°, or has a characteristic peak at 2θ of 15.8±0.2°, or has a characteristic peak at 2θ of 17.9±0.2°, or has a characteristic peak at 2θ of 18.8±0.2°, or has a characteristic peak at 2θ of 19.3±0.2°, or has a characteristic peak at 2θ of 20.0±0.2°, or has a characteristic peak at 2θ of 20.5±0.2°, or has a characteristic peak at 2θ of 21.2±0.2°, or has a characteristic peak at 2θ of 21.8±0.2°, or has a characteristic peak at 2θ of 23.3±0.2'; preferably, the X-ray powder diffraction pattern has characteristic peaks at any 2, 4, 6, 8, 10 or 12 of the above-mentioned 2θ;
the X-ray powder diffraction pattern of the crystal form E of tromethamine salt has a characteristic peak at 2θ of 4.3±0.2°, or has a characteristic peak at 2θ of 6.3±0.2°, or has a characteristic peak at 2θ of 8.6±0.2°, or has a characteristic peak at 2θ of 9.3±0.2°, or has a characteristic peak at 2θ of 13.6±0.2°, or has a characteristic peak at 2θ of 14.1±0.2°, or has a characteristic peak at 2θ of 17.7±0.2°, or has a characteristic peak at 2θ of 18.5±0.2°, or has a characteristic peak at 2θ of 18.9±0.2°, or has a characteristic peak at 2θ of 20.2±0.2°, or has a characteristic peak at 2θ of 20.5±0.2°, or has a characteristic peak at 2θ of 21.4±0.2°, or has a characteristic peak at 2θ of 21.9±0.2°, or has a characteristic peak at 2θ of 22.4±0.2°, or has a characteristic peak at 2θ of 23.4±0.2°, or has a characteristic peak at 2θ of 23.9±0.2°, or has a characteristic peak at 2θ of 25.2±0.2°; preferably, the X-ray powder diffraction pattern has characteristic peaks at any 2, 4, 6, 8, 10 or 12 of the above-mentioned 2θ.

10. The crystal form of tromethamine salt according to claim 8, **characterized in that**
the X-ray powder diffraction pattern of the crystal form A of tromethamine salt has a characteristic peak at one or more of 2θ of 7.7±0.2°, 9.8±0.2°, 14.0±0.2° or 15.1±0.2°, preferably at 2 to 4 of the above-mentioned 2θ, more preferably at 3 or 4 of the above-mentioned 2θ, and most preferably at 4 of the above-mentioned 20; optionally, further, the X-ray powder diffraction pattern can further have a characteristic peak at one or more of 2θ of 14.5±0.2°, 18.7±0.2°, 19.3±0.2°, 20.0±0.2°, 20.5±0.2° or 21.1±0.2°, preferably at 2, 3, 4 or 6 of the above-mentioned 20;
the X-ray powder diffraction pattern of the crystal form B of tromethamine salt has a characteristic peak at one or more of 2θ of 10.1±0.2°, 14.4±0.2°, 18.7±0.2° or 21.9±0.2°; preferably at 2 to 4 of the above-mentioned 2θ, more preferably at 3 or 4 of the above-mentioned 2θ, and most preferably at 4 of the above-mentioned 20; optionally, further, the X-ray powder diffraction pattern can further have a characteristic peak at one or more of 2θ of 8.2±0.2°, 12.3±0.2°, 14.8±0.2°, 19.7+0.2°, 20.5±0.2° or 22.1±0.2°, preferably at 2, 3, 4 or 6 of the above-mentioned 2θ;
the X-ray powder diffraction pattern of the crystal form C of tromethamine salt has a characteristic peak at one or more of 2θ of 3.6±0.2°, 7.1±0.2°, 9.7±0.2°, 14.1±0.2° or 16.0±0.2°; preferably at 2 to 4 of the above-mentioned 2θ, more preferably at 3 or 4 of the above-mentioned 2θ, and most preferably at 4 of the above-mentioned 20; optionally, further, the X-ray powder diffraction pattern can further have a characteristic peak at one or more of 2θ of 15.0±0.2°, 16.5±0.2°, 17.1±0.2°, 17.6±0.2°, 19.7±0.2° or 20.8±0.2°, preferably at 2, 3, 4 or 6 of the above-mentioned 20;
the X-ray powder diffraction pattern of the crystal form D of tromethamine salt has a characteristic peak at one or more of 2θ of 7.4±0.2°, 9.8±0.2°, 13.1±0.2° or 14.0±0.2°; preferably at 2 to 4 of the above-mentioned 2θ, more preferably at 3 or 4 of the above-mentioned 2θ, and most preferably at 4 of the above-mentioned 20; optionally, further, the X-ray powder diffraction pattern can further have a characteristic peak at one or more of 2θ of 7.7±0.2°, 15.1±0.2°, 17.9±0.2°, 18.8±0.2°, 19.3±0.2° or 20.0±0.2°, preferably at 2, 3, 4 or 6 of the above-mentioned 2θ;
the X-ray powder diffraction pattern of the crystal form E of tromethamine salt has a characteristic peak at one or more of 2θ of 4.3±0.2°, 6.3±0.2°, 13.6±0.2° or 18.9±0.2°; preferably at 2 to 4 of the above-mentioned 2θ, more preferably at 3 or 4 of the above-mentioned 2θ, and most preferably at 4 of the above-mentioned 20; optionally, further, the X-ray powder diffraction pattern can further have a characteristic peak at one or more of 2θ of 8.6±0.2°, 14.1±0.2°, 17.7±0.2°, 20.2±0.2°, 20.5±0.2° or 22.4±0.2°, preferably at 2, 3, 4 or 6 of the above-mentioned 2θ.

11. The crystal form of tromethamine salt according to claim 8, **characterized in that**
the X-ray powder diffraction pattern of the crystal form A of tromethamine salt has characteristic peaks at 2θ of 9.8±0.2° and 14.0±0.2°; preferably, the X-ray powder diffraction pattern further has characteristic peaks at 2θ of 7.7±0.2° and 15.1±0.2°; more preferably, the X-ray powder diffraction pattern further has characteristic peaks at 2θ of 18.7±0.2°, 19.3±0.2°, 20.0±0.2° and 21.1±0.2°; further preferably, the X-ray powder diffraction pattern further has characteristic peaks at 2θ of 14.5±0.2° and 20.5±0.2°; more further preferably, the X-ray powder diffraction pattern further has a characteristic peak at one or more of 10.8±0.2°, 11.6±0.2°, 18.0±0.2° and 22.6±0.2°;
the X-ray powder diffraction pattern of the crystal form B of tromethamine salt has characteristic peaks at 2θ of 10.1±0.2° and 14.4±0.2°; preferably, the X-ray powder diffraction pattern further has characteristic peaks at 2θ of 18.7±0.2° and 21.9±0.2°; more preferably, the X-ray powder diffraction pattern further has characteristic peaks at 2θ of 8.2±0.2°, 14.8±0.2°, 20.5±0.2° and 22.1±0.2°; further preferably, the X-ray powder diffraction pattern further has characteristic peaks at 2θ of 12.3±0.2° and 19.7±0.2°; more further preferably, the X-ray powder diffraction pattern further has a characteristic peak at one or more of 16.0±0.2°, 16.2±0.2°, 18.3±0.2° and 20.9±0.2°;
the X-ray powder diffraction pattern of the crystal form C of tromethamine salt has characteristic peaks at 2θ of 3.6±0.2° and 7.1±0.2°; preferably, the X-ray powder diffraction pattern further has characteristic peaks at 2θ of 9.7±0.2° and 14.1±0.2°; more preferably, the X-ray powder diffraction pattern further has characteristic peaks at 2θ of 15.0±0.2°, 16.0±0.2°, 16.5±0.2°, 17.6±0.2° and 20.8±0.2°; further preferably, the X-ray powder diffraction pattern further has characteristic peaks at 2θ of 17.1±0.2° and 19.7±0.2°; more further preferably, the X-ray powder diffraction pattern further has a characteristic peak at one or more of 13.5±0.2°, 19.0±0.2°, 21.8±0.2° and 26.4±0.2°;
the X-ray powder diffraction pattern of the crystal form D of tromethamine salt has characteristic peaks at 2θ of 9.8±0.2° and 14.0±0.2°; preferably, the X-ray powder diffraction pattern further has characteristic peaks at 2θ of 7.4±0.2° and 13.1±0.2°; more preferably, the X-ray powder diffraction pattern further has characteristic peaks at 2θ of 7.7±0.2°, 15.1±0.2°, 18.8±0.2° and 20.0±0.2°; further preferably, the X-ray powder diffraction pattern further has characteristic peaks at 2θ of 17.9±0.2° and 19.3±0.2°; more further preferably, the X-ray powder diffraction pattern further has a characteristic peak at one or more of 14.5±0.2°, 15.4±0.2°, 15.8±0.2° and 21.2±0.2°;
the X-ray powder diffraction pattern of the crystal form E of tromethamine salt has characteristic peaks at 2θ of 6.3±0.2° and 13.6±0.2°; preferably, the X-ray powder diffraction pattern further has characteristic peaks at 2θ of 4.3±0.2° and 18.9±0.2°; more preferably, the X-ray powder diffraction pattern further has characteristic peaks at 2θ of 8.6±0.2°, 17.7±0.2°, 20.2±0.2° and 22.4±0.2°; further preferably, the X-ray powder diffraction pattern further has characteristic peaks at 2θ of 14.1±0.2° and 20.5±0.2°; more further preferably, the X-ray powder diffraction pattern further has a characteristic peak at one or more of 9.3±0.2°, 18.5±0.2°, 21.4±0.2° and 21.9±0.2°.

12. The crystal form of tromethamine salt according to claim 8, **characterized in that**
the X-ray powder diffraction pattern of the form A is as shown in FIG. 1, and preferably, the form A has a DSC pattern as shown in FIG. 2, or has a TGA pattern as shown in FIG. 3;
the X-ray powder diffraction pattern of the form B at 2θ is as shown in FIG. 4, preferably, the form B has a DSC pattern as shown in FIG. 5;
the X-ray powder diffraction pattern of the form C at 2θ is as shown in FIG. 6, preferably, the form C has a DSC pattern as shown in FIG. 7, or has a TGA pattern as shown in FIG. 8;
the X-ray powder diffraction pattern of the form D at 2θ is as shown in FIG. 9, preferably, the form D has a DSC pattern as shown in FIG. 10;
and the X-ray powder diffraction pattern of the form E at 2θ is as shown in FIG. 11, preferably, the form E has a DSC pattern as shown in FIG. 12.

13. The crystal form of tromethamine salt according to claim 8, **characterized in that** the positions of diffraction peaks with the top ten relative peak intensities in the X-ray powder diffraction patterns of the crystal form A, the crystal form B, the crystal form C, the crystal form D and the crystal form E have a 2θ deviation of ±0.2° to ±0.5°, preferably ±0.2° to ±0.3°, and more preferably ±0.2° from the corresponding positions of diffraction peaks in the FIG. 1, FIG. 4, FIG. 6, FIG. 9 and FIG. 11.

14. A method for preparing the base salt of the compound according to any one of claims 1-13, **characterized in that** the method specifically comprises the following steps:
1) weighing an appropriate amount of a free acid and dissolving the free acid in a good solvent;
2) weighing an appropriate amount of a counter-ion base and dissolving the counter-ion base in an organic solvent; wherein the amount of the counter-ion base is preferably 1.0 to 1.5 equivalents;
3) combining the above two solutions, and stirring the combined solution to allow precipitation, or dropwise adding a poor solvent followed by the stirring to allow precipitation; and
4) subjecting the mixture to rapid centrifugation or standing to evaporate to dryness so as to obtain a target product;
wherein:
the good solvent is selected from acetone, tetrahydrofuran, ethyl formate, ethyl acetate, 2-methyl-tetrahydrofuran, 2-butanone, n-butanol, 1,4-dioxane, isobutanol, N,N-dimethylformamide, N,N-dimethylacetamide, n-propanol or tert-butanol; preferably 2-methyl-tetrahydrofuran, ethyl acetate, 2-butanone, acetone or ethyl formate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyl-tetrahydrofuran, 2-butanone, 3-pentanone, heptane, methyl tert-butyl ether, isopropyl ether, 1,4-dioxane, tert-butanol, or N,N-dimethylformamide; preferably methanol, ethanol, or acetonitrile; the above good solvent and organic solution must be miscible when used;
the poor solvent is selected from heptane, methyl tert-butyl ether, cyclohexane, toluene, isopropyl ether, and ethyl acetate; preferably methyl tert-butyl ether and isopropyl ether;
the counter-ion base is an organic base or an inorganic base, and the organic base is selected from diethylamine, diethanolamine, triethylamine, triethanolamine, choline hydroxide, tromethamine, meglumine, N-hydroxyethylmorpholine, piperazine, N-hydroxyethylpyrrolidine, N,N-dibenzylethylenediamine, 2-diethylaminoethanol, ethanolamine, betaine, L-arginine, lysine, phenethylbenzylamine, benzathine penicillin, dimethylaminoethanol, imidazole or a mixture thereof; preferably, diethylamine, diethanolamine, triethylamine, triethanolamine, choline hydroxide, tromethamine, or a mixture thereof; further preferably, tromethamine; the inorganic base is selected from sodium hydroxide, potassium hydroxide, calcium hydroxide, magnesium hydroxide, zinc hydroxide, ammonia water or a mixture thereof, preferably sodium hydroxide, potassium hydroxide, calcium hydroxide, magnesium hydroxide or a mixture thereof;
alternatively,
1) weighing an appropriate amount of a free acid, and suspending the free acid with a poor solvent;
2) weighing an appropriate amount of a counter-ion base and dissolving the counter-ion base in an organic solvent; wherein the amount of the counter-ion base is preferably 1.0 to 1.5 equivalents;
3) combining the above two solutions and stirring the combined solution to allow dissolution, followed by continue stirring;
4) subjecting the mixture to rapid centrifugation or standing to evaporate to dryness so as to obtain a target product;
wherein:
the poor solvent is selected from ethanol, ethyl acetate, ethyl formate, isopropanol, isopropyl acetate, methyl tert-butyl ether, dichloromethane, methanol, acetonitrile, chlorobenzene, benzene, toluene, n-butanol, isobutanol or 3-pentanone; preferably, ethanol, ethyl acetate, isopropanol and isopropyl acetate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-methyl-tetrahydrofuran, 2-butanone, 3-pentanone, heptane, methyl tert-butyl ether, isopropyl ether, 1,4-dioxane, tert-butanol, or N,N-dimethylformamide; preferably methanol, ethanol, or acetonitrile; the above good solvent and organic solution must be miscible when used;
the counter-ion base is an organic base or an inorganic base, and the organic base is selected from diethylamine, diethanolamine, triethylamine, triethanolamine, choline hydroxide, tromethamine, meglumine, N-hydroxyethylmorpholine, piperazine, N-hydroxyethylpyrrolidine, N,N-dibenzylethylenediamine, 2-diethylaminoethanol, ethanolamine, betaine, L-arginine, lysine, phenethylbenzylamine, benzathine penicillin, dimethylaminoethanol, imidazole or a mixture thereof; preferably, diethylamine, diethanolamine, triethylamine, triethanolamine, choline hydroxide, tromethamine, or a mixture thereof; further preferably, tromethamine; the inorganic base is selected from sodium hydroxide, potassium hydroxide, calcium hydroxide, magnesium hydroxide, zinc hydroxide, ammonia water or a mixture thereof, preferably sodium hydroxide, potassium hydroxide, calcium hydroxide, magnesium hydroxide or a mixture thereof;
alternatively,
1) weighing an appropriate amount of the base salt of the compound, and suspending the base salt with a poor solvent preferably at a suspension density of 50-200 mg/mL;
2) shaking the obtained suspension at a certain temperature for a certain time, preferably at a temperature of 25-50°C for preferably 1-15 days;
3) quickly centrifuging the suspension obtained above, removing a supernatant, placing a remaining solid in a vacuum drying oven and drying to a constant weight to afford a target product;
wherein:
the poor solvent is selected from dichloromethane, 1,4-dioxane, acetonitrile, chlorobenzene, benzene, toluene, acetone, ethyl acetate, water, 88% acetone, isopropyl acetate, 3-pentanone, ethyl formate, tetrahydrofuran, 2-methyl-tetrahydrofuran, isopropanol, n-butanol, isobutanol, n-propanol, methyl tert-butyl ether, n-heptane, tert-butanol or 2-butanone.

15. A pharmaceutical composition, **characterized in that** the pharmaceutical composition comprises a therapeutically effective amount of the base salt or the crystal form thereof of the compound or the stereoisomer thereof according to any one of claims 1 to 14, and one or more pharmaceutically acceptable carriers, diluents or excipients.

16. The base salt or the crystal form thereof of the compound or the stereoisomer thereof according to any one of claims 1 to 14, and the pharmaceutical composition according to claim 15, **characterized in that** the base salt or the crystal form thereof of the compound or the stereoisomer thereof and the pharmaceutical composition comprise a therapeutically effective amount of the base salt or the crystal form thereof, and the therapeutically effective amount includes 0.0001-99%, 0.0001-95%, 0.0001-90%, 0.0001-85%, 0.0001-80%, 0.0001-75%, 0.0001-70%, 0.001-60%, 0.001-55%, 0.01-50%, 0.01-40%, 0.01-30%, 0.01-20%, 0.01-10% or 0.01-5%.

17. The base salt or the crystal form thereof of the compound or the stereoisomer thereof according to any one of claims 1 to 14, or the pharmaceutical composition according to claim 15 for use in the preparation of a GLP-1 receptor agonist drug.

18. The base salt or the crystal form thereof of the compound or the stereoisomer thereof according to any one of claims 1 to 14, or the pharmaceutical composition according to claim 15 for use in the preparation of a drug for treating metabolism related diseases, wherein preferably, the metabolism related diseases are selected from diabetes, obesity or non-alcohol steatohepatitis related diseases or other related diseases caused by diabetes, obesity or non-alcoholic steatohepatitis.
